(19) Europäisches Patentamt
European Patent Office
Office européen des brevets

(11) Veröffentlichungsnummer: **0 442 073 B1**

# (12) EUROPÄISCHE PATENTSCHRIFT

(45) Veröffentlichungstag der Patentschrift: **25.01.95**

(21) Anmeldenummer: **90123777.6**

(22) Anmeldetag: **11.12.90**

(51) Int. Cl.6: **C07D 487/04**, C07D 471/04, C07D 487/14, C07D 471/14, C07D 491/147, A01N 43/90, A01N 57/16, //(C07D471/04, 221:00,209:00),(C07D487/04, 209:00,209:00),(C07D487/14, 209:00,209:00,209:00), (C07D471/14,221:00,209:00, 209:00)

## (54) Polycyclische 3-Aryl-pyrrolidin-2,4,-dion-Derivate.

(30) Priorität: **13.02.90 DE 4004321**
**10.10.90 DE 4032090**

(43) Veröffentlichungstag der Anmeldung:
**21.08.91 Patentblatt 91/34**

(45) Bekanntmachung des Hinweises auf die Patenterteilung:
**25.01.95 Patentblatt 95/04**

(84) Benannte Vertragsstaaten:
**BE CH DE ES FR GB IT LI NL**

(56) Entgegenhaltungen:
**EP-A- 0 262 399**
**EP-A- 0 355 599**

(73) Patentinhaber: **BAYER AG**

**D-51368 Leverkusen (DE)**

(72) Erfinder: **Bertram, Heinz-Jürgen, Dr.**
**Nesselroder Strasse 27**
**W-5300 Bonn (DE)**
Erfinder: **Fischer, Reiner, Dr.**
**Nelly-Sachs-Strasse 23**
**W-4019 Monheim 2 (DE)**
Erfinder: **Hagemann, Hermann, Dr.**
**Kandinsky-Strasse 52**
**W-5090 Leverkusen 1 (DE)**
Erfinder: **Krüger, Bernd-Wieland, Dr.**
**Unterboschbach 19**
**W-5060 Bergisch-Gladbach (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**LIEBIGS ANNALEN DER CHEMIE, Nr. 5, 1985, Seiten 1095-1098, Weinheim, DE; R.SCHMIERER et al.: "Cyclisierung von N-Acylalanin- und N-Acylglycinestern"**

Erfinder: **Schenke, Thomas, Dr.**
**Mühlenstrasse 113**
**W-5060 Bergisch-Gladbach 2 (DE)**
Erfinder: **Erdelen, Christoph, Dr.**
**Unterbüscherhof 22**
**W-5653 Leichlingen 1 (DE)**
Erfinder: **Krauskopf, Birgit, Dr.**
**Kicke 19**
**W-5060 Bergisch Gladbach 1 (DE)**
Erfinder: **Lürssen, Klaus, Dr.**
**August-Kierspel-Strasse 145**
**W-5060 Bergisch Gladbach (DE)**
Erfinder: **Santel, Hans-Joachim, Dr.**
**Grünstrasse 9a**
**W-5090 Leverkusen 1 (DE)**
Erfinder: **Schmidt, Robert R., Dr.**
**Im Waldwinkel 110**
**W-5060 Bergisch Gladbach (DE)**
Erfinder: **Wachendorff, Ulrike, Dr.**
**Kriescherstrasse 81**
**W-4019 Monheim (DE)**

## Beschreibung

Die Erfindung betrifft neue polycyclische 3-Aryl-pyrrolidin-2,4-dion-Derivate, mehrere Verfahren zu ihrer Herstellung und ihre Verwendung als Insektizide, Akarizide und Herbizide.

Von 3-Acyl-pyrrolidin-2,4-dionen sind pharmazeutische Eigenschaften vorbeschrieben (S. Suzuki et. al. Chem. Pharm. Bull. 15 1120 (1967)). Weiterhin wurden N-Phenylpyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist.

Es wurden nun neue polycyclische 3-Arylpyrrolidin-2,4-dion-Derivate der Formel (I)

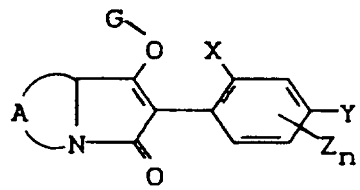

(I)

gefunden,
in welcher die Gruppierung

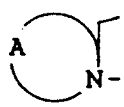

eine der folgenden Bedeutungen 1 bis 29 annimmt:

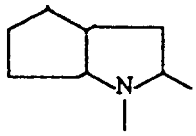
1

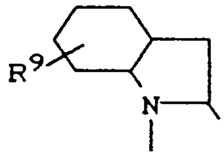
2

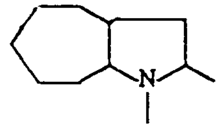
3

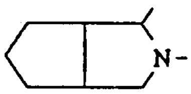
4

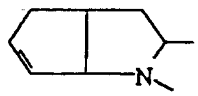
5

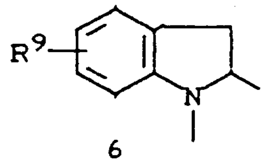
6

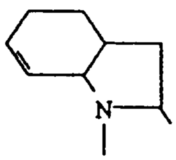
7

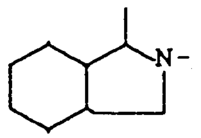
8

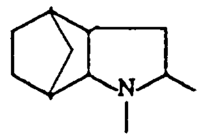
9

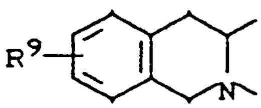
10

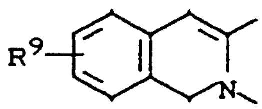
11

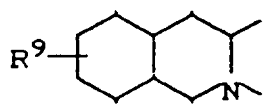
12

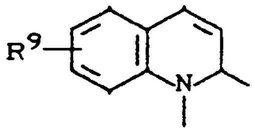
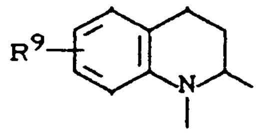
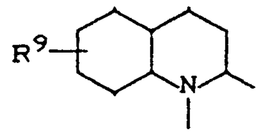

13 14 15

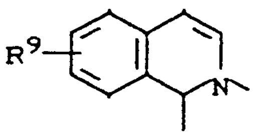
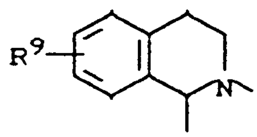
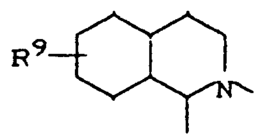

16 17 18

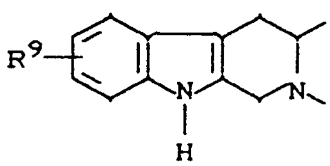
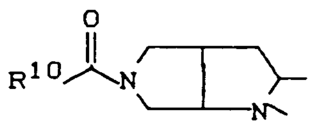
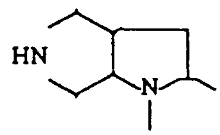

19 20 21

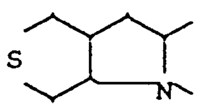
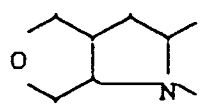
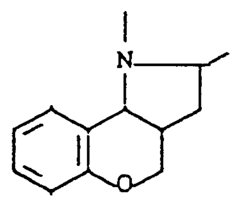

22 23 24

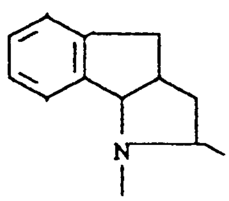
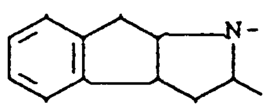
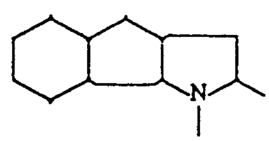

25 26 27

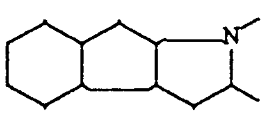
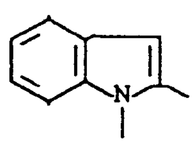

28 29

X für $C_1$-$C_6$-Alkyl, Halogen oder $C_1$-$C_6$-Alkoxy steht,
Y für Wasserstoff, $C_1$-$C_6$-Alkyl, Halogen, $C_1$-$C_6$-Alkoxy, $C_1$-$C_3$-Halogenalkyl steht,
Z für $C_1$-$C_6$-Alkyl, Halogen, $C_1$-$C_6$-Alkoxy steht,
n für eine Zahl von 0 bis 3 steht,
G für Wasserstoff (a) oder für die Gruppen

-CO-$R^1$, (b)

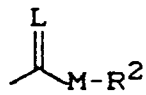 (c)

-$SO_2$-$R^3$ (d)

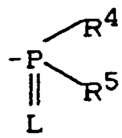 (e)

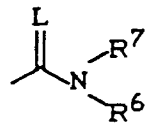 (f)

oder E (g)
steht,

in welchen

E für ein Metallionäquivalent oder ein Ammoniumion steht,

L und M für Sauerstoff und/oder Schwefel steht,

$R^1$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxyl-$C_2$-$C_8$-alkyl oder Cycloalkyl mit 3-8 Ringatomen, das durch Sauerstoff- und/oder Schwefelatome unterbrochen sein kann, steht,
für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl;
für gegebenenfalls durch Halogen, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl-$C_1$-$C_6$-alkyl steht,
für gegebenenfalls durch Halogen und/oder $C_1$-$C_6$-Alkyl substituiertes Pyridyl, Pyrimidyl, Thiazolyl oder Pyrazolyl steht,
für gegebenenfalls durch Halogen und $C_1$-$C_6$-Alkyl-substituiertes Phenoxy-$C_1$-$C_6$-alkyl steht,
für gegebenenfalls durch Halogen, Amino und $C_1$-$C_6$-Alkyl-substituiertes Pyridyloxy-$C_1$-$C_6$-alkyl, Pyrimidyloxy-$C_1$-$C_6$-alkyl oder Thiazolyloxy-$C_1$-$C_6$-alkyl steht,

$R^2$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen, Nitro, $C_1$-$C_6$-Alkyl, $C_1$-$C_6$-Alkoxy, $C_1$-$C_6$-Halogenalkyl substituiertes Phenyl oder Benzyl steht,

$R^3$, $R^4$ und $R^5$ unabhängig voneinander für gegebenenfalls durch Halogen substituiertes $C_1$-$C_8$-Alkyl, $C_1$-$C_8$-Alkoxy, $C_1$-$C_8$-Alkylamino, Di-($C_1$-$C_8$)-Alkylamino, $C_1$-$C_8$-Alkylthio, $C_2$-$C_5$-Alkenylthio, $C_2$-$C_5$-Alkinylthio, $C_3$-$C_7$-Cycloalkylthio, für gegebenenfalls durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl, $C_1$-$C_4$-Halogenalkyl substituiertes Phenyl, Phenoxy oder Phenylthio stehen,

$R^6$ und $R^7$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_{20}$-Halogenalkyl, $C_1$-$C_{20}$-Alkyl oder $C_1$-$C_{20}$-Alkoxy substituiertes Phenyl, für gegebenenfalls durch Halogen, $C_1$-$C_{20}$-Alkyl, $C_1$-$C_{20}$-Halogenalkyl oder $C_1$-$C_{20}$-Alkoxy substituiertes Benzyl steht oder zusammen für einen gegebenenfalls durch Sauerstoff unterbrochenen $C_2$-$C_6$-Alkylenring stehen.

$R^9$ für Wasserstoff, Halogen oder gegebenenfalls durch Halogen substituiertes $C_1$-$C_2$-Alkyl oder $C_1$-$C_2$-Alkoxy steht,

$R^{10}$ für $C_1$-$C_7$-Alkoxy, Amino, $C_1$-$C_4$-Alkylamino, $C_1$-$C_4$-Dialkylamino steht.

Unter Einbeziehung der verschiedenen Bedeutungen (a), (b), (c), (d), (e), (f) und (g) der Gruppe G der allgemeinen Formel (I) ergeben sich folgende hauptsächlichen Strukturen (Ia) bis (Ig):

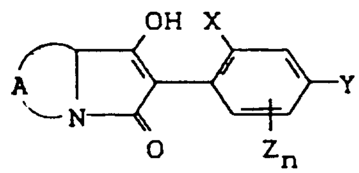

(Ia)

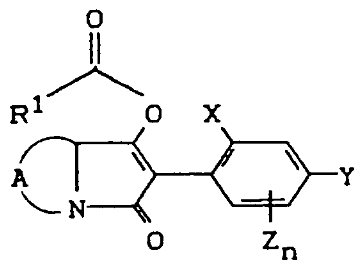

(Ib)

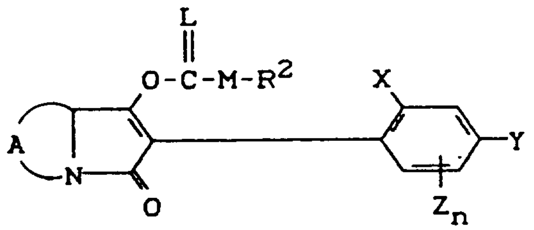

(Ic)

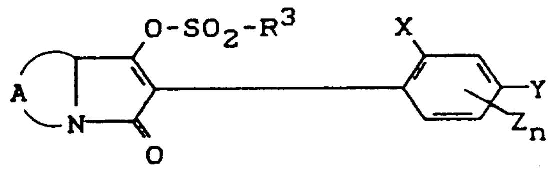

(Id)

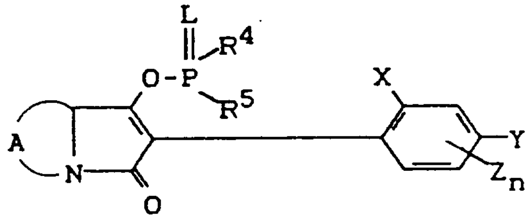

(Ie)

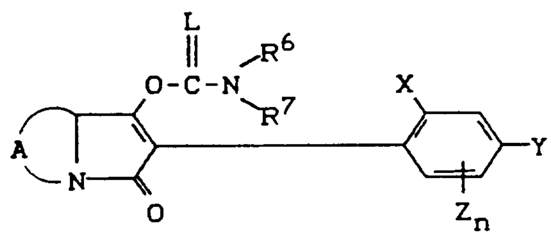

(If)

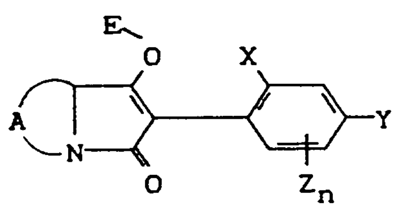

(Ig)

worin

A, E, L, M, X, Y, $Z_n$, $R^1$, $R^2$, $R^3$, $R^4$, $R^5$, $R^6$ und $R^7$ die oben angebenenen Bedeutungen besitzen.

Aufgrund eines oder mehrerer Chiralitätszentren, fallen die Verbindungen der Formel (Ia)-(If) im allgemeinen als Stereoisomerengemisch an. Sie können sowohl in Form ihrer Diastereomerengemische als auch als reine Diastereomere oder Enantiomere verwendet werden.

Weiterhin wurde gefunden, daß man 3-Aryl-pyrrolidon-2,4-dione bzw. deren Enole der Formel (Ia)

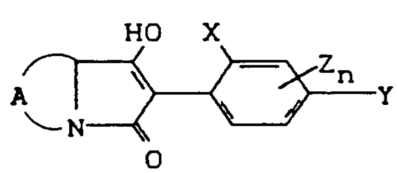

(Ia)

in welcher

A, X, Y, Z und n  die oben angegebene Bedeutung haben,

erhält, wenn man

(A)

N-Acylaminosäureester der Formel (II)

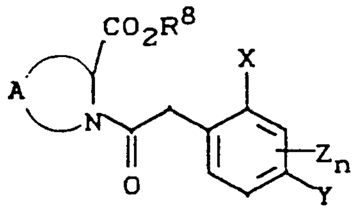

(II)

in welcher

A, X, Y, Z und n die oben angegebene Bedeutung haben

und

$R^8$ für Alkyl steht,

in Gegenwart eines Verdünnungsmittels und in Gegenwart einer Base intramolekular kondensiert.

(B)

Außerdem wurde gefunden, daß man Verbindungen der Formel (Ib)

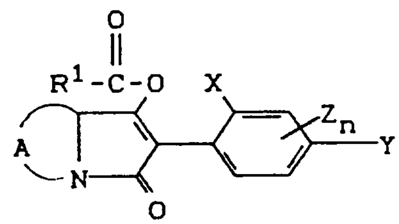

(Ib)

in welcher

A, X, Y, Z, $R^1$ und n die oben angegebene Bedeutung haben,

erhält, wenn man Verbindungen der Formel (Ia),

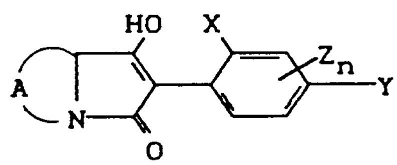

(Ia)

in welcher

A, X, Y, Z und n die oben angegebene Bedeutung haben,

α) mit Säurehalogeniden der allgemeinen Formel (III)

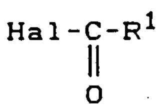

(III)

in welcher

$R^1$ die oben angegebene Bedeutung hat

und

Hal für Halogen, insbesondere Chlor und Brom steht,

gegebenenfalls in Gegenwart eines Verdünnungsmittels und gegebenenfalls in Gegenwart eines Säurebindemittels umsetzt

oder

β) mit Carbonsäureanhydriden der allgemeinen Formel (IV)

$R^1$-CO-O-CO-$R^1$ (IV)

in welcher

$R^1$ die oben angegebene Bedeutung hat,

gegebenenfalls in Gegenwart eines Verdünnungsmittels und gegebenenfalls in Gegenwart eines Säurebindemittels,

umsetzt.

(C)

Ferner wurde gefunden, daß man Verbindungen der Formel (Ic)

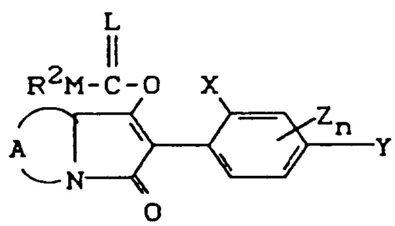

(Ic)

in welcher

A, X, Y, Z, $R^2$ und n die oben angegebene Bedeutung haben,

L für Sauerstoff

und

M für Sauerstoff oder Schwefel steht,

erhält, wenn man Verbindungen der Formel (Ia)

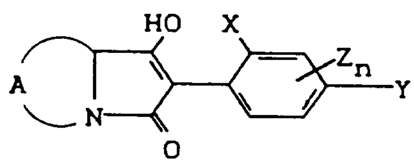

(Ia)

in welcher

A, X, Y, Z und n die oben angegebene Bedeutung haben

mit Chlorameisensäureester oder Chlorameisensäurethiolester der allgemeinen Formel (V)

$R^2$-M-CO-Cl (V)

in welcher

$R^2$ und M die oben angegebene Bedeutung haben,

gegebenenfals in Gegenwart eines Verdünnungsmittels und gegebenenfalls in Gegenwart eines Säurebindemittels umsetzt.

D) Ferner wurde gefunden, daß man Verbindungen der Formel (Ic)

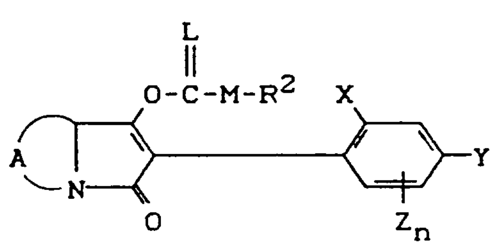

(Ic)

in welcher

A, $R^2$, X, Y, Z und n die oben angegebene Bedeutung haben,

L für Schwefel

und

M für Sauerstoff oder Schwefel steht,

erhält, wenn man Verbindungen der Formel (Ia)

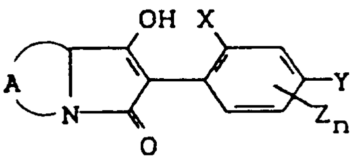

(Ia)

in welcher

A, X, Y, Z und n die oben angegebene Bedeutung haben

α) mit Chlormonothioameisensäureestern oder Chlordithioameisensäureestern der allgemeinen Formel (VI)

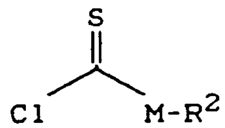

(VI)

in welcher

M und $R^2$ die oben angegebene Bedeutung haben

gegebenenfalls in Gegenwart eines Verdünnungsmittels und gegebenenfalls in Gegenwart eines Säurebindemittels umsetzt,

oder

β) mit Schwefelkohlenstoff und anschließend mit Alkylhalogeniden der allgemeinen Formel (VII)

$R^2$-Hal (VII)

in welcher

$R^2$ die oben angegebene Bedeutung hat

und

Hal für Chlor, Brom, Jod

steht, umsetzt.

E) Außerdem wurde gefunden, daß man Verbindungen der Formel (Id)

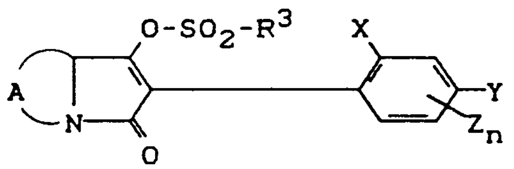

(Id)

in welcher

A, X, Y, Z, $R^3$ und n die oben angegebene Bedeutung haben,

erhält, wenn man Verbindungen der Formel (Ia)

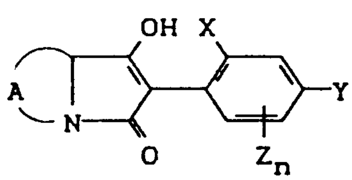

(Ia)

in welcher

A, X, Y, Z und n die oben angegebene Bedeutung haben,

mit Sulfonsäurechloriden der allgemeinen Formel (VIII)

$R^3$-$SO_2$-Cl (VIII)

in welcher

$R^3$ die oben angegebene Bedeutung hat gegebenenfalls in Gegenwart eines Verdünnungsmittels und gegebenenfalls in Gegenwart eines Säurebindemittels,

umsetzt.

F) Weiterhin wurde gefunden, daß man 3-Aryl-pyrrolidin-2,4-dione der Formel (Ie)

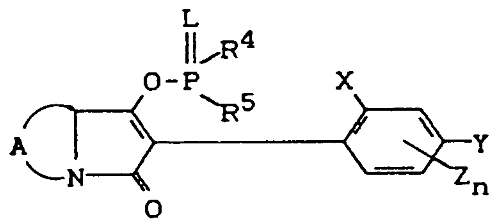

(Ie)

in welcher

A, L, X, Y, Z, $R^4$, $R^5$ und n die oben angegebene Bedeutung haben,

erhält, wenn man

3-Aryl-pyrrolidin-2,4-dione der Formel (Ia) bzw. deren Enole

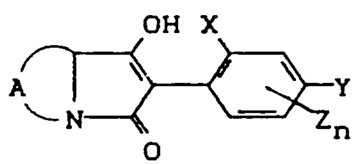

(Ia)

in welcher

A, X, Y, Z und n die oben angegebene Bedeutung haben

mit Phosphorverbindungen der allgemeinen Formel (IX)

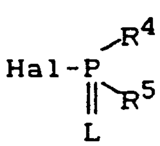

(IX)

in welcher

L, $R^4$ und $R^5$ die oben angegebene Bedeutung haben

und

Hal für Halogen, insbesondere Chlor und Brom steht,

gegebenenfalls in Gegenwart eines Verdünnungsmittels und gegebenenfalls in Gegenwart eines Säurebindemittels umsetzt.

G) Ferner wurde gefunden, daß man Verbindungen der Formel (If)

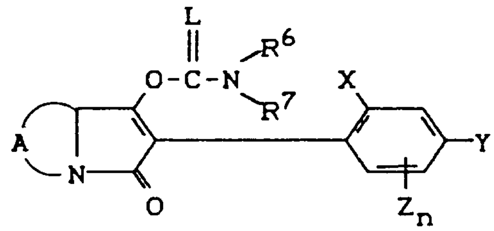

(If)

in welcher
A, L, X, Y, Z, $R^6$, $R^7$ und n die oben angegebene Bedeutung haben,
erhält, wenn man Verbindungen der Formel (Ia),

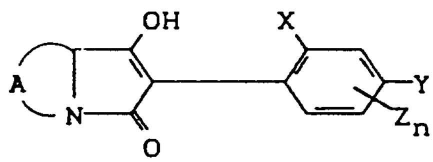

(Ia)

in welcher
A, X, Y, Z und n die oben angegebene Bedeutung haben
α) mit Isocyanaten der allgemeinen Formel (X)

$R^6$-N=C=O (X)

in welcher
$R^6$ die oben angegebene Bedeutung hat
gegebenenfalls in Gegenwart eines Verdünnungsmittels und gegebenenfalls in Gegenwart eines Katalysators umsetzt,
oder
β) mit Carbamidsäurechloriden oder Thiocarbamidsäurechloriden der allgemeinen Formel (XI)

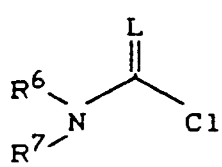

(XI)

in welcher
L, $R^6$ und $R^7$ die oben angegebene Bedeutung haben,
gegebenenfalls in Gegenwart eines Verdünnungsmittels und gegebenenfalls in Gegenwart eines Säurebindemittels,
umsetzt.
H) Weiterhin wurde gefunden, daß man Verbindungen der Formel (Ig)

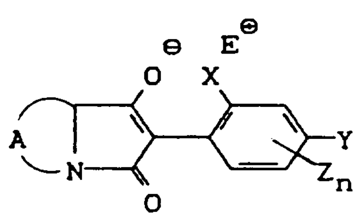

(Ig)

in welcher

X, Y, Z, A und n    die oben angegebene Bedeutung haben,

und E für ein Metallionäquivalent oder für ein Ammoniumion steht, erhält, wenn man Verbindungen der Formel (Ia)

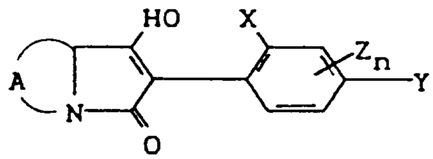

(Ia)

in welcher

X, Y, Z, A, und n    die oben angegebene Bedeutung haben,

mit Metallhydroxiden oder Aminen der allgemeinen Formeln (XII) und (XIII)

$Me_sOH_t$    (XII)

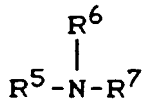

(XIII)

in welchen

| | |
|---|---|
| Me | für ein- oder zweiwertige Metallionen |
| s und t | für die Zahl 1 und 2 und |
| $R^5$, $R^6$ und $R^7$ | unabhängig voneinander für Wasserstoff und Alkyl |

stehen,

gegebenenfalls in Gegenwart eines Verdünnungsmittels, umsetzt.

Weiterhin wurde gefunden, daß sich die neuen polycyclischen 3-Arylpyrrolidin-2,4-dion-Derivate der Formel (I) durch hervorragende insektizide, akarizide und herbizide Wirkungen auszeichnen.

Bevorzugt sind polycyclische 3-Arylpyrrolidin-2,4-dion-Derivate der Formel (I) in welcher die Gruppierung

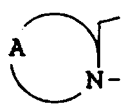

eine der folgenden Bedeutungen 1 bis 29 annimmt:

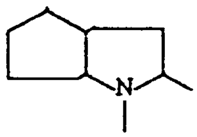

1

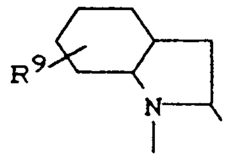

2

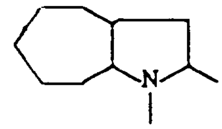

3

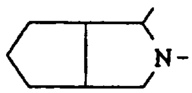

4

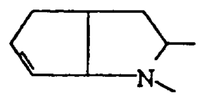

5

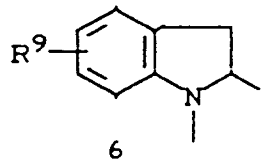

6

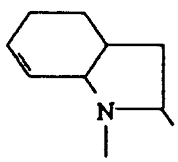

7

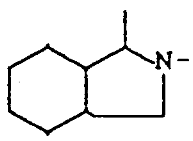

8

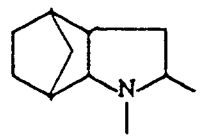

9

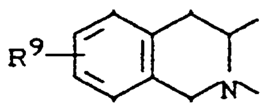

10

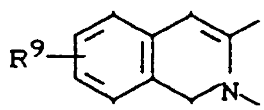

11

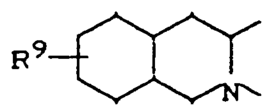

12

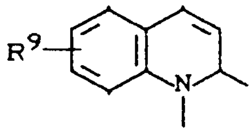

13

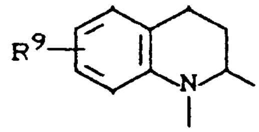

14

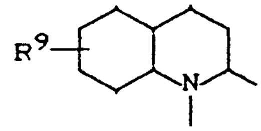

15

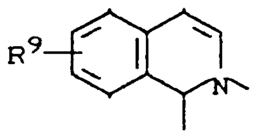

16

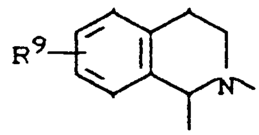

17

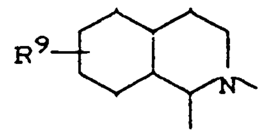

18

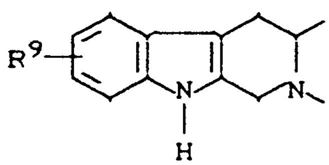

19

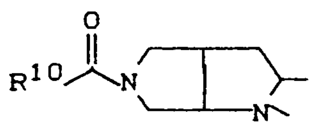

20

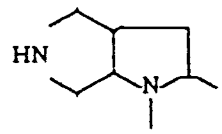

21

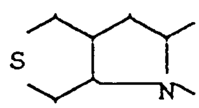

22

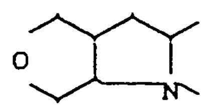

23

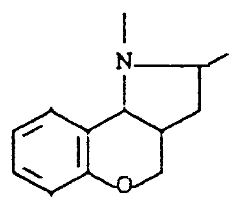

24

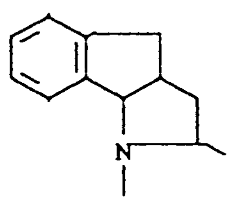

25

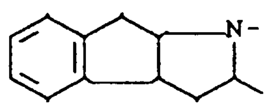

26

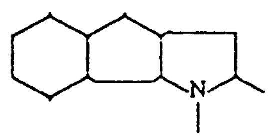

27

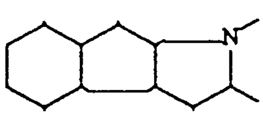

28

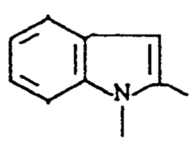

29

| | |
|---|---|
| X | für $C_1$-$C_4$-Alkyl, Halogen, $C_1$-$C_4$-Alkoxy steht, |
| Y | für Wasserstoff, $C_1$-$C_4$-Alkyl, Halogen, $C_1$-$C_4$-Alkoxy, $C_1$-$C_2$-Halogenalkyl steht, |
| Z | für $C_1$-$C_4$-Alkyl, Halogen, $C_1$-$C_4$-Alkoxy steht, |
| n | für eine Zahl von 0-3 steht, |
| G | für Wasserstoff (a) oder für die Gruppen |

-CO-$R^1$, (b)

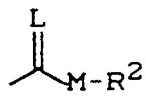 (c)

-$SO_2$-$R^3$ (d)

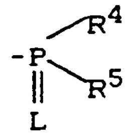 (e) 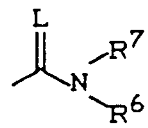 (f)

oder E (g) steht,
in welchen

| | |
|---|---|
| E | für ein Metallionäquivalent oder ein Ammoniumion steht |
| L und M | jeweils für Sauerstoff und/oder Schwefel steht, |
| $R^1$ | für gegebenenfalls durch Halogen substituiertes $C_1$-$C_{16}$-Alkyl, $C_2$-$C_{16}$-Alkenyl, $C_1$-$C_6$-Alkoxy-$C_2$-$C_6$-alkyl, $C_1$-$C_{16}$-Alkylthio-$C_2$-$C_6$-alkyl, $C_1$.$C_6$-Polyalkoxy-$C_2$-$C_6$-alkyl und Cycloalkyl mit 3-7 Ringatomen, das durch 1-2 Sauerstoff- und/oder Schwefelatome unterbrochen sein kann steht,<br>für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy substituierets Phenyl steht,<br>für gegebenenfalls durch Halogen, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl-$C_1$-$C_4$-alkyl steht,<br>für gegebenenfalls durch Halogen- und $C_1$-$C_6$-Alkyl-substituiertes Pyridyl, Pyrimidyl, Thiazolyl oder Pyrazolyl steht,<br>für gegebenenfalls durch Halogen- und $C_1$-$C_4$-Alkyl-substituiertes Phenoxy-$C_1$-$C_5$-alkyl steht,<br>für gegebenenfalls durch Halogen, Amino und $C_1$-$C_4$-Alkyl substituiertes Pyridyloxy-$C_1$-$C_5$-alkyl, Pyrimidyloxy-$C_1$-$C_5$-alkyl oder Thiazolyloxy-$C_1$-$C_5$-alkyl steht, |
| $R^2$ | für gegebenenfalls durch Halogen substituiertes: $C_1$-$C_{16}$-Alkyl, $C_2$-$C_{16}$-Alkenyl, $C_1$-$C_{16}$-Alkox-$C_2$-$C_6$-alkyl, $C_1$-$C_6$-Polyalkoxy-$C_2$-$C_6$-alkyl steht,<br>für gegebenenfalls durch Halogen, Nitro, $C_1$-$C_4$-Alkyl, $C_1$-$C_3$-Alkoxy, $C_1$-$C_3$-Halogenalkyl substituiertes Phenyl oder Benzyl steht, |
| $R^3$, $R^4$ und $R^5$ | unabhängig voneinander für gegebenenfalls durch Halogen substituiertes $C_1$-$C_6$-Alkyl, $C_1$-$C_6$-Alkoxy, $C_1$-$C_6$-Alkylamino, Di-($C_1$-$C_6$)-Alkylamino, $C_1$-$C_6$-Alkylthio, $C_3$-$C_4$-Alkenylthio, $C_2$-$C_4$-Alkinylthio, $C_3$-$C_6$-Cycloalkylthio, für gegebenenfalls durch Fluor, Chlor, Brom, Nitro, Cyano, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkyl, $C_1$-$C_3$-Halogenalkyl substiutiertes Phenyl, Phenoxy oder Phenylthio stehen, |
| $R^6$ und $R^7$ | unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_5$-Halogenalkyl, $C_1$-$C_5$-Alkyl oder $C_1$-$C_5$-Alkoxy substituiertes Phenyl, für gegebenenfalls durch Halogen, $C_1$-$C_5$-Alkyl,$C_1$-$C_5$-Halogenalkyl oder $C_1$-$C_5$-Alkoxy substituiertes Benzyl steht, |
| $R^9$ | für Wasserstoff, Halogen oder gegebenenfalls durch Halogen substituiertes $C_1$-$C_2$-Alkyl oder $C_1$-$C_2$-Alkoxy steht, |
| $R^{10}$ | für $C_1$-$C_7$-Alkoxy, Amino, $C_1$-$C_4$-Alkylamino, $C_1$-$C_4$-Dialkylamino steht. |

Besonders bevorzugt sind Verbindungen der Formel (I) in welcher die Gruppierung

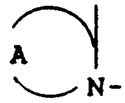

eine der folgenden Bedeutungen 1 bis 29 annimmt:

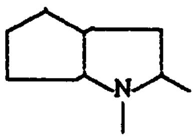

1

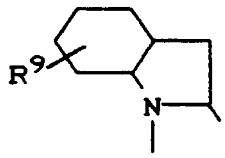

2

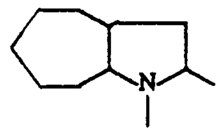

3

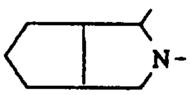

4

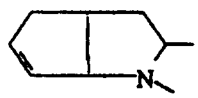

5

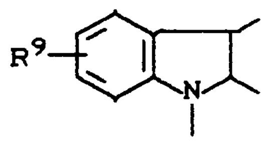

6

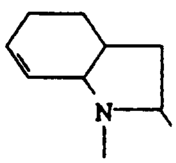

7

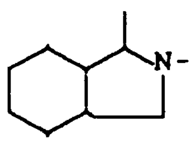

8

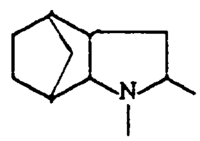

9

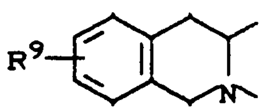

10

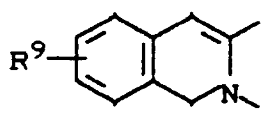

11

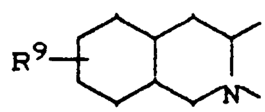

12

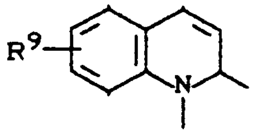
13

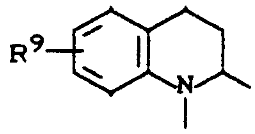
14

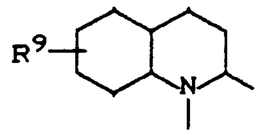
15

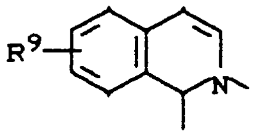
16

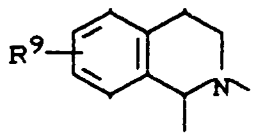
17

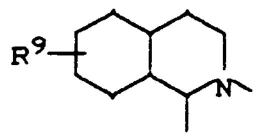
18

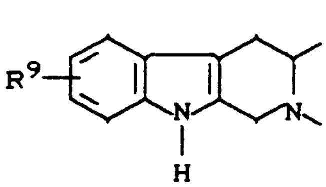
19

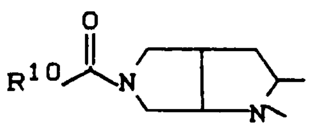
20

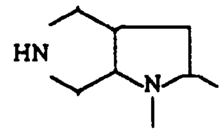
21

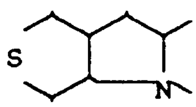
22

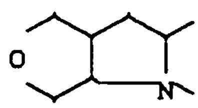
23

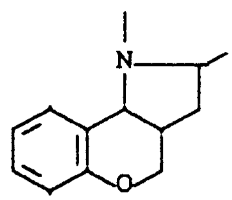
24

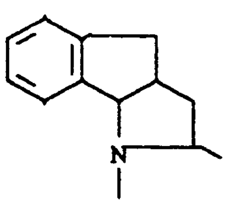
25

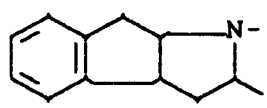
26

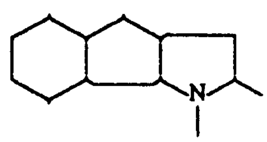
27

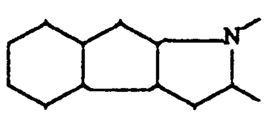
28

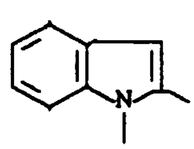
29

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,

Z für Methyl, Ethyl, i-Propyl, Butyl, i-Butyl, tert.-Butyl, Fluor, Chlor, Brom, Methoxy und Ethoxy steht,

n für eine Zahl von 0-3 steht,

G für Wasserstoff (a) oder für die Gruppen

-CO-$R^1$, (b)

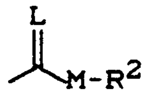 (c)

-$SO_2$-$R^3$ (d)

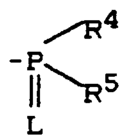 (e) 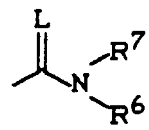 (f)

oder E (g)

steht,

in welchen

E für ein Metallionäquivalent oder ein Ammoniumion steht,

L und M für Sauerstoff und/oder Schwefel steht,

$R^1$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Alkylthio-$C_2$-$C_6$-alkyl, $C_1$-$C_4$-Polyalkoxy-$C_2$-$C_4$-alkyl und Cycloalkyl mit 3-6 Ringatomen, das durch 1-2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro-substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy-substituiertes Phenyl-$C_1$-$C_3$-alkyl steht,
für gegebenenfalls durch Fluor, Chlor, Brom, Methyl, Ethyl-substituiertes Pyridyl, Pyrimidyl, Thiazolyl und Pyrazolyl steht,
für gegebenenfalls durch Fluor, Chlor, Methyl, Ethyl-substituiertes Phenoxy-$C_1$-$C_4$-alkyl steht,
für gegebenenfalls durch Fluor, Chlor, Amino, Methyl-, Ethyl, substituiertes Pyridyloxy-$C_1$-$C_4$-alkyl, Pyrimidyloxy-$C_1$-$C_4$-alkyl und Thiazolyloxy-$C_1$ -$C_4$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,

$R^3$, $R^4$ und $R^5$ unabhängig voneinander für gegebenenfalls durch Fluor oder Chlor substituiertes $C_1$-$C_4$-Alkyl, $C_1$-$C_4$-Alkoxy, $C_1$-$C_4$-Alkylamino, Di-($C_1$-$C_4$-Alkyl)-amino, $C_1$-$C_4$-Alkylthio, für gegebenenfalls durch Fluor, Chlor, Brom, Nitro, Cyano, $C_1$-$C_2$-Alkoxy, $C_1$-$C_4$-Fluoralkoxy, $C_1$-$C_2$-Chloralkoxy, $C_1$-$C_2$-Alkylthio, $C_1$-$C_2$-Fluoralkylthio, $C_1$-$C_2$-Chloralkylthio, $C_1$-$C_3$-Alkyl substituiertes Phenyl, Phenoxy oder Phenylthio stehen,

$R^6$ und $R^7$ unabhängig voneinander für gegebenenfalls durch Fluor, Chlor, Brom substituiertes $C_1$-$C_{10}$-Alkyl, $C_1$-$C_{10}$-Alkoxy, $C_1$-$C_{10}$-Alkoxy-($C_1$-$C_{10}$)alkyl, für gegebenenfalls durch Fluor, Chlor, Brom, $C_1$-$C_{20}$-Halogenalkyl, $C_1$-$C_{20}$-Alkyl oder $C_1$-$C_4$-Alkoxy substituiertes Phenyl, für gegebenenfalls durch Fluor, Chlor, Brom, $C_1$-$C_4$-Alkyl, $C_1$-$C_4$-Halogenalkyl oder $C_1$-$C_4$-Alkoxy substituiertes Benzyl steht,

$R^9$ für Wasserstoff, Halogen oder gegebenenfalls durch Halogen substituiertes $C_1$-$C_2$-Alkyl oder $C_1$-$C_2$-Alkoxy steht,

$R^{10}$ für Methoxy, Ethoxy, Propoxy, iso-Propoxy, Butoxy, iso-Butoxy, sec.-Butoxy, tert.-Butoxy, Phenoxy, Benzyloxy, Amino, $C_1$-$C_2$-Alkylamino, $C_1$-$C_2$-Dialkylamino steht.

Verwendet man gemäß Verfahren (A) N-2,6-Dichlorphenylacetyl-perhydrochinolin-2-carbonsäureethylester, so kann der Verlauf des erfindungsgemäßen Verfahrens durch folgendes Reaktionsschema wiedergegeben werden:

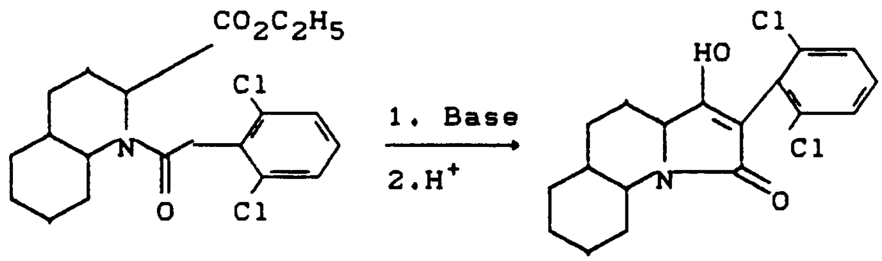


Verwendet man gemäß Verfahren (B) (Variante $\alpha$) 10-(2,4,6 Trimethylphenyl)-1-aza-tricyclo(6,3,$0^{1.8}$,$0^{2.6}$)-undecan-9,11-dion und Pivaloylchlorid als Ausgangsstoff, so kann der Verlauf des erfindungsgemäßen Verfahrens durch folgendes Reaktionsschema wiedergegeben werden.

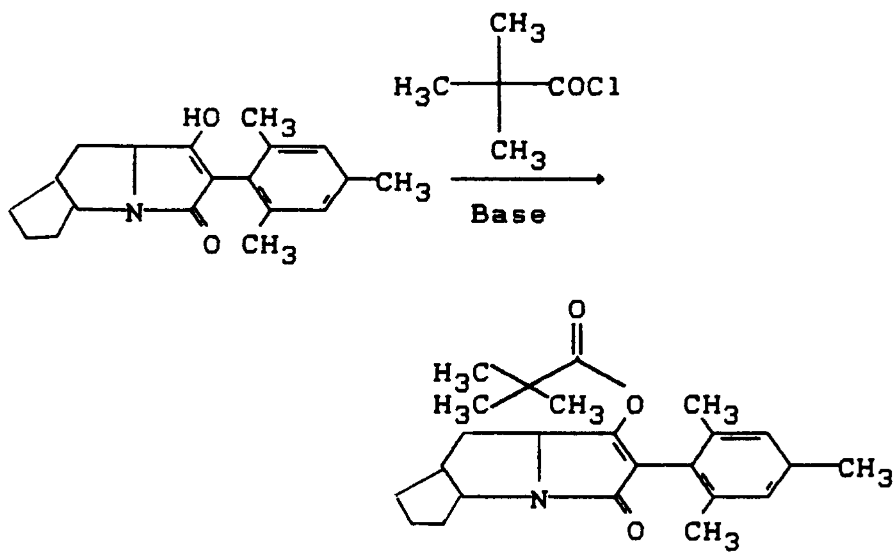


Verwendet man gemäß Verfahren B (Variante $\beta$) 12-(2,4,5-Trimethylphenyl)-1-aza-tricyclo-(8,3,$0^{1.10}$,$0^{3.8}$)-tridecan-11,13-dion und Acetanhydrid als Ausgangsverbindungen, so kann der Verlauf des erfindungsgemäßen Verfahrens durch folgendes Reaktionsschema wiedergegeben werden.

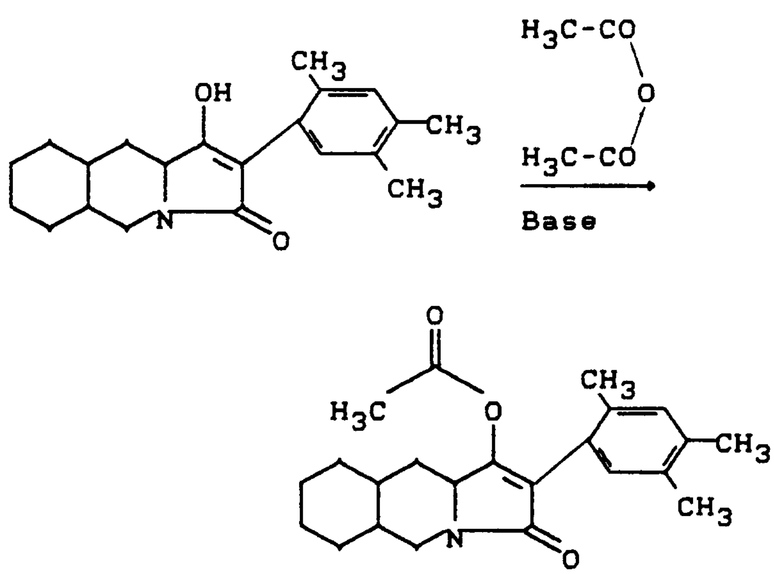

Verwendet man gemäß Verfahren C 12-(2,4-Dichlorphenyl)-1-aza-tricyclo-(8,3,$0^{1.10}$,$0^{2.7}$)-tridecan-11,13-dion und Chlorameisensäureethoxyethylester als Ausgangsverbindungen, so kann der Verlauf des erfindungsgemäßen Verfahrens durch folgendes Reaktionsschema wiedergegeben werden.

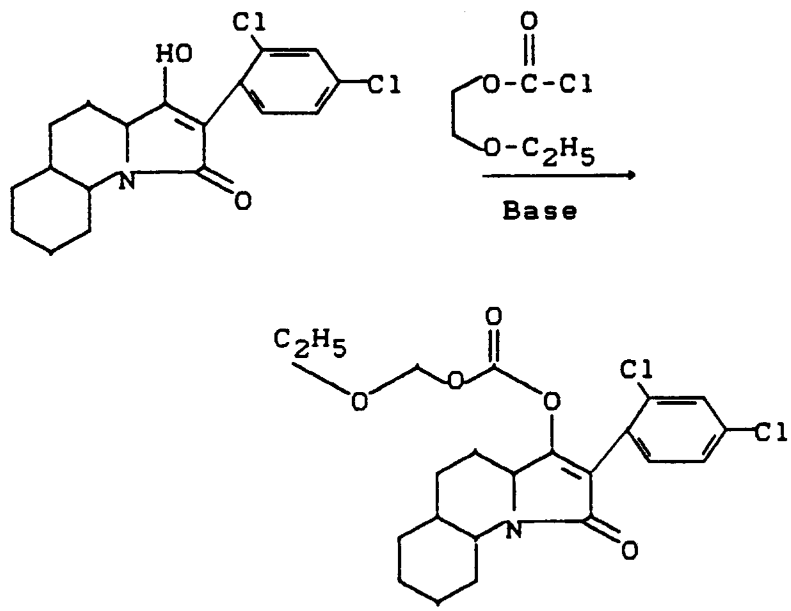


Verwendet man gemäß Verfahren ($D_\alpha$) 11-(2,4,6-Trimethylphenyl)-1-aza-tricyclo-(7,3,$0^{1.9}$,$0^{3.8}$)-dodecan-10,12-dion und Chlormonothioameisensäuremethylester als Ausgangsprodukte, so kann der Reaktionsverlauf wie folgt wiedergegeben werden:

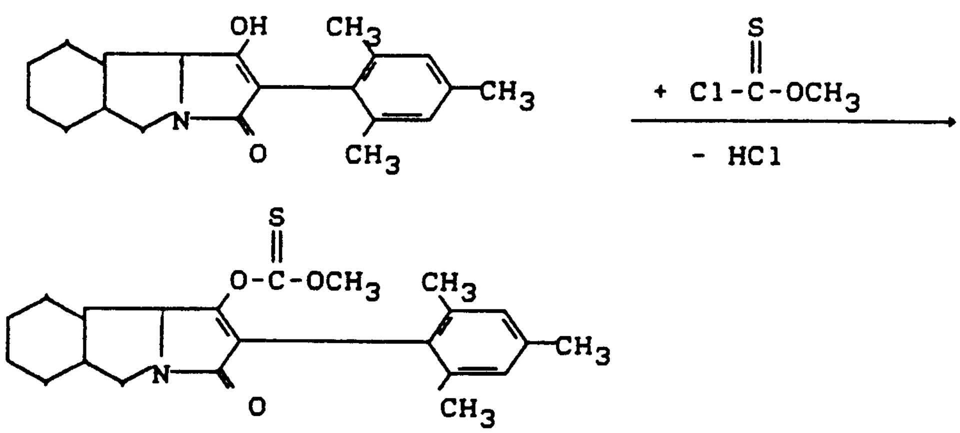


Verwendet man gemäß Verfahren ($D_\beta$) 11-(2,4,6-Trimethylphenyl)-1-aza-tricyclo-(7,3,$0^{1.8}$,$0^{2.7}$)-dodecan-10,12-dion, Schwefelkohlenstoff und Methyliodid als Ausgangskomponenten, so kann der Reaktionsverlauf wie folgt wiedergegeben werden:

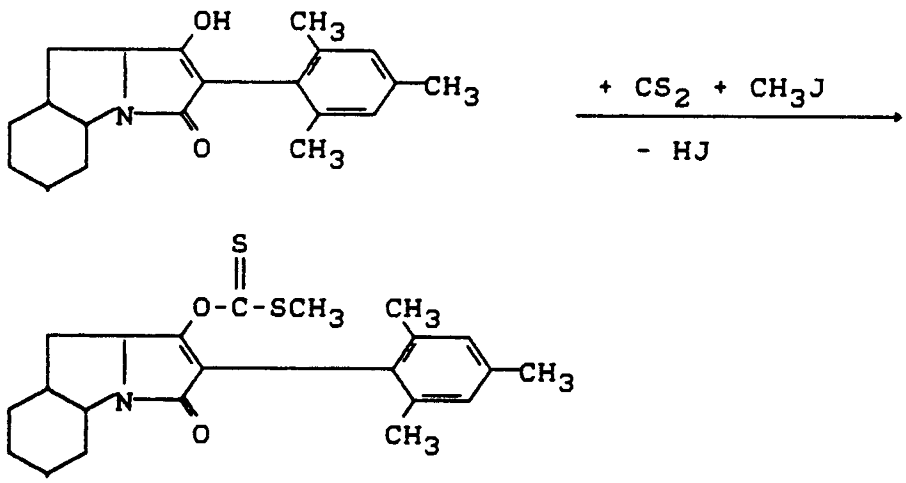


Verwendet man gemäß Verfahren (E) 10-(2,4,6-Trimethylphenyl)-1-aza-tricyclo-($6,3,0^{1.8},0^{2.6}$)-undecan-9,11-dion und Methansulfonsäurechlorid als Ausgangsprodukt, so kann der Reaktionsverlauf durch folgendes Reaktionsschema wiedergegeben werden:

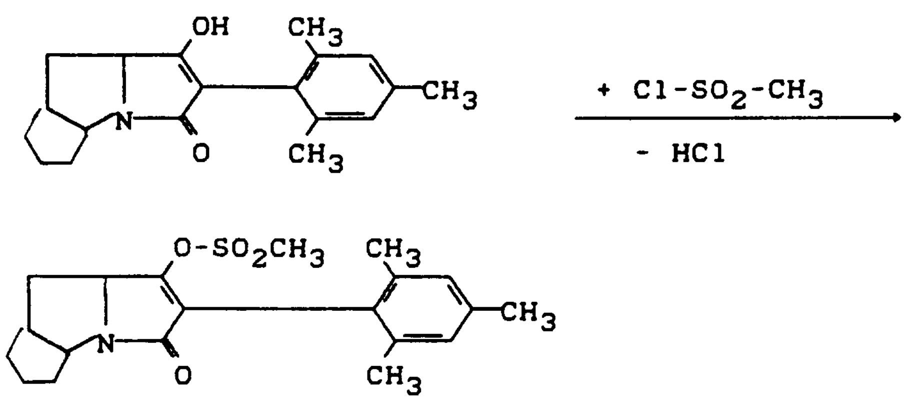


Verwendet man gemäß Verfahren (F) 10-(2,4,6-Trimethylphenyl)-1,4-diaza-(4-N-ethoxycarbonyl)-tricyclo-($6,3,0^{1.8},0,^{2.6}$)-undecan-9,11-dion und Methanthiophosphonsäurechlorid-(2,2,2-trifluorethylester) als Ausgangsprodukte, so kann der Reaktionsverlauf durch folgendes Reaktionsschema wiedergegeben werden:

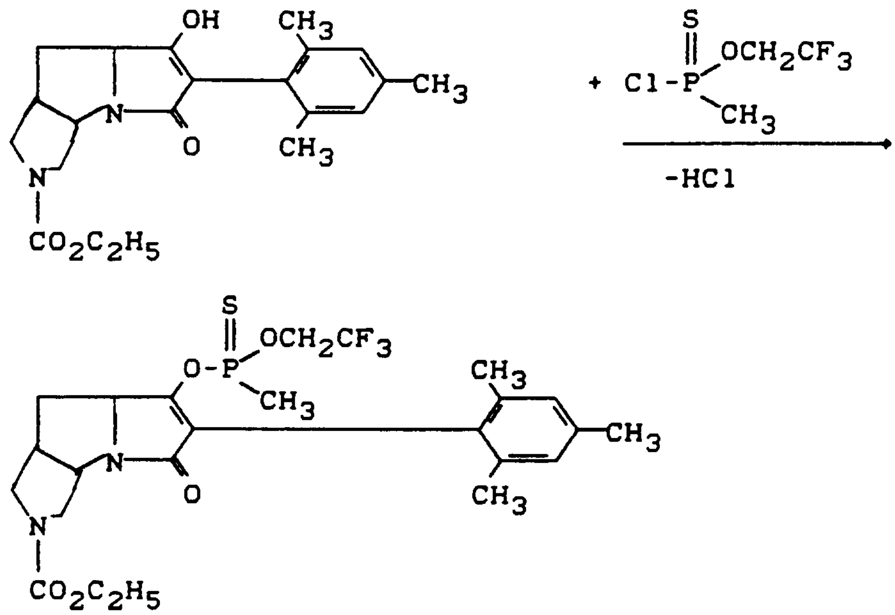


Verwendet man gemäß Verfahren ($G_\alpha$) 10-(2,4,6-Trimethylphenyl)-1-aza-tricyclo-(6,3,$0^{1.8}$,$0^{2.6}$)-undecan-9,11-dion und Ethylisocyanat als Ausgangsprodukte, so kann der Reaktionsverlauf durch folgendes Reaktionsschema wiedergegeben werden:

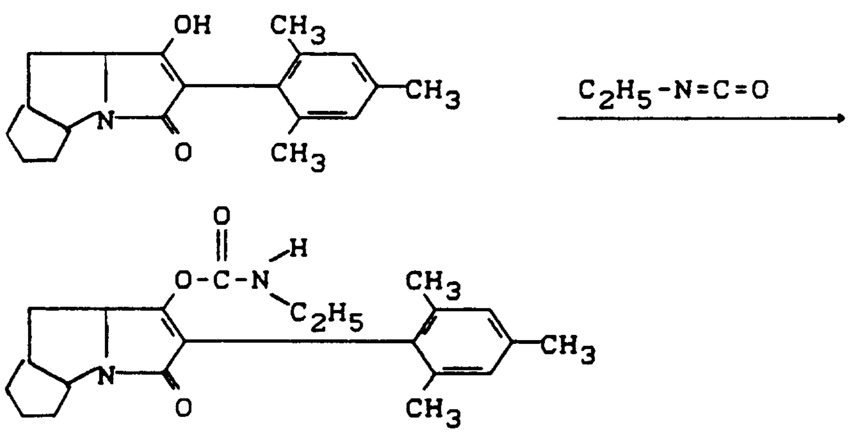


Verwendet man gemäß Verfahren ($G_\beta$) 11-(2,4,6-Trimethylphenyl)-1-aza-tricyclo-(7,3,$0^{1.9}$,$0^{2.7}$)-dodecan-10,12-dion und Dimethylcarbamidsäurechlorid als Ausgangsprodukte, so kann der Reaktionsverlauf durch folgendes Schema wiedergegeben werden:

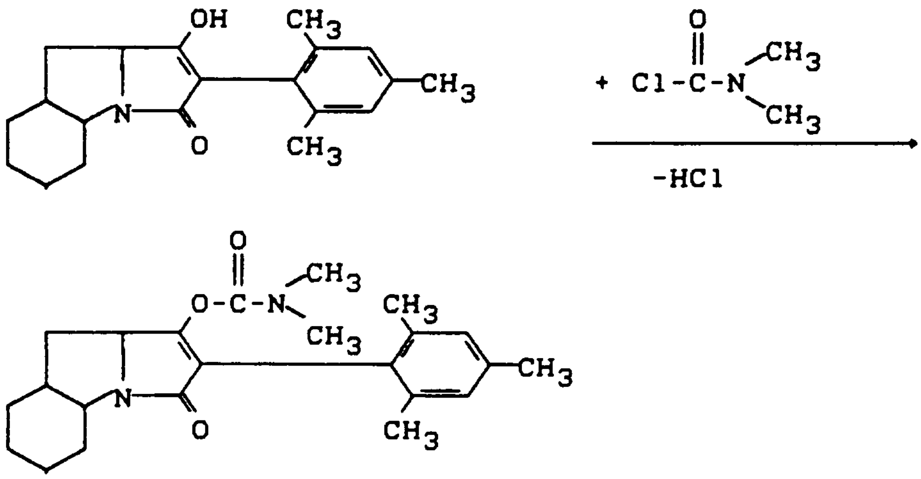


Verwendet man gemäß Verfahren (H) 10-(2,4,6-Trimethylphenyl)-1-aza-tricyclo-(6,3,$0^{1,8}$,$0^{2,6}$)-undecan-9,11-dion und NaOH als Komponenten, so kann der Verlauf des erfindungsgemäßen Verfahrens durch folgendes Reaktionsschema wiedergegeben werden:

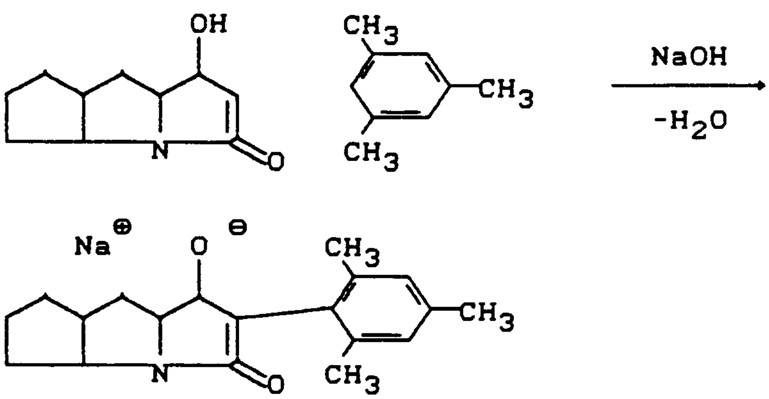


Die bei dem obigen Verfahren (A) als Ausgangsstoffe benötigten Verbindungen der Formel (II)

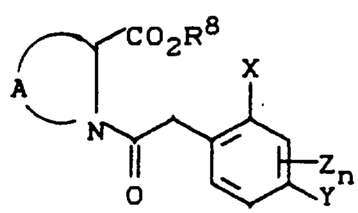

(II)

in welcher

A, X, Y, Z, n und $R^8$ die oben angegebene Bedeutung haben sind bekannt oder lassen sich nach im Prinzip bekannten Methoden in einfacher Weise herstellen. So erhält man z.B. Acyl-aminosäureester der Formel (II), wenn man

a) Aminosäureester der Formel (XIV)

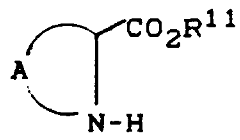

(XIV)

in welcher
$R^{11}$ für Wasserstoff (XIVa) oder Alkyl (XIVb) steht
und
A die oben angegebene Bedeutung hat,
mit Phenylessigsäurehalogeniden der Formel (XV)

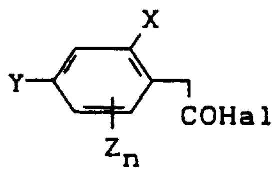

(XV)

in welcher
X, Y, Z und n die oben angegebene Bedeutung haben und
Hal für Chlor oder Brom steht,
acycliert (Chem, Reviews 52 237-416 (1953));
oder wenn man Acylaminosäuren der Formel (IIa),

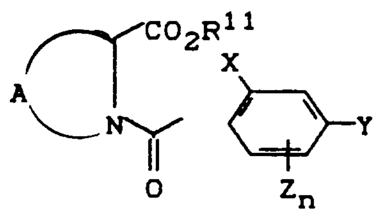

(IIa)

in welcher
A, X, Y, Z und n die oben angegebene Bedeutung haben
und
$R^{11}$ für Wasserstoff steht,
verestert (Chem. Ind. (London) 1568 (1968).

Verbindungen der Formel (IIa) sind beispielsweise aus den Phenylessigsäurehalogeniden der Formel (XV) und Aminosäuren der Formel XIV a) nach Schotten-Baumann (Organikum 9. Auflage 446 (1970) VEB Deutscher Verlag der Wissenschaften, Berlin) erhältlich.

Beispielhaft seien folgende Verbindungen der Formel (II) genannt:

1. 2-Aza-bicyclo-(3,3,0)-octan-N-(2,4-dichlorphenylacetyl)-3-carbonsäureethylester
2. 2-Aza-bicyclo-(3,3,0)-octan-N-(2,6-dichlorphenylacetyl)-3-carbonsäureethylester
3. 2-Aza-bicyclo-(3,3,0)-octan-N-(2,4,6-trichlorphenylacetyl-3-carbonsäureethylester
4. 2-Aza-bicyclo-(3,3,0)-octan-N-(2,4-dimethylphenylacetyl)-3-carbonsäureethylester
5. 2-Aza-bicyclo-(3,3,0)-octan-N-(2,6-dimethylphenylacetyl)-3-carbonsäureethylester
6. 2-Aza-bicyclo-(3,3,0)-octan-N-(2,4,6-trimethylphenylacetyl)-3-carbonsäureethylester
7. 7-Aza-bicyclo-(4,3,0)-nonan-N-(2,4,6-trimethylphenylacetyl)-8-carbonsäureethylester
8. 8-Aza-bicyclo-(5,3,0)-decan-N-(2,4,6-trimethylphenylacetyl)-9-carbonsäureethylester
9. 3-Aza-bicyclo-(3,3,0)-octan-N-(2,4,6-trimethylphenylacetyl)-2-carbonsäureethylester
10. 3-Aza-bicyclo(3,3,0)-octan-7-en-N-(2,4,6-trimethylphenylacetyl)-3-carbonsäureethylester
11. Indolin-N-(2,4,6-trimethylphenyl-acetyl)-2-carbonsäureethylester

12. 9-Aza-bicyclo-(4,3,0)-non-2-en-N-(2,4,6-trimethylphenyl-acetyl)-8-carbonsäureethylester
13. 8-Aza-bicyclo-(4,3,0)-nonan-N-(2,4,6-trimethylphenyl-acetyl)-7-carbonsäureethylester
14. 7-Aza-tricyclo-(4,3,$1^{2.5}$,$0^{1.6}$)-decan-N-(2,4,6-trimethylphenyl-acetyl)-8-carbonsäureethylester
15. 1,2-Dihydrochinolin-N-(2,4,6-trimethylphenylacetyl-2-carbonsäureethylester
16. 1,2,3,4-Tetrahydrochinolin-N-(2,4,6-trimethylphenylacetyl-2-carbonsäureethylester
17. Perhydrochinolin-N-(2,4,6-trimethylphenyl-acetyl)-2-carbonsäureethylester
18. 1,2-Dihydroisochinolin-N-(2,4,6-trimethylphenylacetyl)-2-carbonsäureethylester
19. 1,2,7,8-Tetrahydroisochinolin-N-(2,4,6-trimethylphenyl-acetyl-2-carbonsäureethylester
20. Perhydroisochinolin-N-(2,4,6-trimethylphenylacetyl)-2-carbonsäureethylester
21. Perhydroindol-N-(2,4,6-trimethylphenyl-acetyl)-2-carbonsäureethylester
22. 2,7-Diaza-7-(ethoxycarbonyl)-bicyclo-(3,3,0)-octan-N-(2,4,6-trimethylphenyl-acetyl)-3-carbonsäureethylester
23. 10-Aza-tricyclo-(7,3,$0^{1.9}$,$0^{3.8}$)-dodecan-(2,4,6-trimethylphenyl-acetyl)-11-carbonsäureethylester
24. 10-Aza-tricyclo-(7,3,$0^{1.9}$,$0^{2.7}$)-dodecan-(2,4,6-trimethylphenyl-acetyl)-11-carbonsäureethylester.

Verbindungen der Formel XIV, in welcher

A, B und $R^{11}$ die oben angegebene Bedeutung haben sind bekannt oder nach literaturbekannten Verfahren erhältlich (Henning, R., Urbach, H., Tetrahedron Lett. 24, 5339-5342 (1983); EP-A-52 870; US-A-4 291 163; EP-A-173-199 ; Urbach, H. Henning, R. Tetrahedron Lett. 26, 1839-1842 (1985).

Das Verfahren (A) ist dadurch gekennzeichnet, daß Verbindungen der Formel (II) in welcher A, X, Y, Z, n und $R^8$ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464oder TDA 1eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0 °C und 250 °C, vorzugsweise zwischen 50 °C und 150 °C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Adogen 464 = Methyltrialkyl($C_8$-$C_{10}$)ammoniumchlorid

TDA 1 = Tris-(methoxyethoxyethyl)-amin

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hüning-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20 °C und +100 °C, vorzugsweise zwischen 0 °C und 50 °C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren $D_\alpha$ setzt man pro Mol Ausgangsverbindung der Formel (Ia) ca. 1 Mol Chlormonothioameisensäureester bzw. Chlordithioameisensäureester der Formel (VII) bei 0 bis 120 °C, vorzugsweise bei 20 bis 60 °C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren $D_\beta$ setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50 °C und insbesondere bei 20 bis 30 °C.

Oft ist es zweckmäßig zunächst aus der Verbindung der Formel (II) durch Zusatz eines Deprotonierungsmittels (wie z.B. Kaliumtertiärbutylat oder Natriumhydrid) das entsprechende Salz herzustellen. Man setzt die Verbindung (II) solange mit Schwefelkohlenstoff um bis die Bildung der Zwischenverbindung abgeschlossen ist, z.B. nach mehrstündigem Rühren bei Raumtemperatur.

Die weitere Umsetzung mit dem Alkylhalogenid der Formel (VIII) erfolgt vorzugsweise bei 0 bis 70 °C und insbesondere bei 20 bis 50 °C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren E) setzt man pro Mol Ausgangsverbindung der Formel (Ia) ca. 1 Mol Sulfonsäurechlorid (VIII) bei 0 bis 150 °C, vorzugsweise bei 20 bis 70 °C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weitern Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren E kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel a) 0,3 bis 1,5 mol Sulfonsäurechlorid VIII, bevorzugt 0,5 mol bei 0 ° bis 150 °C, vorzugsweise bei 20 bis 70 °C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen -40 °C und 150 °C, vorzugsweise zwischen -10 und 110 °C um.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen

Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren $G_\alpha$ setzt man pro Mol Ausgangsverbindung der Formel Ia ca. 1 Mol Isocyanat der Formel (X) bei 0 bis 100 °C, vorzugsweise bei 20 bis 50 °C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden, Es wird vorzugsweise bei Normaldruck gearbeitet.

Beim Herstellungsverfahren $G_\beta$ setzt man pro Mol Ausgangsverbindung der Formel (Ia) ca. 1 Mol Carbamidsäurechlorid bzw. Thiocarbamidsäurechlorid der Formel (XI) bei 0 bis 150 °C, vorzugsweise bei 20 bis 70 °C um.

Als gegebenenfalls zugesetzte Verdünnungsmittel kommen aller inerten polaren organischen Lösungsmittel in Frage wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugswiese wird bei Normaldruck gearbeitet. Die Aufarbeitung geschieht nach üblichen Methoden.

Das Verfahren (H) ist dadurch gekennzeichnet, daß man Verbindungen der Formel (Ia) mit Metallhydroxiden (XII) oder Aminen (XIII) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperaturen liegen im allgemeinen zwischen -20 °C und 100 °C, vorzugsweise zwischen 0 °C und 50 °C.

Bei der Durchführung des erfindungsgemäßen Verfahrens (H) werden die Ausgangsstoffe der Formel (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

Beispiel 1

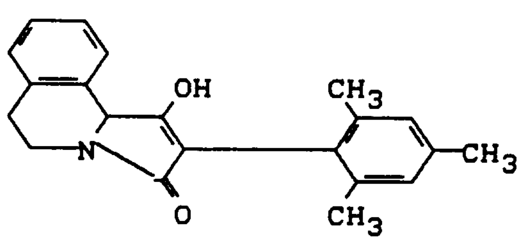

3,6 g (0,12 Mol) Natriumhydrid (80%ige Suspension) werden in 60 ml abs. Toluol vorgelegt. Unter Rückfluß werden 30,9 g (0,085 Mol) 1.2.7.8-Tetrahydroisochinolin-N-(2,4,6-trimethylphenyl-acetyl)-2-carbonsäureethylester gelöst in 90 ml abs. Toluol zugetropft und unter Dünnschicht-Chromatographie-Kontrolle weitergerührt. Nach Beendigung der Reaktion wird überschüssiges Natriumhydrid mit Ethanol vernichtet und der Ansatz einrotiert. Den Rückstand nimmt man in Wasser auf, säuert mit konzentrierter Salzsäure bei 0-20 °C an, saugt ab und trocknet im Vakuum über $P_2O_5$. Die Reinigung erfolgt durch Auskochen mit Methyl-tert.-butyl-Ether/n-Hexan. Auf diese Weise erhält man 19,43 g (≙ 71,1 % der Theorie) 1-Aza-12-(2,4,6-trimethylphenyl)-tricyclo-$(8,3,0^{1.10},0^{4.9})$-tridecan-4,6,8-trien-11,13-dion vom Schmelzpunkt 78 °C.

Analog wurden erhalten:

**Tabelle 1**

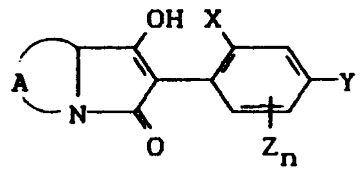

(Ia)

| Bsp. Nr. | 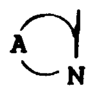 | X | Y | $Z_n$ | Fp.°C |
|---|---|---|---|---|---|
| 2 | 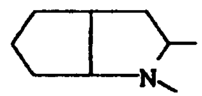 | Cl | Cl | H | |
| 3 | 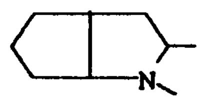 | Cl | H | 6-Cl | |
| 4 | 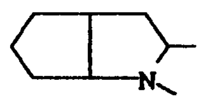 | $CH_3$ | $CH_3$ | H | |
| 5 | 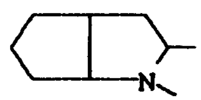 | $CH_3$ | H | 6-$CH_3$ | |
| 6 | 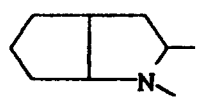 | $CH_3$ | $CH_3$ | 6-$CH_3$ | > 230 |

**Tabelle 1** (Fortsetzung)

| Bsp. Nr. | 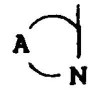 | X | Y | $Z_n$ | Fp.°C |
|---|---|---|---|---|---|
| 7 | 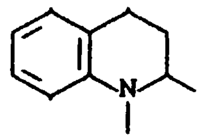 | $CH_3$ | $CH_3$ | $6-CH_3$ | 151 |
| 8 | 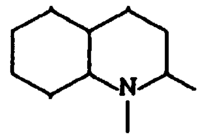 | $CH_3$ | $CH_3$ | $6-CH_3$ | > 230 |
| 9 | 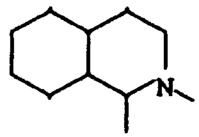 | $CH_3$ | $CH_3$ | $6-CH_3$ | 204 |
| 10 | 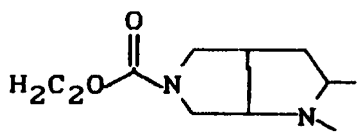 | $CH_3$ | $CH_3$ | $6-CH_3$ | 146 |
| 11 | 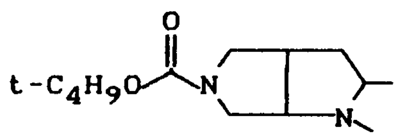 | $CH_3$ | $CH_3$ | $6-CH_3$ | |
| 11a | 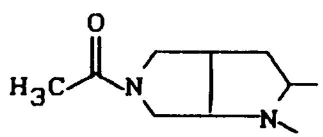 | $CH_3$ | $CH_3$ | $6-CH_3$ | 165 |

Tabelle 1 (Fortsetzung)

| Bsp. Nr. | 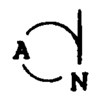 | X | Y | $Z_n$ | Fp.°C |
|---|---|---|---|---|---|
| 11b | 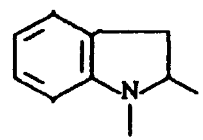 | $CH_3$ | $CH_3$ | $6-CH_3$ | >220 |
| 11c | 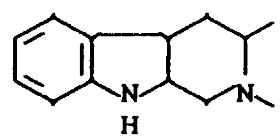 | $CH_3$ | $CH_3$ | $6-CH_3$ | >220 |

Beispiel 12

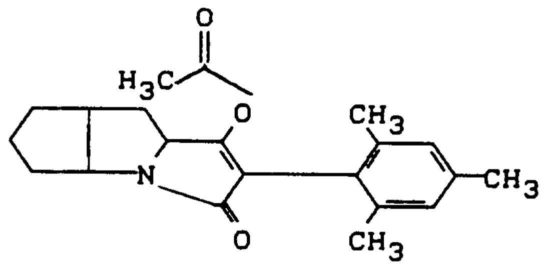

5,95 g (20 mmol) 1-Aza-tricyclo-(6,3,$0^{1.8}$` ,$0^{2.6}$)-10-(2,4,6-trimethylphenyl)-undecan-9,11-dionsuspendiert in 70 ml Methyl-tert.-butyl-Ether werden mit 1,63 ml absolutem Pyridin und 3,4 ml (20 mmol) Hünig-Base versetzt. Bei 0-10 °C tropft man 1,5 ml (20 mmol) Acetylchlorid in 5 ml Methyl-tert.-butyl-Ether zu, rührt 15 Minuten nach, filtriert den Niederschlag ab, rotiert im Vakuum ein und chromatographiert den Rückstand an Kieselgel mit Cyclohexan-Essigester 1:1. Nach Kristallisation aus n-Hexan erhielt man 4,7 g (= 69,2 % der Theorie) 1-Aza-9-acetoxytricyclo-(6,3,$0^{1.8}$,$0^{2.6}$)-10-(2,4,6-(trimethylphenyl)-9-undecan-11-on vom Schmelzpunkt 100 °C.

Analog erhält man

**Tabelle 2**

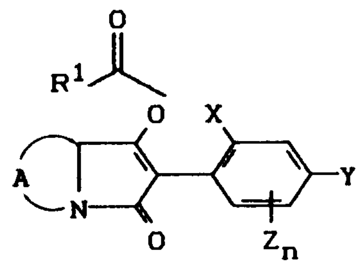

| Bsp. Nr. | 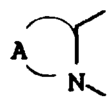 | X | Y | $Z_n$ | $R^1$ | Fp.° C |
|---|---|---|---|---|---|---|
| 13 | 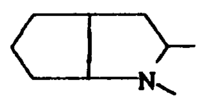 | Cl | Cl | H | $CH_3$- | 104 |
| 14 | " | Cl | Cl | H | $(CH_3)_3C$- | Öl |
| 15 | " | Cl | H | 6-Cl | $CH_3$- | 185 |
| 16 | " | Cl | H | 6-Cl | $(CH_3)_3C$- | 132 |
| 17 | " | $CH_3$ | $CH_3$ | H | $CH_3$- | |
| 18 | " | $CH_3$ | $CH_3$ | H | $(CH_3)_3C$- | |
| 19 | " | $CH_3$ | H | 6-$CH_3$ | $CH_3$- | |

**Tabelle 2** (Fortsetzung)

| Bsp. Nr. | 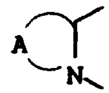 | X | Y | $Z_n$ | $R^1$ | Fp.°C |
|---|---|---|---|---|---|---|
| 20 | 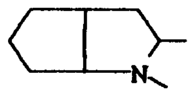 | $CH_3$ | H | 6-$CH_3$ | $(CH_3)_3C-$ | |
| 20a | " | $CH_3$ | $CH_3$ | 6-$CH_3$ | $CH_3CH_2-$ | |
| 21 | " | $CH_3$ | $CH_3$ | 6-$CH_3$ | $(CH_3)_2CH-$ | Öl |
| 22 | " | $CH_3$ | $CH_3$ | 6-$CH_3$ | $(CH_3)_3C-$ | 104 |
| 23 | " | $CH_3$ | $CH_3$ | 6-$CH_3$ | $CH_3-(CH_2)_3-$ | Öl |
| 23a | " | $CH_3$ | $CH_3$ | 6-$CH_3$ | $(CH_3)_2CH-CH_2-$ | Öl |
| 24 | " | $CH_3$ | $CH_3$ | 6-$CH_3$ | $C_2H_5-C(CH_3)_2$ | Öl |
| 25 | " | $CH_3$ | $CH_3$ | 6-$CH_3$ | $(CH_3)_3C-CH_2-$ | Öl |
| 26 | " | $CH_3$ | $CH_3$ | 6-$CH_3$ | $(CH_3)_2CH-C(CH_3)_2$ | 98 |
| 27 | " | $CH_3$ | $CH_3$ | 6-$CH_3$ | $CH_2=CH-(CH_2)_8-$ | Öl |
| 27a | " | $CH_3$ | $CH_3$ | 6-$CH_3$ | $C_{17}H_{35}-$ | Öl |
| 28 | " | $CH_3$ | $CH_3$ | 6-$CH_3$ | 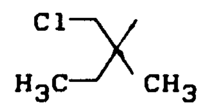 | 102 |

<u>Tabelle 2</u> (Fortsetzung)

| Bsp. Nr. | 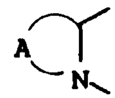 | X | Y | $Z_n$ | $R^1$ | Fp.°C |
|---|---|---|---|---|---|---|
| 29 | 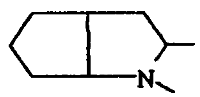 | $CH_3$ | $CH_3$ | 6-$CH_3$ | 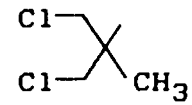 | |
| 30 | " | $CH_3$ | $CH_3$ | 6-$CH_3$ | 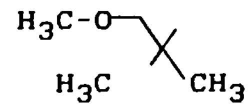 | Öl |
| 31 | " | $CH_3$ | $CH_3$ | 6-$CH_3$ | 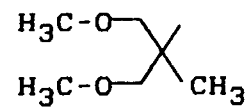 | Öl |
| 32 | " | $CH_3$ | $CH_3$ | 6-$CH_3$ | 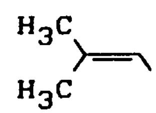 | 120 |
| 33 | " | $CH_3$ | $CH_3$ | 6-$CH_3$ | $H_3C-S-CH_2$ | |
| 34 | " | $CH_3$ | $CH_3$ | 6-$CH_3$ | 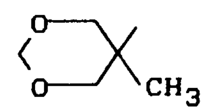 | 121 |

<u>Tabelle 2</u> (Fortsetzung)

| Bsp. Nr. | 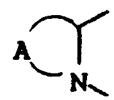 | X | Y | $Z_n$ | $R^1$ | Fp. °C |
|---|---|---|---|---|---|---|
| 34a | 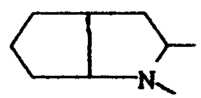 | $CH_3$ | $CH_3$ | 6-$CH_3$ | 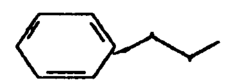 | Öl |
| 34b | " | $CH_3$ | $CH_3$ | 6-$CH_3$ | 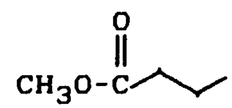 | 113 |
| 34c | " | $CH_3$ | $CH_3$ | 6-$CH_3$ | 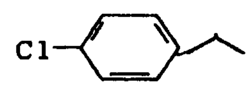 | Öl |
| 35 | 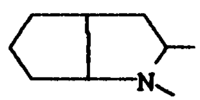 | $CH_3$ | $CH_3$ | 6-$CH_3$ | 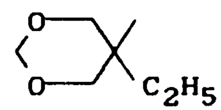 | 125 |

<u>Tabelle 2</u> (Fortsetzung)

| Bsp. Nr. | 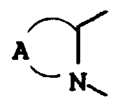 | X | Y | $Z_n$ | $R^1$ | Fp.°C |
|---|---|---|---|---|---|---|
| 35a | 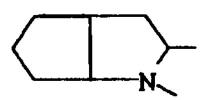 | $CH_3$ | $CH_3$ | 6-$CH_3$ | 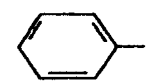 | 69 |
| 35b | " | $CH_3$ | $CH_3$ | 6-$CH_3$ | 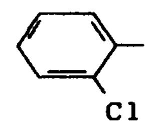 | Öl |
| 35c | " | $CH_3$ | $CH_3$ | 6-$CH_3$ | 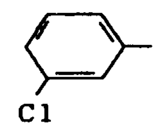 | Harz |
| 35d | " | $CH_3$ | $CH_3$ | 6-$CH_3$ | 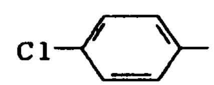 | 125 |
| 36 | " | $CH_3$ | $CH_3$ | 6-$CH_3$ | 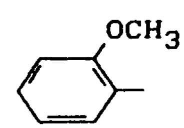 | 75 |

Tabelle 2 (Fortsetzung)

| Bsp. Nr. | 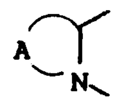 | X | Y | $Z_n$ | $R^1$ | Fp.°C |
|---|---|---|---|---|---|---|
| 37 | 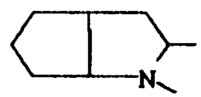 | $CH_3$ | $CH_3$ | 6-$CH_3$ | 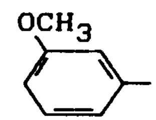 | 83 |
| 38 | " | $CH_3$ | $CH_3$ | 6-$CH_3$ | 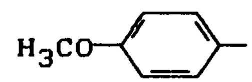 | 146 |
| 39 | 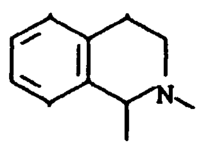 | $CH_3$ | $CH_3$ | 6-$CH_3$ | $CH_3$- | 194 |

**Tabelle 2** (Fortsetzung)

| Bsp. Nr. | 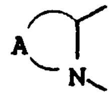 | X | Y | $Z_n$ | $R^1$ | Fp.° C |
|---|---|---|---|---|---|---|
| 40 | 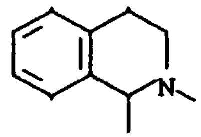 | $CH_3$ | $CH_3$ | $6-CH_3$ | $(CH_3)_3C-$ | |
| 41 | 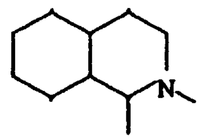 | $CH_3$ | $CH_3$ | $6-CH_3$ | $CH_3$ | Harz |
| 42 | " | $CH_3$ | $CH_3$ | $6-CH_3$ | $(CH_3)_3C-$ | 153 |
| 43 | 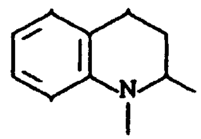 | $CH_3$ | $CH_3$ | $6-CH_3$ | $CH_3-$ | |
| 44 | " | $CH_3$ | $CH_3$ | $6-CH_3$ | $(CH_3)_3C-$ | |
| 45 | 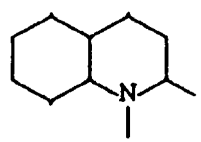 | $CH_3$ | $CH_3$ | $6-CH_3$ | $CH_3-$ | 170 |

Tabelle 2 (Fortsetzung)

| Bsp. Nr. | 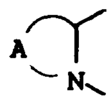 | X | Y | $Z_n$ | $R^1$ | Fp.° C |
|---|---|---|---|---|---|---|
| 46 | 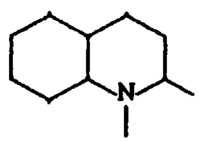 | $CH_3$ | $CH_3$ | 6-$CH_3$ | $(CH_3)_3C$- | 148 |
| 47 | 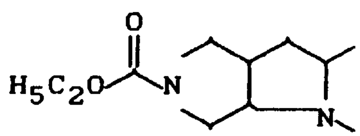 | $CH_3$ | $CH_3$ | 6-$CH_3$ | $CH_3$- | Öl |
| 48 | " | $CH_3$ | $CH_3$ | 6-$CH_3$ | $(CH_3)_3C$- | Öl |
| 48a | 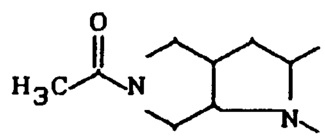 | $CH_3$ | $CH_3$ | 6-$CH_3$ | $CH_3$- | 180 |
| 48b | 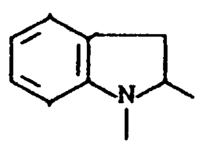 | $CH_3$ | $CH_3$ | 6-$CH_3$ | $(CH_3)_3C$- | 90 |

Beispiel 49

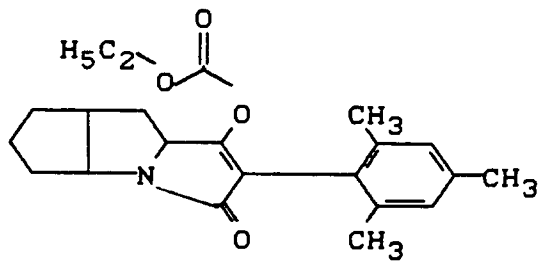

5,95 g (20 mmol) 1-Aza-tricyclo-(6,3,$0^{1.8}$,$0^{2.6}$)-10-(2,4,6-trimethylphenyl)-undecan-9,11-dion suspendiert in 70 ml Methyl-tert.-butyl-Ether werden mit 1,63 ml absolutem Pyridin und 3,4 ml (20 mmol) Hünig-Base versetzt. Bei 0-10 °C tropft man 2 ml (20 mmol) Chlorameisensäureethylester in 5 ml Methyl-tert.-butyl-Ether zu, rührt 15 Minuten nach, filtriert den Niederschlag ab, rotiert im Vakuum ein und chromatographiert den Rückstand an Kieselgel mit Cyclohexan-Essigester 1:1. Man erhält 6,1 g (=82,5 % der Theorie) 1-Aza-9-ethoxycarbonyloxy-tricyclo-(6,3,$0^{1.8}$,$0^{2.6}$)-10-(2,4,6-trimethylphenyl)-9-undecan-11-on als gelbes Öl.

Analog erhält man:

**Tabelle 3**

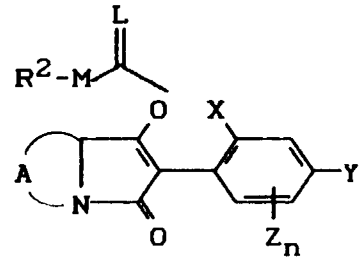

(Ic)

| Bsp. Nr. | 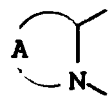 | X | Y | $Z_n$ | L | M | $R^2$ | Fp. °C |
|---|---|---|---|---|---|---|---|---|
| 50 | 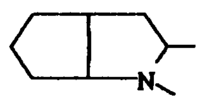 | Cl | Cl | H | O | O | $C_2H_5$- | 89 |
| 50a | " | Cl | Cl | H | O | O | $(CH_3)_2CH$- | 100 |
| 50b | " | Cl | Cl | H | O | O | $(CH_3CH_2\text{-}CH(CH_3)$- | Öl |
| 51 | " | Cl | H | 6-Cl | O | O | $C_2H_5$- | 98 |
| 51a | " | Cl | H | 6-Cl | O | O | $(CH_3)_2CH$- | 132 |
| 51b | " | Cl | H | 6-Cl | O | O | $CH_3CH_2\text{-}CH(CH_3)$- | 65 |

Tabelle 3 (Fortsetzung)

| Bsp. Nr. | 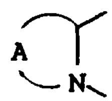 | X | Y | $Z_n$ | L | M | $R^2$ | Fp.°C |
|---|---|---|---|---|---|---|---|---|
| 52 | 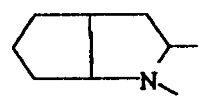 | $CH_3$ | $CH_3$ | H | O | O | $C_2H_5$- | |
| 53 | | $CH_3$ | H | 6-$CH_3$ | O | O | $C_2H_5$- | |
| 54 | " | $CH_3$ | $CH_3$ | 6-$CH_3$ | O | O | $CH_3$- | Öl |
| 55 | " | $CH_3$ | $CH_3$ | 6-$CH_3$ | O | O | $(CH_3)_2CH$- | 104 |

Tabelle 3 (Fortsetzung)

| Bsp. Nr. | 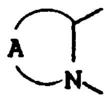 | X | Y | $Z_n$ | L | M | $R^2$ | Fp.°C |
|---|---|---|---|---|---|---|---|---|
| 56 | 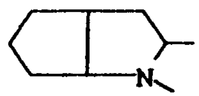 | $CH_3$ | $CH_3$ | 6-$CH_3$ | O | O | $(CH_3)_2CH-CH_2-$ | 78 |
| 57 | " | $CH_3$ | $CH_3$ | 6-$CH_3$ | O | O | $C_2H_5-CH(CH_3)-$ | 82 |
| 58 | " | $CH_3$ | $CH_3$ | 6-$CH_3$ | O | O | $(CH_3)_3C-$ | |
| 59 | " | $CH_3$ | $CH_3$ | 6-$CH_3$ | O | O | $(CH_3)_3C-CH_2-$ | 107 |
| 60 | " | $CH_3$ | $CH_3$ | 6-$CH_3$ | O | O | 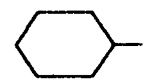 | Öl |
| 60a | " | $CH_3$ | $CH_3$ | 6-$CH_3$ | O | O | 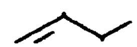 | Öl |
| 61 | " | $CH_3$ | $CH_3$ | 6-$CH_3$ | O | O | $C_2H_5O$ 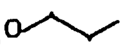 | Öl |
| 62 | " | $CH_3$ | $CH_3$ | 6-$CH_3$ | O | O | $C_2H_5O$ 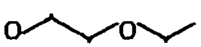 | |
| 63 | " | $CH_3$ | $CH_3$ | 6-$CH_3$ | O | O | 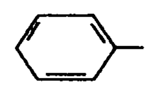 | Öl |

Tabelle 3 (Fortsetzung)

| Bsp. Nr. | 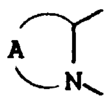 | X | Y | $Z_n$ | L | M | $R^2$ | Fp.° C |
|---|---|---|---|---|---|---|---|---|
| 63a | 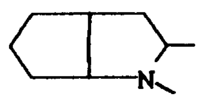 | $CH_3$ | $CH_3$ | 6-$CH_3$ | O | O | 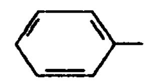 | Öl |
| 63b | " | $CH_3$ | $CH_3$ | 6-$CH_3$ | O | O | $C_2H_5O$ 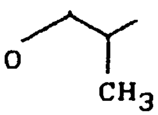 | Öl |
| 63c | " | $CH_3$ | $CH_3$ | 6-$CH_3$ | O | O | $C_3H_7O$ 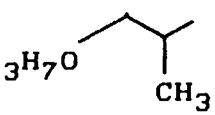 | Öl |
| 63d | " | $CH_3$ | $CH_3$ | 6-$CH_3$ | O | O | $(CH_3)_2CHO$ 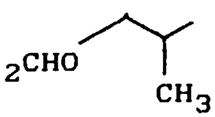 | Öl |
| 63e | " | $CH_3$ | $CH_3$ | 6-$CH_3$ | O | O | $C_2H_5O$ 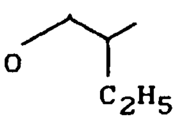 | Öl |

Versuchsbeschreibung / Beispiel Nr. 64

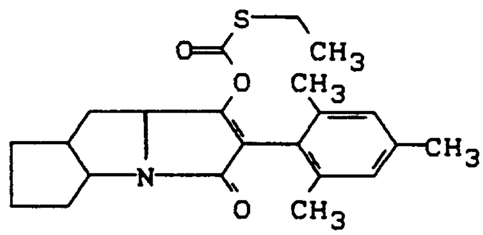

2,97 g 1-Aza-tricyclo-(6.3.$0^{1.8}0^{2.6}$)-10-(2,4,6-trimethylphenyl)-undecan-9.11-dion und 20 ml absolutes Dimethylformamid werden mit 0,3 g Natriumhydrid (80%ig) versetzt. Nach Beendigung der Wasserstoffentwicklung kühlt man auf 10 ° C ab und gibt 1,5 ml (0,014 mol)

$$C_2H_5O-\underset{\underset{O}{\|}}{C}-S-CH_2-CH_3$$

zu. Dann läßt man einige Stunden rühren, nimmt in 100 ml Wasser auf, extrahiert dreimal mit Methylenchlorid und wäscht einmal mit 40 ml 5%iger Salzsäure, trocknet mit Natriumsulfat und dampft ein.

Ausbeute 2,73 g (71% d. Th.) der Titelverbindung

Fp: 112 ° C.

Versuchsbeschreibung / Beispiel Nr. 64c

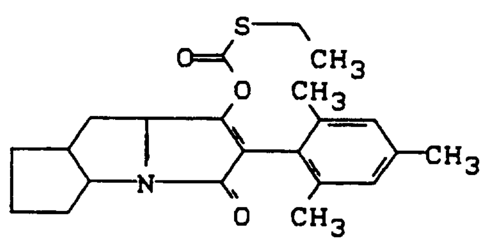

2,97 g 0,01 mol) 1-Aza-tricyclo-(6.3.$0^{1.8}0^{2.6}$)-10-(2,4,6-trimethylphenyl)-undecan-9.11-dion und 20 ml absolutes Dimethylformamid werden mit 0,3 g Natriumhydrid (80%ig) versetzt. Wenn die Wasserstoffentwicklung beendet ist, gibt man 3 ml (0,01 mol) Schwefelkohlenstoff zu und läßt 3 Stunden bei Raumtemperatur rühren. Weiterhin gibt man 3 ml (0,05 mol) Methyljodid zu und läßt einige Stunden bei Raumtemperatur rühren. Schließlich nimmt man in 100 ml Wasser auf, extrahiert dreimal mit Methylenchlorid, trocknet über Natriumsulfat und dampft ein.

Ausbeute 1,77 g der Titelverbindung (46% d. Th.)

Fp: 90-91 ° C.

Tabelle 3 (Fortsetzung)

| Bsp. Nr. | 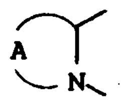 | X | Y | $Z_n$ | L | M | $R^2$ | Fp.° C |
|---|---|---|---|---|---|---|---|---|
| 64 | 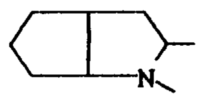 | $CH_3$ | $CH_3$ | 6-$CH_3$ | O | S | $C_2H_5$- | 112 |
| 64a | " | $CH_3$ | $CH_3$ | 6-$CH_3$ | O | S | $CH_3$-$(CH_2)_3$- | Öl |
| 64b | " | $CH_3$ | $CH_3$ | 6-$CH_3$ | O | S | 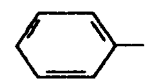 | 105 |
| 64c | " | $CH_3$ | $CH_3$ | 6-$CH_3$ | S | S | $CH_3$- | 90-91 |
| 64d | " | $CH_3$ | $CH_3$ | 6-$CH_3$ | S | S | $C_2H_5$- | 94-95 |
| 64e | " | $CH_3$ | $CH_3$ | 6-$CH_3$ | S | S | $(CH_3)_2CH$- | 80-81 |

**Tabelle 3** (Fortsetzung)

| Bsp. Nr. | 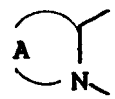 | X | Y | $Z_n$ | L | M | $R^2$ | Fp. °C |
|---|---|---|---|---|---|---|---|---|
| 65 | 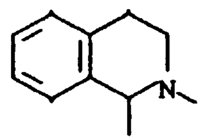 | $CH_3$ | $CH_3$ | $6-CH_3$ | O | O | $C_2H_5-$ | |
| 66 | " | $CH_3$ | $CH_3$ | $6-CH_3$ | O | O | $(CH_3)_2CH-$ | |
| 67 | " | $CH_3$ | $CH_3$ | $6-CH_3$ | O | O | $(CH_3)_3C-CH_2-$ | |
| 68 | 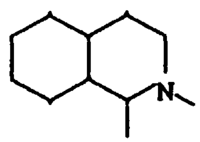 | $CH_3$ | $CH_3$ | $6-CH_3$ | O | O | $C_2H_5-$ | |
| 69 | " | $CH_3$ | $CH_3$ | $6-CH_3$ | O | O | $(CH_3)_2CH-$ | |
| 70 | 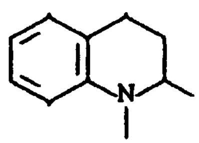 | $CH_3$ | $CH_3$ | $6-CH_3$ | O | O | $C_2H_5-$ | |

Tabelle 3 (Fortsetzung)

| Bsp. Nr. | 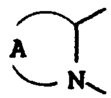 | X | Y | $Z_n$ | L | M | $R^2$ | Fp.°C |
|---|---|---|---|---|---|---|---|---|
| 71 | 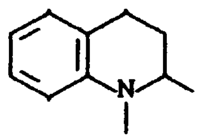 | $CH_3$ | $CH_3$ | 6-$CH_3$ | O | O | $(CH_3)_2CH-$ | |
| 72 | 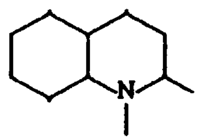 | $CH_3$ | $CH_3$ | 6-$CH_3$ | O | O | $C_2H_5-$ | |
| 73 | " | $CH_3$ | $CH_3$ | 6-$CH_3$ | O | O | $(CH_3)_2CH-$ | |
| 74 | 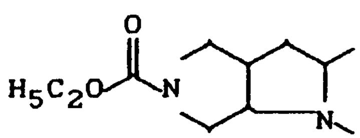 | $CH_3$ | $CH_3$ | 6-$CH_3$ | O | O | $C_2H_5-$ | |
| 75 | " | $CH_3$ | $CH_3$ | 6-$CH_3$ | O | O | $(CH_3)_2CH-$ | Öl |
| 76 | " | $CH_3$ | $CH_3$ | 6-$CH_3$ | O | O | $C_2H_5-\underset{CH_3}{\underset{\vert}{CH}}-$ | Öl |
| 76a | 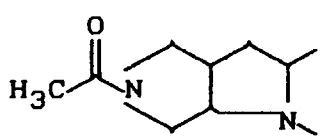 | $CH_3$ | $CH_3$ | 6-$CH_3$ | O | O | $CH_3-$ | 180 |

Versuchsbeschreibung / Beispiel Nr. 72

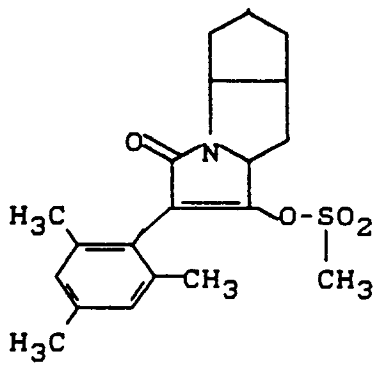

3,56 g 1-Aza-tricyclo-(6.3.0$^{1.8}$0$^{2.6}$)-10-(2,4,6-trimethylphenyl)-undecan-9.11-dion und 20 ml absolutes Dimethylformamid versetzt man mit 0,3 g Natriumhydrid (80%ig). Nach Beendigung der Wasserstoffentwicklung kühlt man auf 10 °C ab, gibt 1,6 g (0,014 mol) Methansulfonsäurechlorid hinzu und läßt eine Stunde bei Raumtemperatur rühren. Man rührt in 100 ml Wasser ein, extrahiert dreimal mit Methylenchlorid, wäscht einmal mit 40 ml 5%iger Salzsäure, trocknet über Natriumsulfat und dampft ein. Ausbeute: 2,80 g (75% d. Th.) der Titelverbindung.
Fp: 149 °C.

**Tabelle 4**

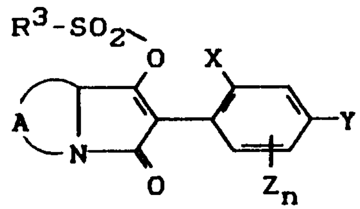 (Id)

| Bsp. Nr. | 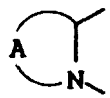 | X | Y | $Z_n$ | $R^3$ | Fp° C |
|---|---|---|---|---|---|---|
| 77 | 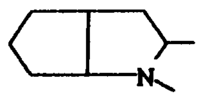 | $CH_3$ | $CH_3$ | $6-CH_3$ | $CH_3-$ | 149 |
| 78 | " | $CH_3$ | $CH_3$ | $6-CH_3$ | 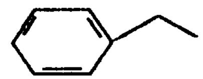 | |
| 79 | " | $CH_3$ | $CH_3$ | $6-CH_3$ | 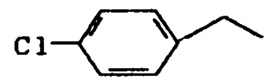 | |
| 80 | " | $CH_3$ | $CH_3$ | $6-CH_3$ | 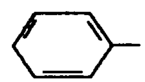 | |
| 81 | " | $CH_3$ | $CH_3$ | $6-CH_3$ | 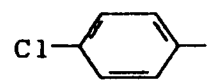 | |

<u>Tabelle 5</u>

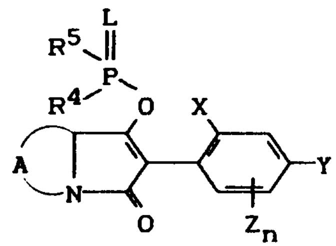

(Ie)

| Bsp. Nr. | 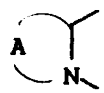 | X | Y | $Z_n$ | L | $R^4$ | $R^5$ | Fp. |
|---|---|---|---|---|---|---|---|---|
| 82 | 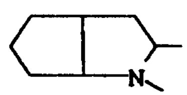 | $CH_3$ | $CH_3$ | 6-$CH_3$ | S | $CF_3CH_2O$- | $CH_3$ | |
| 83 | " | $CH_3$ | $CH_3$ | 6-$CH_3$ | S | $C_2H_5$-O- | $C_3H_7$-S- | |
| 84 | " | $CH_3$ | $CH_3$ | 6-$CH_3$ | O | $CH_3$-O- | $C_2H_5$-S- | |
| 85 | " | $CH_3$ | $CH_3$ | 6-$CH_3$ | O | $CH_3$-O- | $(CH_3)_2CH$-S- | |
| 86 | " | $CH_3$ | $CH_3$ | 6-$CH_3$ | O | $CH_3$-O- | $C_2H_5$ \ CH-S- / $CH_3$ | |

Versuchsbeschreibung / Beispiel Nr. 87a

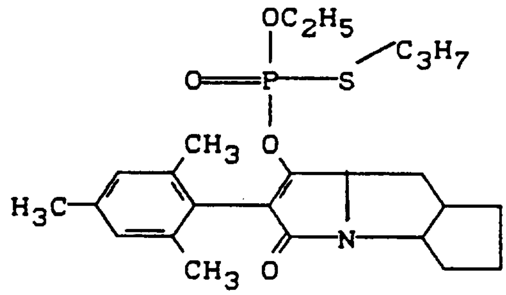

6,5 g (0,034 mol) der Verbindung

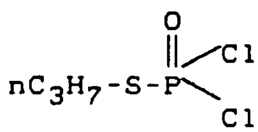

werden in 100 ml Toluol vorgelegt und mit 5,2 ml (0,037 mol) Triethylamin versetzt. Danach werden bei -5° bis 0°C 1,6 g Ethanol (0,034 mol) in 10 ml Toluol zugetropft. Man läßt 2 Stunden bei Raumtemperatur nachrühren. Dann werden 5,2 ml Triethylamin zugetropft und 10 g 1-Aza-tricyclo-(6.3-$0^{1.8}$, $0^{2.6}$)-10-(2,4,6-trimethylphenyl)-undecan-9.11-dion zugegeben und in üblicher Art und Weise aufgearbeitet. Die Reinigung erfolgt durch Abfiltrieren über eine Kieselgelfritte (Toluol:Aceton 8:2).

Ausbeute: 8,20 g der Titelverbindung (52% d. Th.)

$n_D^{20}$ = 1,5600.

<u>Tabelle 5</u> (Fortsetzung)

| Bsp. Nr. | 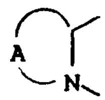 | X | Y | $Z_n$ | L | $R^4$ | $R^5$ | Fp. |
|---|---|---|---|---|---|---|---|---|
| 87 | 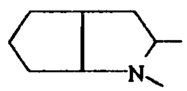 | $CH_3$ | $CH_3$ | 6-$CH_3$ | O | $C_2H_5O$- | $C_2H_5$-S- | |
| 87a | " | $CH_3$ | $CH_3$ | 6-$CH_3$ | O | $C_2H_5$-O- | $C_3H_7$-S- | $n_D^{20}$ :1.560 |
| 88 | " | $CH_3$ | $CH_3$ | 6-$CH_3$ | O | $C_2H_5$-O- | $(CH_3)_2CH$-S- | |
| 89 | " | $CH_3$ | $CH_3$ | 6-$CH_3$ | O | $C_2H_5$-O- | 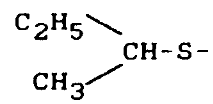 | |
| 89a | " | $CH_3$ | $CH_3$ | 6-$CH_3$ | S | $CH_3$ | $CH_3$-O- | 128 |
| 89b | " | $CH_3$ | $CH_3$ | 6-$CH_3$ | S | $C_2H_5$ | $CH_3$-O- | 128 |
| 89c | " | $CH_3$ | $CH_3$ | 6-$CH_3$ | S | $CH_3$ | $C_2H_5$-S- | Öl |
| 89d | " | $CH_3$ | $CH_3$ | 6-$CH_3$ | S | $CH_3$ | $C_3H_7$-S- | 109 |
| 89e | " | $CH_3$ | $CH_3$ | 6-$CH_3$ | S | $CH_3$ | $(CH_3)_2CH$-S- | $n_D^{20}$ :1.581 |

<u>Tabelle 5</u> (Fortsetzung)

| Bsp. Nr. | 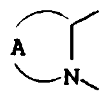 | X | Y | $Z_n$ | L | $R^4$ | $R^5$ | Fp. |
|---|---|---|---|---|---|---|---|---|
| 89f | 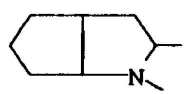 | $CH_3$ | $CH_3$ | 6-$CH_3$ | S | $CH_3$ | 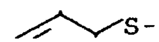 | 79 |
| 89g | " | $CH_3$ | $CH_3$ | 6-$CH_3$ | S | $CH_3$ | $C_4H_9$-S- | 88 |
| 89h | " | $CH_3$ | $CH_3$ | 6-$CH_3$ | S | $CH_3$ | $(CH_3)_2CH-CH_2$-S- | 91 |
| 89i | " | $CH_3$ | $CH_3$ | 6-$CH_3$ | S | $CH_3$ | $(CH_3)_3C$-S- | $n_D^{20}$ :1.568 |
| 89j | " | $CH_3$ | $CH_3$ | 6-$CH_3$ | S | $CH_3$ | $CH_3-(CH_2)_4$-S- | Öl |
| 89k | " | $CH_3$ | $CH_3$ | 6-$CH_3$ | S | $CH_3$ | $(CH_3)_2CH-(CH_2)_2$-S- | Öl |
| 89l | " | $CH_3$ | $CH_3$ | 6-$CH_3$ | S | $C_2H_5$ | $C_2H_5$-S- | 65 |
| 89m | " | $CH_3$ | $CH_3$ | 6-$CH_3$ | S | $C_2H_5$ | $(CH_3)_2CH$-S- | $n_D^{20}$ : 1.573 |
| 89n | " | $CH_3$ | $CH_3$ | 6-$CH_3$ | S | $C_2H_5$ | $CH_3(CH_2)_3$-S- | $n_D^{20}$ : 1.5634 |
| 89o | " | $CH_3$ | $CH_3$ | 6-$CH_3$ | S | $C_2H_5$ | $(CH_3)_3C$- | 104 |

Versuchsbeschreibung/Beispiel Nr. 90

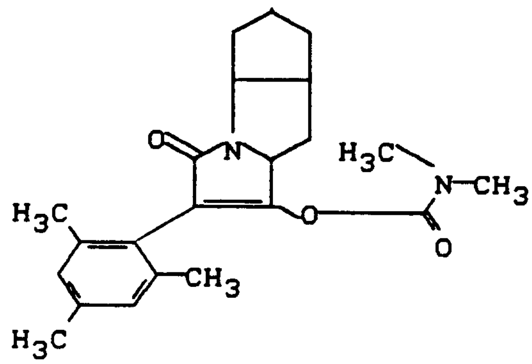

3,56 g 1-Aza-tricyclo-(6.3.$0^{1.8}$,$0^{2.6}$)-10-(2,4,6-trimethylphenyl)-undecan-9.11-dion und 15 ml absolutes Dimethylformamid werden mit 0,3 g Natriumhydrid (80%ig) versetzt. Nach Beendigung der Wasserstoffentwicklung kühlt man auf 10 °C ab und gibt 1,6 g N,N-Dimethylcarbamidsäurechlorid zu. Anschließend läßt man 1 Stunde bei 80 °C rühren.

Man rührt in 35 ml 1%iger NaOH ein, extrahiert dreimal mit Methylenchlorid, wäscht einmal mit 15%iger Salzsäure, trocknet über Natriumsulfat und dampft ein.

Ausbeute: 1,37 g (39% d. Th.) der Titelverbindung.

Fp: 149 °C

Tabelle 6

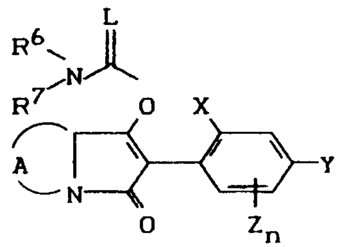

(If)

| Bsp. Nr. | 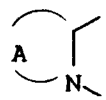 | X | Y | $Z_n$ | L | $R^6$ | $R^7$ | Fp. |
|---|---|---|---|---|---|---|---|---|
| 90 | 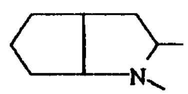 | $CH_3$ | $CH_3$ | $6-CH_3$ | O | $CH_3-$ | $CH_3-$ | 149 |
| 91 | " | $CH_3$ | $CH_3$ | $6-CH_3$ | S | $CH_3-$ | $CH_3-$ | 78 |
| 92 | " | $CH_3$ | $CH_3$ | $6-CH_3$ | O | $CH_2=CHCH_2-$ | $CH_2=CH-CH_2-$ | |
| 93 | " | $CH_3$ | $CH_3$ | $6-CH_3$ | O | $-(CH_2)_2-O-(CH_2)_2-$ | | |
| 94 | " | $CH_3$ | $CH_3$ | $6-CH_3$ | S | $-(CH_2)_5-$ | | |
| 95 | " | $CH_3$ | $CH_3$ | $6-CH_3$ | S | 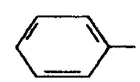 | $C_2H_5-$ | |

Beispiel 96

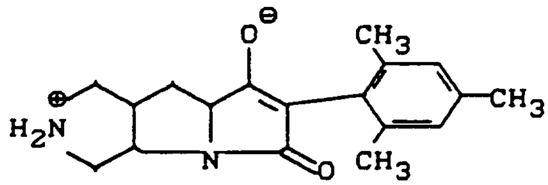

7,41 g (20 mmol) 1,4-Diaza-4-N-(ethoxycarbonyl)-tricyclo-(6,3,$0^{1.8}$,$0^{2.6}$)-10-(2,4,6-trimethylphenyl)-undecan-9,11-dion werden in 100 ml Wasser mit 15,78 g (50 mmol) Bariumhydroxid-octahydrat 10 Stunden lang unter Rückfluß erhitzt. Nach dem Abkühlen werden portionsweise 6,9 g (50 mmol) Kaliumcarbonat zugefügt. Nach 15 Minuten saugt man ab, wäscht mit 20 ml Wasser nach und stellt das Filtrat mit 1N Salzsäure auf pH 7 ein. Man saugt erneut ab und trocknet im Vakuum. Auf diese Weise erhält man 5,18 g (= 86,9 % der Theorie) des inneren Salzes obiger Struktur vom Schmelzpunkt > 230 °C als farbloses Pulver.

Tabelle 7

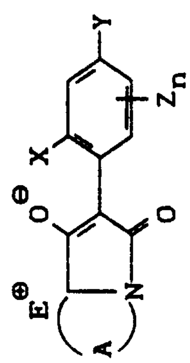

(Ig)

| Bsp. Nr. | 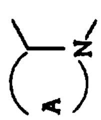 | X | Y | $Z_n$ | $E^{\oplus}$ |
|---|---|---|---|---|---|
| 97 | 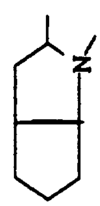 | $CH_3$ | $CH_3$ | 6-$CH_3$ | $Na^{\oplus}$ |
| 98 | " | $CH_3$ | $CH_3$ | 6-$CH_3$ | $NH_4^{\oplus}$ |
| 99 | " | $CH_3$ | $CH_3$ | 6-$CH_3$ | 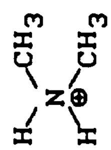 |

Zwischenprodukte

Beispiel: 1A

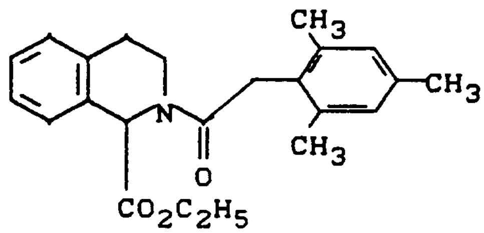

17,4 g (85 mmol) 1,2,7,8-Tetrahydroisochinolin-2-carbonsäureethylester werden in 130 ml absolutem Tetrahydrofuran vorgelegt, mit 11,9 ml (85 mmol) Triethylamin versetzt und bei 0 bis 10 °C 16,7 g (85 mmol) Mesitylenessigsäurechlorid hinzugetropft. Nach 30 Minuten rührt man in 400 ml Eiswasser und 100 ml 1N Salzsäure ein, extrahiert mit Methylenchlorid, trocknet und rotiert im Vakuum ein. Man erhält 30,9 g (= 99,5 % der Theorie) 1,2,7,8-Tetrahydroisochinolin-N-(2,4,6-trimethylphenylacetyl)-2-carbonsäureethylester als gelbes Öl.

In Analogie wurden hergestellt:

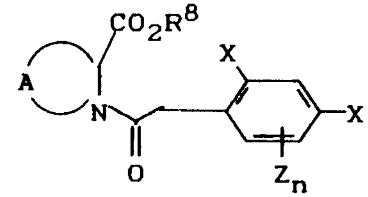

(II)

| Bsp. Nr. | 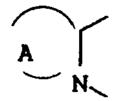 | X | Y | $Z_n$ | $R^8$ | Fp.°C |
|---|---|---|---|---|---|---|
| 2A | 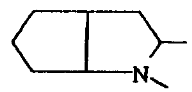 | $CH_3$ | $CH_3$ | 6-$CH_3$ | $C_2H_5$ | Öl |
| 3A | 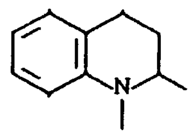 | $CH_3$ | $CH_3$ | 6-$CH_3$ | $C_2H_5$ | Öl |
| 4A | 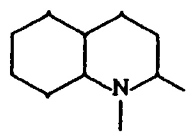 | $CH_3$ | $CH_3$ | 6-$CH_3$ | $C_2H_5$ | Öl |
| 5A | 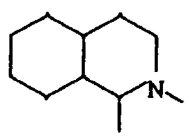 | $CH_3$ | $CH_3$ | 6-$CH_3$ | $C_2H_5$ | Öl |
| 6A | 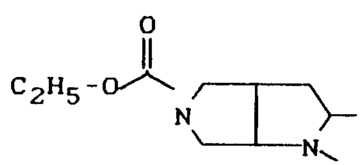 | $CH_3$ | $CH_3$ | 6-$CH_3$ | $C_2H_5$ | Öl |

In Analogie wurden hergestellt:

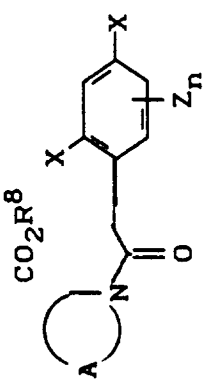

(II)

| Bsp. Nr. | 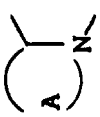 | X | Y | $Z_n$ | $R^8$ | Fp.°C |
|---|---|---|---|---|---|---|
| 7A | 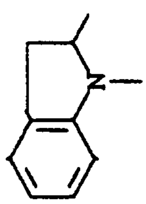 | $CH_3$ | $CH_3$ | 6-$CH_3$ | $CH_3$ | Öl |
| 8A | 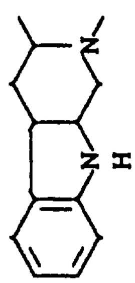 | $CH_3$ | $CH_3$ | 6-$CH_3$ | $CH_3$ | Öl |
| 9A | 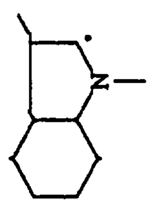 | Cl | Cl | H | $C_2H_5$ | Öl |
| 10A | 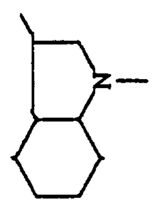 | Cl | H | H | 6-Cl | Öl |

Die Wirkstoffe eignen sich bei guter Pflanzenverträglichkeit und günstiger Warmblütertoxizität zur Bekämpfung von tierischen Schädlingen, insbesondere Insekten und Spinnentieren die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.. Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven, Läuse, Haarlinge, Federlinge, Flöhe und endoparasitisch lebende Würmer.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Charakteristisch für die erfindungsgemäßen Verbindungen ist, daß sie eine selektive Wirksamkeit gegen monokotyle Unkräuter im Vor- und Nachlaufverfahren (Pre- und Post-emergence) bei guter Kulturflanzenverträglichkeit aufweisen.

Die erfindungsgemäßen Wirkstoffe können z.B. bei den folgenden Pflanzen verwendet werden:
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorragenden Wirkung gegen Schadpflanzen gute Verträglichkeit gegenüber wichtigen Kulturpflanzen, wie z. B. Weizen, Baumwolle, Sojabohnen, Citrusfrüchten und Zuckerrüben, und können daher als selektive Unkrautbekämpfungsmittel eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden,

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren, Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Beispiel A

Tetranychus-Test (resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden. (6), (12), (22), (25), (47), (48).

Beispiel B

Nephotettix-Test

Lösungsmittel: 7 Gewichtsteile Dimethylformamid
Emulgator: 1 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeute 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: (6), (12), (22), (25), (47), (48), (49).

Beispiel C

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle, Es bedeuten:

0 % = keine Wirkung (wie unbehandelte Kontrolle)
100 % = totale Vernichtung

Bei diesem Test zeigen z.B. die folgenden Verbindungen der Herstellungsbeispiele überlegene Wirksamkeit gegenüber dem Stand der Technik: (6), (10), (12), (22), (49).

Beispiel D

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung wurde 1 Gewichtsteil Wirkstoff mit der angegebenen Menge Lösungsmittel vermischt, die angegebene Menge Emulgator zugegeben und das Konzentrat anschließend mit Wasser auf die gewünschte Konzentration verdünnt.

Mit der Wirkstoffzubereitung spritzt man Testpflanzen, welche eine Höhe von 5-15 cm haben so, daß die jeweils gewünschten Wirkstoffmengen pro Flächeneiheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = Keine Wirkung (wie unbehandelte Kontrole)
100 % = totale Vernichtung.

Bei diesem Test zeigen z.B. die folgenden Verbindungen der Herstellungsbeispiele überlegene Wirksamkeit gegenüber dem Stand der Technik: (6), (10), (12), (22), (49).

## Patentansprüche

**1.** Polycyclische 3-Arylpyrrolidin-2,4-dion-Derivate der Formel (I)

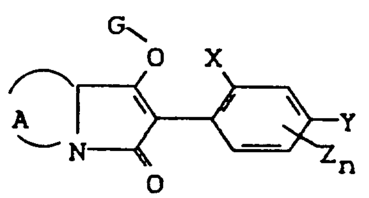

(I)

in welcher
die Gruppierung

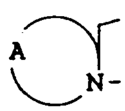

eine der folgenden Bedeutungen 1 bis 29 annimmt:

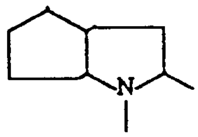

1

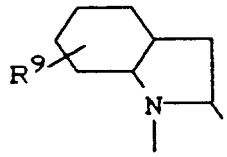

2

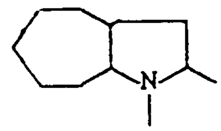

3

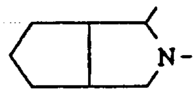

4

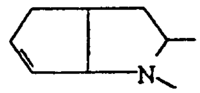

5

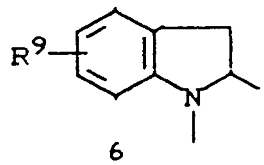

6

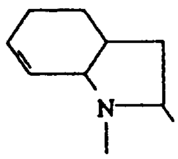

7

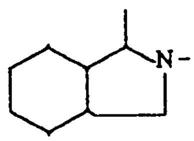

8

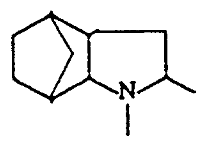

9

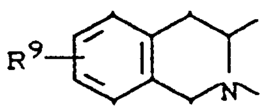

10

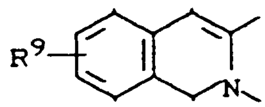

11

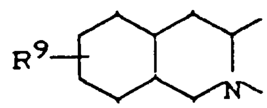

12

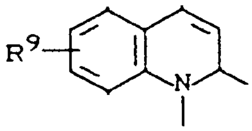

13

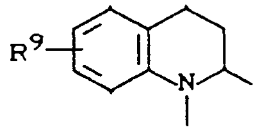

14

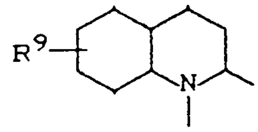

15

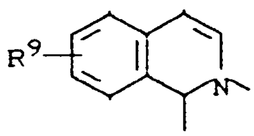

16

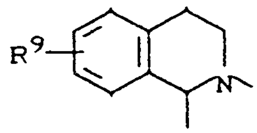

17

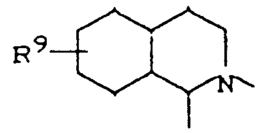

18

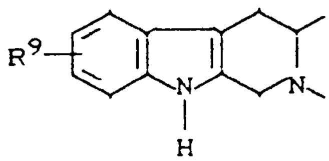

19

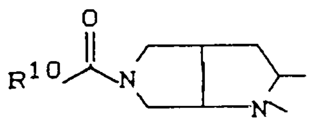

20

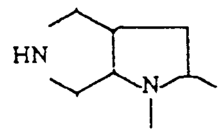

21

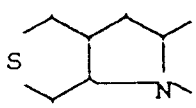

22

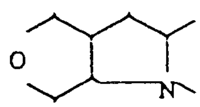

23

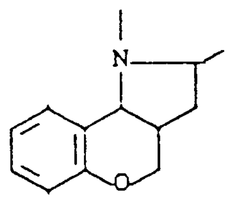

24

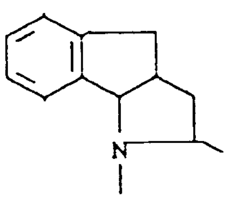

25

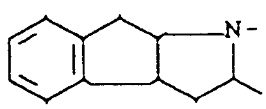

26

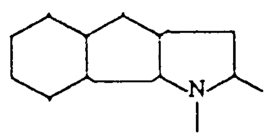

27

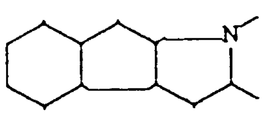

28

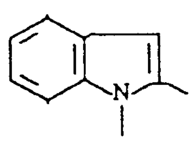

29

X für $C_1$-$C_6$-Alkyl, Halogen oder $C_1$-$C_6$-Alkoxy steht,
Y für Wasserstoff, $C_1$-$C_6$-Alkyl, Halogen, $C_1$-$C_6$-Alkoxy, $C_1$-$C_3$-Halogenalkyl steht,
Z für $C_1$-$C_6$-Alkyl, Halogen, $C_1$-$C_6$-Alkoxy steht,
n für eine Zahl von 0-3 steht,
G für Wasserstoff (a) oder für die Gruppen

-CO-$R^1$, (b)

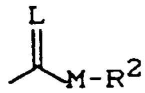 (c)

-$SO_2$-$R^3$ (d)

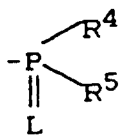 (e)

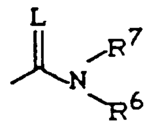 (f)

E (g)

steht,

in welchen

E für ein Metallionäquivalent oder ein Ammoniumion steht,

L und M für Sauerstoff und/oder Schwefel steht,

$R^1$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxyl-$C_2$-$C_8$-alkyl oder Cycloalkyl mit 3-8 Ringatomen, das durch Sauerstoffund/oder Schwefelatome unterbrochen sein kann, steht,

für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl;

für gegebenenfalls durch Halogen, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl-$C_1$-$C_6$-alkyl steht,

für gegebenenfalls durch Halogen und/oder $C_1$-$C_6$-Alkyl substituiertes Pyridyl, Pyrimidyl, Thiazolyl oder Pyrazolyl steht,

für gegebenenfalls durch Halogen und/oder $C_1$-$C_6$-Alkyl substituiertes Phenoxy-$C_1$-$C_6$-alkyl steht,

für gegebenenfalls durch Halogen, Amino und $C_1$-$C_6$-Alkyl substituiertes Pyridyloxy-$C_1$-$C_6$-alkyl, Pyrimidyloxy-$C_1$-$C_6$-alkyl oder Thiazolyloxy-$C_1$-$C_6$-alkyl steht,

$R^2$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,

für gegebenenfalls durch Halogen, Nitro, $C_1$-$C_6$-Alkyl, $C_1$-$C_6$-Alkoxy, $C_1$-$C_6$-Halogenalkylsubstituiertes Phenyl oder Benzyl steht,

$R^3$, $R^4$ und $R^5$ unabhängig voneinander für gegebenenfalls durch Halogen substituiertes $C_1$-$C_8$-Alkyl, $C_1$-$C_8$-Alkoxy, $C_1$-$C_8$-Alkylamino, Di-($C_1$-$C_8$)-Alkylamino, $C_1$-$C_8$-Alkylthio, $C_2$-$C_5$-Alkenylthio, $C_2$-$C_5$-Alkinylthio, $C_3$-$C_7$-Cycloalkylthio, für gegebenenfalls durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl, $C_1$-$C_4$-Halogenalkyl substituiertes Phenyl, Phenoxy oder Phenylthio stehen,

$R^6$ und $R^7$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_{20}$-Halogenalkyl, $C_1$-$C_{20}$-Alkyl oder $C_1$-$C_{20}$-Alkoxy substituiertes Phenyl, für gegebenenfalls durch Halogen, $C_1$-$C_{20}$-Alkyl, $C_1$-$C_{20}$-Halogenalkyl oder $C_1$-$C_{20}$-Alkoxy substituiertes Benzyl steht oder zusammen für einen gegebenenfalls durch Sauerstoff unterbrochenen $C_2$-$C_6$-Alkylenring stehen,

$R^9$ für Wasserstoff, Halogen oder gegebenenfalls durch Halogen substituiertes $C_1$-$C_2$-Alkyl oder $C_1$-$C_2$-Alkoxy steht,

$R^{10}$ für $C_1$-$C_7$-Alkoxy, Amino, $C_1$-$C_4$-Alkylamino, $C_1$-$C_4$-Dialkylamino steht.

**2.** Polycyclische 3-Arylpyrrolidin-2,4-dion-Derivate der Formel (I) gemäß Anspruch 1, dadurch gekennzeichnet, daß es sich um eine der folgenden Strukturen (Ia) bis (Ig) handelt:

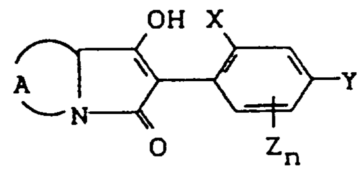

(Ia)

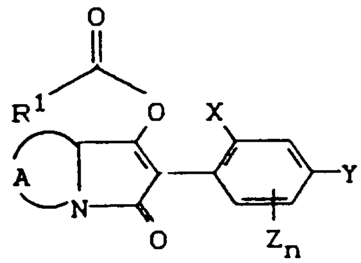

(Ib)

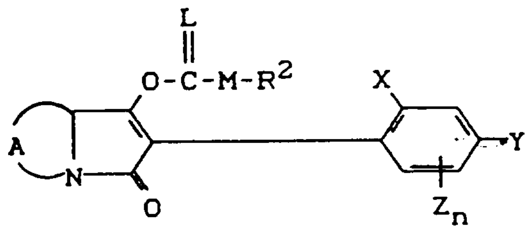

(Ic)

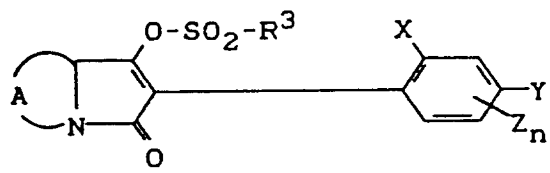

(Id)

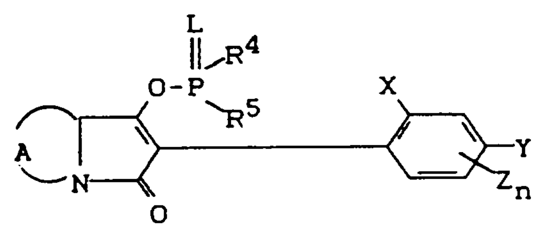

(Ie)

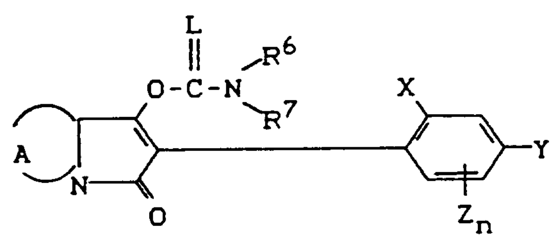

(If)

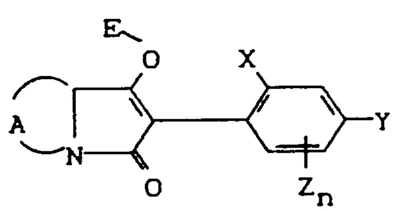

(Ig)

worin

A, E, L, M, X, Y, $Z_n$, $R^1$, $R^2$, $R^3$, $R^4$, $R^5$, $R^6$ und $R^7$ die in Anspruch 1 angegebenen Bedeutungen besitzen.

**3.** Polycyclische 3-Arylpyrrolidin-2,4-dion-Derivate der Formel (I) gemäß Anspruch 1, dadurch gekennzeichnet, daß die Gruppierung

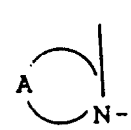

für eine der folgenden Bedeutungen 1 bis 29 steht:

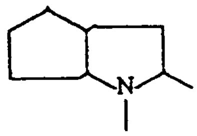

1

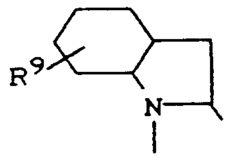

2

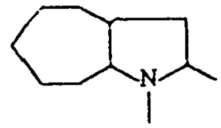

3

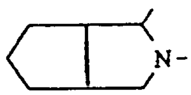

4

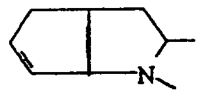

5

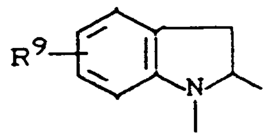

6

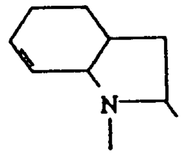

7

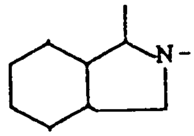

8

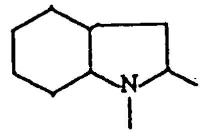

9

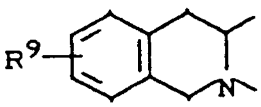

10

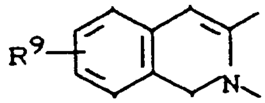

11

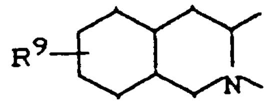

12

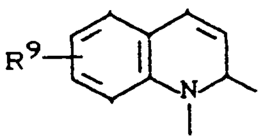

13

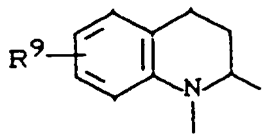

14

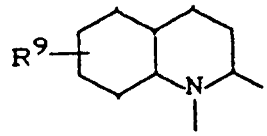

15

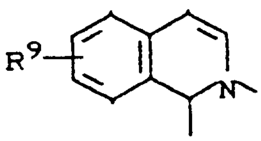

16

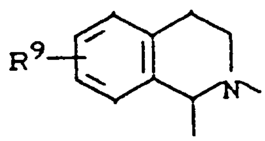

17

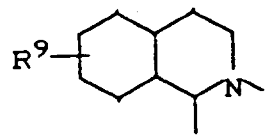

18

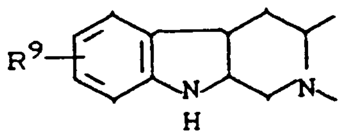

19

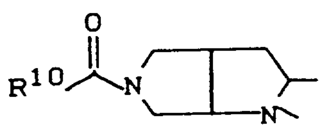

20

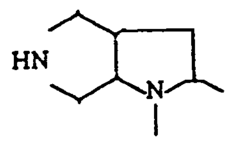

21

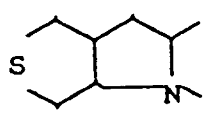

22

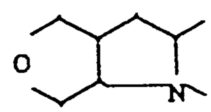

23

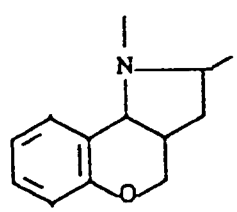

24

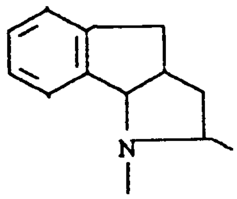 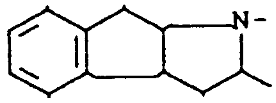 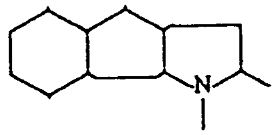

25 26 27

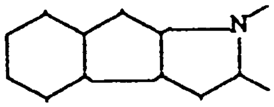 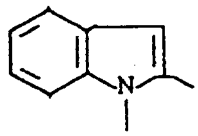

28 29

X für $C_1$-$C_4$-Alkyl, Halogen, $C_1$-$C_4$-Alkoxy steht,
Y für Wasserstoff, $C_1$-$C_4$-Alkyl, Halogen, $C_1$-$C_4$-Alkoxy, $C_1$-$C_2$-Halogenalkyl steht,
Z für $C_1$-$C_4$-Alkyl, Halogen, $C_1$-$C_4$-Alkoxy steht,
n für eine Zahl von 0-3 steht,
G für Wasserstoff (a) oder für die Gruppen

-CO-$R^1$, (b)

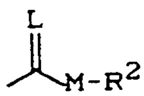 (c)

-$SO_2$-$R^3$ (d)

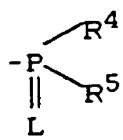 (e) 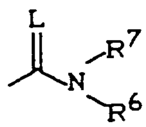 (f)

oder E (g)
steht,

in welchen

E für ein Metallionäquivalent oder ein Ammoniumion steht
L und M jeweils für Sauerstoff und/oder Schwefel steht,
$R^1$ für gegebenenfalls durch Halogen substituiertes $C_1$-$C_{16}$-Alkyl, $C_2$-$C_{16}$-Alkenyl, $C_1$-$C_6$-Alkoxy-$C_2$-$C_6$-alkyl, $C_1$-$C_{16}$-Alkylthio-$C_2$-$C_6$-alkyl, $C_1$.$C_6$-Polyalkoxy-$C_2$-$C_6$-alkyl und Cycloalkyl mit 3-7 Ringatomen, das durch 1-2 Sauerstoff- und/oder Schwefelatome unterbrochen sein kann, steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl steht,
für gegebenenfalls durch Halogen, $C_1$-$C_4$-Alkyl, $C_1$-$C_4$-Alkoxy, $C_1$-$C_3$-Halogenalkyl, $C_1$-$C_3$-Halogenalkoxy substituierets Phenyl-$C_1$-$C_4$-alkyl steht,
für gegebenenfalls durch Halogen und $C_1$-$C_6$-Alkyl substituiertes Pyridyl, Pyrimidyl, Thiazolyl oder Pyrazolyl steht,
für gegebenenfalls durch Halogen und $C_1$-$C_4$-Alkyl substituiertes Phenoxy-$C_1$-$C_5$-alkyl steht,
für gegebenenfalls durch Halogen, Amino, $C_1$-$C_4$-Alkyl-substituiertes Pyridyloxy-

$C_1$-$C_5$-alkyl, Pyrimidyloxy-$C_1$-$C_5$-alkyl oder Thiazolyloxy-$C_1$-$C_5$-alkyl steht,

$R^2$ für gegebenenfalls durch Halogen substituiertes $C_1$-$C_{16}$-Alkyl, $C_2$-$C_{16}$-Alkenyl, $C_1$-$C_{16}$-Alkox-$C_2$-$C_6$-alkyl, $C_1$-$C_6$-Polyalkoxy-$C_2$-$C_6$-alkyl steht, für gegebenenfalls durch Halogen, Nitro, $C_1$-$C_4$-Alkyl, $C_1$-$C_3$-Alkoxy, $C_1$-$C_3$-Halogenalkylsubstituiertes Phenyl oder Benzyl steht,

$R^3$, $R^4$ und $R^5$ unabhängig voneinander für gegebenenfalls durch Halogen substituiertes $C_1$-$C_6$-Alkyl, $C_1$-$C_6$-Alkoxy, $C_1$-$C_6$-Alkylamino, Di-($C_1$-$C_6$)-Alkylamino, $C_1$-$C_6$-Alkylthio, $C_3$-$C_4$-Alkenylthio, $C_2$-$C_4$-Alkinylthio, $C_3$-$C_6$-Cycloalkylthio, für gegebenenfalls durch Fluor, Chlor, Brom, Nitro, Cyano, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkyl, $C_1$-$C_3$-Halogenalkyl substiutiertes Phenyl, Phenoxy oder Phenylthio stehen,

$R^6$ und $R^7$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_5$-Halogenalkyl, $C_1$-$C_5$-Alkyl oder $C_1$-$C_5$-Alkoxy substituiertes Phenyl, für gegebenenfalls durch Halogen, $C_1$-$C_5$-Alkyl, $C_1$-$C_5$-Halogenalkyl oder $C_1$-$C_5$-Alkoxy substituiertes Benzyl steht,

$R^9$ für Wasserstoff, Halogen oder gegebenenfalls durch Halogen substituiertes $C_1$-$C_2$-Alkyl oder $C_1$-$C_2$-Alkoxy steht,

$R^{10}$ für $C_1$-$C_7$-Alkoxy, Amino, $C_1$-$C_4$-Alkylamino, $C_1$-$C_4$-Dialkylamino steht.

**4.** Polycyclische 3-Arylpyrrolidin-2,4-dion-Derivate der Formel (I) gemäß Anspruch 1, dadurch gekennzeichnet, daß die Gruppierung

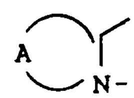

für eine der folgenden Bedeutungen 1 bis 29 steht:

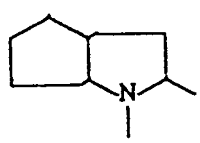

1

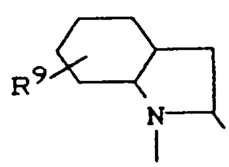

2

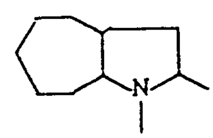

3

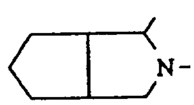

4

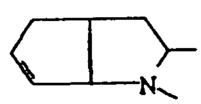

5

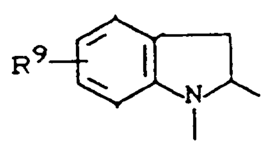

6

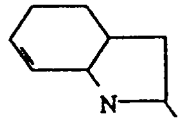
7

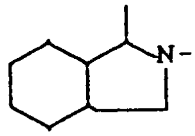
8

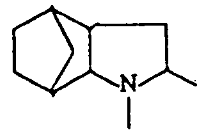
9

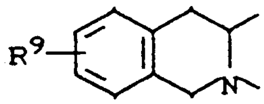
10

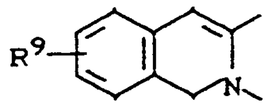
11

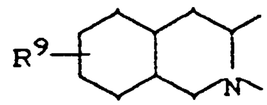
12

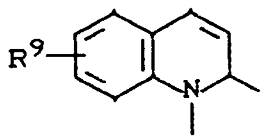
13

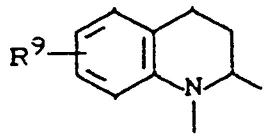
14

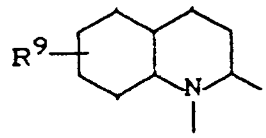
15

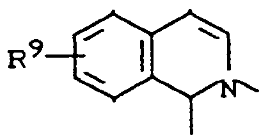
16

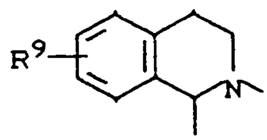
17

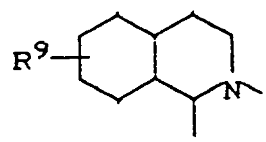
18

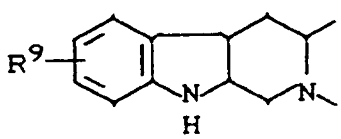
19

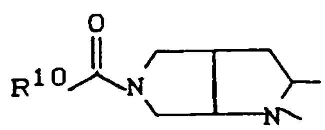
20

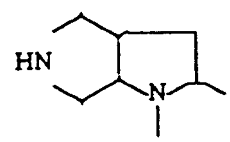
21

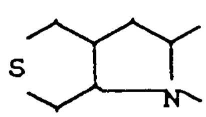
22

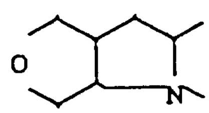
23

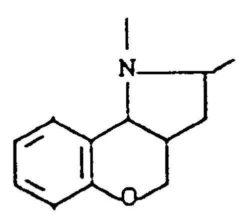
24

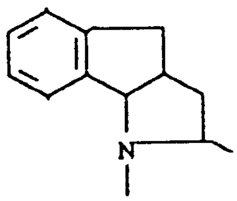 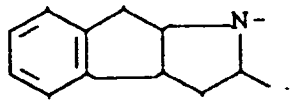 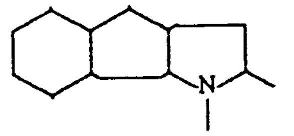

25 26 27

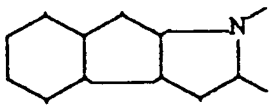 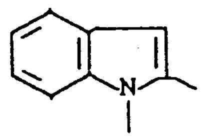

28 29

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,

Z für Methyl, Ethyl, i-Propyl, Butyl, i-Butyl, tert.-Butyl, Fluor, Chlor, Brom, Methoxy und Ethoxy steht,

n für eine Zahl von 0-3 steht,

G für Wasserstoff (a) oder für die Gruppen

$-CO-R^1$, (b)

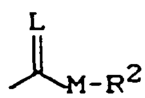 (c)

$-SO_2-R^3$ (d)

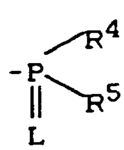 (e) 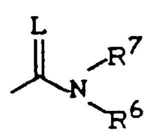 (f)

oder E (g) steht,

in welchen

E für ein Metallionäquivalent oder ein Ammoniumion steht,

L und H für Sauerstoff und/oder Schwefel steht,

$R^1$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Alkylthio-$C_2$-$C_6$-alkyl, $C_1$-$C_4$-Polyalkoxyl-$C_2$-$C_4$-alkyl und Cycloalkyl mit 3-6 Ringatomen, das durch 1-2 Sauerstoff- und/oder Schwefelatome unterbrochen sein kann, steht,

für gegebenenfalls durch Fluor, Chlor, Brom, Methyl, Ethyl, Propyl, i-Propyl, Methoxy, Ethoxy, Trifluormethyl, Trifluormethoxy, Nitro-substituiertes Phenyl steht,

für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-$C_1$-$C_3$-alkyl steht,

für gegebenenfalls durch Fluor, Chlor, Brom, Methyl, Ethyl substituiertes Pyridyl, Pyrimidyl, Thiazolyl oder Pyrazolyl steht,

für gegebenenfalls durch Fluor, Chlor, Methyl, Ethyl substituiertes Phenoxy-$C_1$-$C_4$-alkyl steht,
für gegebenenfalls durch Fluor, Chlor, Amino, Methyl, Ethyl, substituiertes Pyridyloxy-$C_1$-$C_4$-alkyl, Pyrimidyloxy-$C_1$-$C_4$-alkyl oder Thiazolyloxy-$C_1$ -$C_4$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht, oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,

$R^3$, $R^4$ und $R^5$ unabhängig voneinander für gegebenenfalls durch Fluor oder Chlor substituiertes $C_1$-$C_4$-Alkyl, $C_1$-$C_4$-Alkoxy, $C_1$-$C_4$-Alkylamino, Di-($C_1$-$C_4$-Alkyl)-amino, $C_1$-$C_4$-Alkylthio, für gegebenenfalls durch Fluor, Chlor, Brom, Nitro, Cyano, $C_1$-$C_2$-Alkoxy, $C_1$-$C_4$-Fluoralkoxy, $C_1$-$C_2$-Chloralkoxy, $C_1$-$C_2$-Alkylthio, $C_1$-$C_2$-Fluoralkylthio, $C_1$-$C_2$-Chloralkylthio, $C_1$-$C_3$-Alkyl substituiertes Phenyl, Phenoxy oder Phenylthio stehen,

$R^6$ und $R^7$ unabhängig voneinander für gegebenenfalls durch Fluor, Chlor, Brom substituiertes $C_1$-$C_{10}$-Alkyl, $C_1$-$C_{10}$-Alkoxy, $C_1$-$C_{10}$-Alkoxy-($C_1$-$C_{10}$)alkyl, für gegebenenfalls durch Fluor, Chlor, Brom $C_1$-$C_{20}$-Halogenalkyl, $C_1$-$C_{20}$-Alkyl oder $C_1$-$C_4$-Alkoxy substituiertes Phenyl, für gegebenenfalls durch Fluor, Chlor, Brom, $C_1$-$C_4$-Alkyl, $C_1$-$C_4$-Halogenalkyl oder $C_1$-$C_4$-Alkoxy substituiertes Benzyl steht,

$R^9$ für Wasserstoff, Halogen oder gegebenenfalls durch Halogen substituiertes $C_1$-$C_2$-Alkyl oder $C_1$-$C_2$-Alkoxy steht,

$R^{10}$ für Methoxy, Ethoxy, Propoxy, iso-Propoxy, Butoxy, iso-Butoxy, sek.-Butoxy, tert.-Butoxy, Phenoxy, Benzyloxy, Amino, $C_1$-$C_2$-Alkylamino, $C_1$-$C_2$-Dialkylamino steht.

**5.** Verfahren zur Herstellung von polycyclischen 3-Arylpyrrolidin-2,4-dion-Derivaten der Formel (I) gemäß Anspruch 1

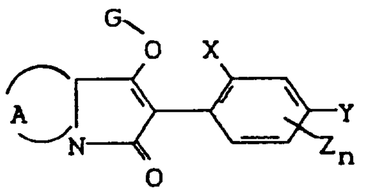
(I)

in welcher
A, G, X, Y, Z und n die in Anspruch 1 angegebene Bedeutung haben
dadurch gekennzeichnet, daß man zum Erhalt von 3-Aryl-pyrrolidin-2,4-dionen bzw. deren Enolen der Formel (Ia)

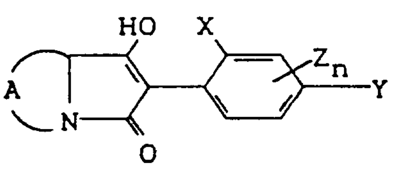
(Ia)

in welcher
A, X, Y, Z und n die oben angegebene Bedeutung haben,
(A)
N-Acylaminosäureester der Formel (II)

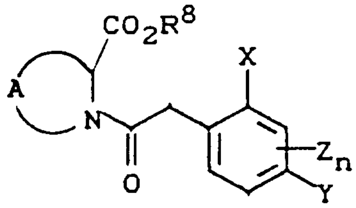

(II)

in welcher

A, Y, Z und n die oben angegebene Bedeutung haben

und

$R^8$ für Alkyl steht,

in Gegenwart eines Verdünnungsmittels und in Gegenwart einer Base intramolekular kondensiert.

(B)

oder daß man zum Erhalt von Verbindungen der Formel (Ib)

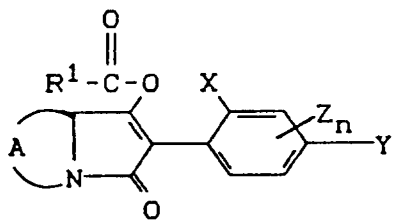

(Ib)

in welcher

A, X, Y, Z, $R^1$ und n die oben angegebene Bedeutung haben,

Verbindungen der Formel (Ia),

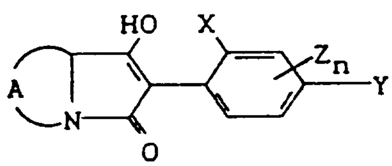

(Ia)

in welcher

A, X, Y, Z und n die oben angegebene Bedeutung haben,

α) mit Säurehalogeniden der allgemeinen Formel (III)

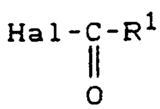

(III)

in welcher

$R^1$ die oben angegebene Bedeutung hat

und

Hal für Halogen, insbesondere Chlor und Brom steht,

gegebenenfalls in Gegenwart eines Verdünnungsmittels und gegebenenfalls in Gegenwart eines Säurebindemittels umsetzt oder

β) mit Carbonsäureanhydriden der allgemeinen Formel (IV)

$R^1$-CO-O-CO-$R^1$ (IV)

in welcher

$R^1$ die oben angegebene Bedeutung hat,

gegebenenfalls in Gegenwart eines Verdünnungsmittels und gegebenenfalls in Gegenwart eines Säurebindemittels,

umsetzt,

(C)

oder daß man zum Erhalt von Verbindungen der Formel (Ic)

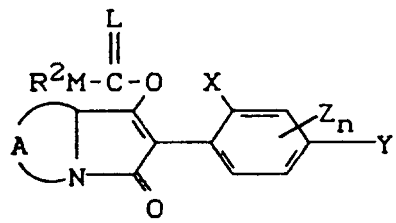

(Ic)

in welcher

A, X, Y, Z, $R^2$ und n die oben angegebene Bedeutung haben,

L für Sauerstoff

und

M für Sauerstoff oder Schwefel steht,

Verbindungen der Formel (Ia)

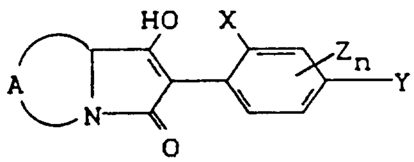

(Ia)

in welcher

A, X, Y, Z und n die oben angegebene Bedeutung haben,

mit Chlorameisensäureester oder Chlorameisensäurethiolester der allgemeinen Formel (V)

$R^2$-M-CO-Cl (V)

in welcher

$R^2$ und M die oben angegebene Bedeutung haben,

gegebenenfals in Gegenwart eines Verdünnungsmittels und gegebenenfalls in Gegenwart eines Säurebindemittels umsetzt,

(D)

oder daß man zum Erhalt von Verbindungen der Formel (Ic)

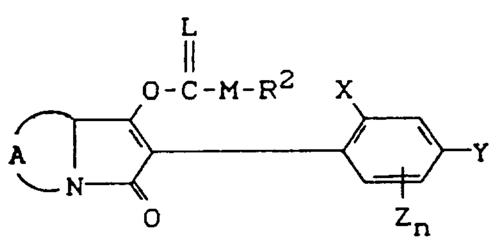

(Ic)

in welcher

A, $R^2$, X, Y, Z und n die oben angegebene Bedeutung haben,

L für Schwefel

und

M für Sauerstoff oder Schwefel steht,

Verbindungen der Formel (Ia)

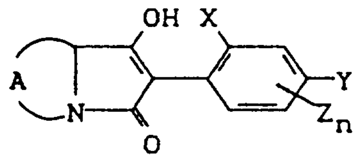

(Ia)

in welcher

A, X, Y, Z und n die oben angegebene Bedeutung haben,

α) mit Chlormonothioameisensäureestern oder Chlordithioameisensäureestern der allgemeinen Formel (VI)

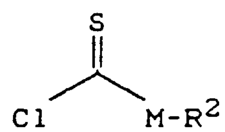

(VI)

in welcher

M und $R^2$ die oben angegebene Bedeutung haben,

gegebenenfalls in Gegenwart eines Verdünnungsmittels und gegebenenfalls in Gegenwart eines Säurebindemittels umsetzt,

oder

β) mit Schwefelkohlenstoff und anschließend mit Alkylhalogeniden der allgemeinen Formel (VII)

$R^2$-Hal (VII)

in welcher

$R^2$ die oben angegebene Bedeutung hat

und

Hal für Chlor, Brom, Jod

steht, umsetzt,

(E)

oder daß man zum Erhalt von Verbindungen der Formel (Id)

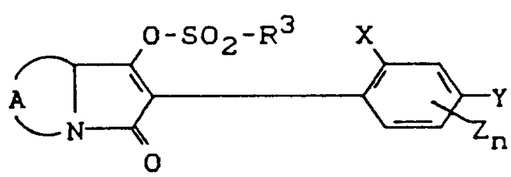

(Id)

in welcher

A, X, Y, Z, $R^3$ und n die oben angegebene Bedeutung haben,

Verbindungen der Formel (Ia)

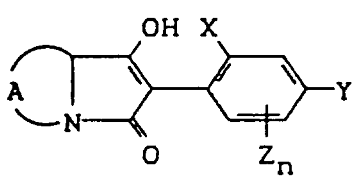

(Ia)

in welcher

A, X, Y, Z und n die oben angegebene Bedeutung haben,
mit Sulfonsäurechloriden der allgemeinen Formel (VIII)

$R^3$-$SO_2$-Cl (VIII)

in welcher
$R^3$ die oben angegebene Bedeutung hat
gegebenenfalls in Gegenwart eines Verdünnungsmittels und gegebenenfalls in Gegenwart eines Säurebindemittels,
umsetzt,
(F)
oder daß man zum Erhalt von 3-Aryl-pyrrolidin-2,4-dionen der Formel (Ie)

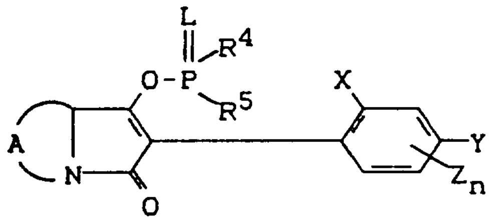

(Ie)

in welcher
A, L, X, Y, Z, $R^4$, $R^5$ und n die oben angegebene Bedeutung haben,
3-Aryl-pyrrolidin-2,4-dione der Formel (Ia) bzw. deren Enole der Formel

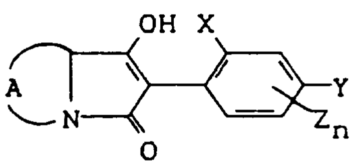

(Ia)

in welcher
A, X, Y, Z und n die oben angegebene Bedeutung haben
mit Phosphorverbindungen der allgemeinen Formel (IX)

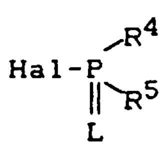

(IX)

in welcher
L, $R^4$ und $R^5$ die oben angegebene Bedeutung haben
und
Hal für Halogen, insbesondere Chlor und Brom steht,
gegebenenfalls in Gegenwart eines Verdünnungsmittels und gegebenenfalls in Gegenwart eines Säurebindemittels umsetzt,
(G)
oder daß man zum Erhalt von Verbindungen der Formel (If)

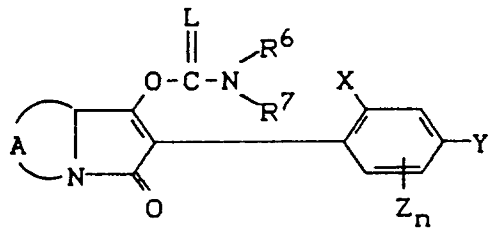

(If)

in welcher
A, L, X, Y, Z, $R^6$, $R^7$ und n die oben angegebene Bedeutung haben,
Verbindungen der Formel (Ia),

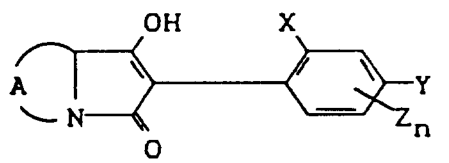

(Ia)

in welcher
A, X, Y, Z und n die oben angegebene Bedeutung haben,
α) mit Isocyanaten der allgemeinen Formel (X)

$R^6$-N=C=O (X)

in welcher
$R^6$ die oben angegebene Bedeutung hat,
gegebenenfalls in Gegenwart eines Verdünnungsmittels und gegebenenfalls in Gegenwart eines Katalysators, umsetzt,
oder
β) mit Carbamidsäurechloriden oder Thiocarbamidsäurechloriden der allgemeinen Formel (XI)

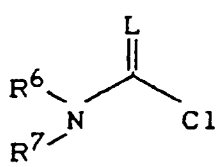

(XI)

in welcher
L, $R^6$ und $R^7$ die oben angegebene Bedeutung haben,
gegebenenfalls in Gegenwart eines Verdünnungsmittels und gegebenenfalls in Gegenwart eines Säurebindemittels,
umsetzt,
(H)
oder daß man zum Erhalt von Verbindungen der Formel (Ig)

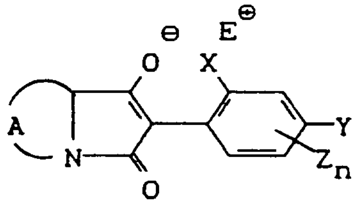

(Ig)

in welcher
X, Y, Z, A und n die oben angegebene Bedeutung haben,
Verbindungen der Formel (Ia)

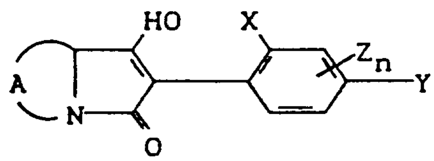

(Ia)

in welcher
X, Y, Z A, und n die oben angegebene Bedeutung haben,
mit Metallhydroxiden oder Aminen der allgemeinen Formeln (XII) und (XIII)

$Me_sOH_t$ (XII)

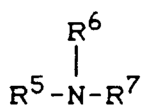

(XIII)

in welchen

| | |
|---|---|
| Me | für ein- oder zweiwertige Metallionen |
| s und t | für die Zahl 1 und 2 und |
| $R^5$, $R^6$ und $R^7$ | unabhängig voneinander für Wasserstoff und Alkyl |

stehen,
gegebenenfalls in Gegenwart eines Verdünnungsmittels, umsetzt.

**6.** Insektizide, akarizide und herbizide Mittel, gekennzeichnet durch einen Gehalt an mindestens einem polycyclischen 3-Arylpyrrolidin-2,4-dion-Derivat der Formel (I) gemäß Anspruch 1.

**7.** Verfahren zur Bekämpfung von Insekten, Spinnentieren und/oder Unkräutern, dadurch gekennzeichnet, daß man polycyclische 3-Arylpyrrolidin-2,4-dion-Derivate der Formel (I) gemäß Anspruch 1 auf Insekten, Spinnentiere und/oder Unkräuter und/oder deren Lebensraum einwirken läßt.

**8.** Verwendung von poycyclischen 3-Arylpyrrolidin-2,4-dion-Derivaten der Formel (I) gemäß Anspruch 1 zur Bekämpfung von Insekten, Spinnentieren und/oder Unkräutern.

**9.** Verfahren zur Herstellung von insektiziden, akariziden und herbiziden Mitteln, dadurch gekennzeichnet, daß man polycyclische 3-Arylpyrrolidin-2,4-dion-Derivate der Formel (I) gemäß Anspruch 1, mit Streckmitteln und/oder oberflächenaktiven Mitteln vermischt.

## Claims

**1.** Polycyclic 3-arylpyrrolidine-2,4-dione derivatives of the formula (I)

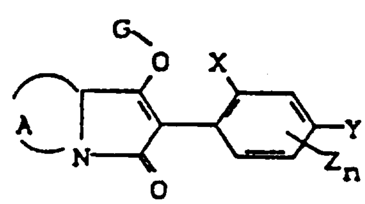

(I)

in which
the group

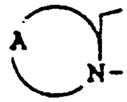

assumes one of the following meanings 1 to 29:

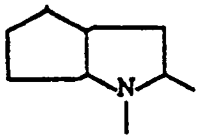

1

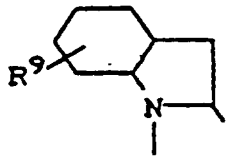

2

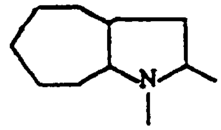

3

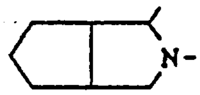

4

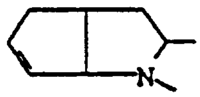

5

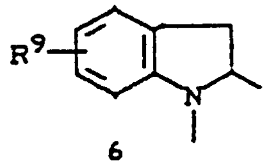

6

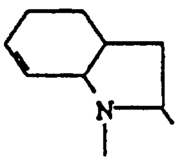

7

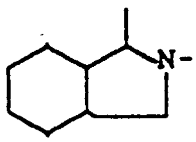

8

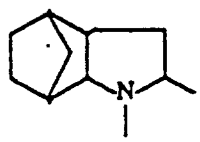

9

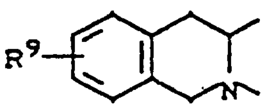

10

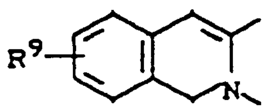

11

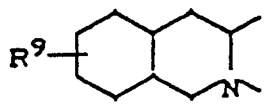

12

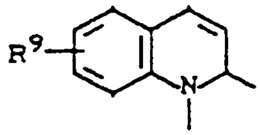
13

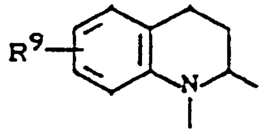
14

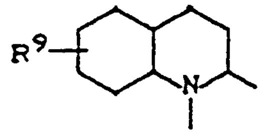
15

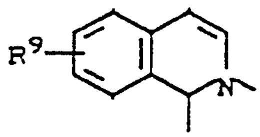
16

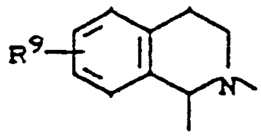
17

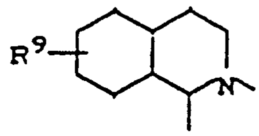
18

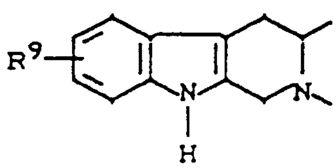
19

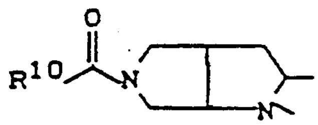
20

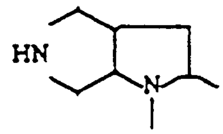
21

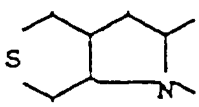
22

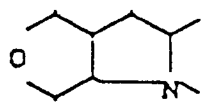
23

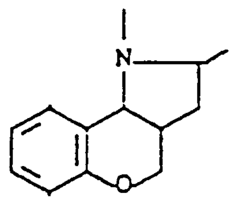
24

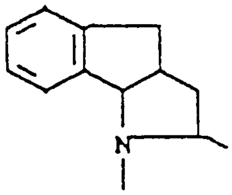
25

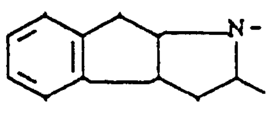
26

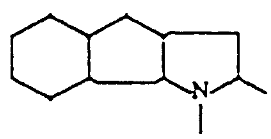
27

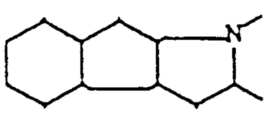
28

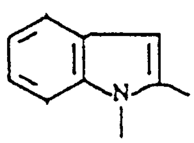
29

X represents $C_1$-$C_6$-alkyl, halogen or $C_1$-$C_6$-alkoxy,
Y represents hydrogen, $C_1$-$C_6$-alkyl, halogen, $C_1$-$C_6$-alkoxy or $C_1$-$C_3$-halogenoalkyl,
Z represents $C_1$-$C_6$-alkyl, halogen or $C_1$-$C_6$-alkoxy,
n represents a number from 0-3,
G represents hydrogen (a) or represents the groups

-CO-$R^1$, (b)

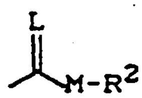 (c)

-$SO_2$-$R^3$ (d)

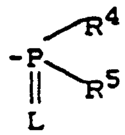 (e)

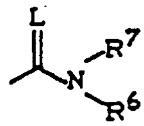 (f)

E (g)

in which

E represents a metal ion equivalent or an ammonium ion,

L and M represent oxygen and/or sulphur,

$R^1$ represents optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-alkylthio-$C_2$-$C_8$-alkyl, $C_1$-$C_8$-polyalkoxy-$C_2$-$C_8$-alkyl or cycloalkyl which has 3-8 ring atoms and which can be interrupted by oxygen and/or sulphur atoms,
or represents phenyl optionally substituted by halogen, nitro, $C_1$-$C_6$-alkyl, $C_1$-$C_6$-alkoxy, $C_1$-$C_6$-halogenoalkyl or $C_1$-$C_6$-halogenoalkoxy;
or represents phenyl-$C_1$-$C_6$-alkyl optionally substituted by halogen, $C_1$-$C_6$-alkyl, $C_1$-$C_6$-alkoxy, $C_1$-$C_6$-halogenoalkyl, $C_1$-$C_6$-halogenoalkoxy,
or represents pyridyl, pyrimidyl, thiazolyl or pyrazolyl which is optionally substituted by halogen and/or $C_1$-$C_6$-alkyl,
or represents phenoxy-$C_1$-$C_6$-alkyl optionally substituted by halogen and/or $C_1$-$C_6$-alkyl,
or represents pyridyloxy-$C_1$-$C_6$-alkyl, pyrimidyloxy-$C_1$-$C_6$-alkyl or thiazolyloxy-$C_1 C_6$-alkyl optionally substituted by halogen, amino and $C_1$-$C_6$-alkyl,

$R^2$ represents optionally halogen-substituted $C_1$-$C_{20}$-alkyl, $C_2$-$C_{20}$-alkenyl, $C_1$-$C_8$-alkoxy-$C_2$-$C_8$-alkyl or $C_1$-$C_8$-polyalkoxy-$C_2$-$C_8$-alkyl,
or represents phenyl or benzyl optionally substituted by halogen, nitro, $C_1$-$C_6$-alkyl, $C_1$-$C_6$-alkoxy or $C_1$-$C_6$-halogenoalkyl,

$R^3$, $R^4$ and $R^5$ independently of one another represent $C_1$-$C_8$-alkyl, $C_1$-$C_8$-alkoxy, $C_1$-$C_8$-alkylamino, di-($C_1$-$C_8$)-alkylamino, $C_1$-$C_8$-alkylthio, $C_2$-$C_5$-alkenylthio, $C_2$-$C_5$-alkinylthio or $C_3$-$C_7$-cycloalkylthio, each of which is optionally substituted by halogen, or represents phenyl, phenoxy or phenylthio, each of which is optionally substituted by halogen, nitro, cyano, $C_1$-$C_4$-alkoxy, $C_1$-$C_4$-halogenoalkoxy, $C_1$-$C_4$-alkylthio, $C_1$-$C_4$-halogenoalkylthio, $C_1$-$C_4$-alkyl or $C_1$-$C_4$-halogenoalkyl,

$R^6$ and $R^7$ independently of one another represent $C_1$-$C_{20}$-alkyl, $C_1$-$C_{20}$-alkoxy, $C_2$-$C_8$-alkenyl or $C_1$-$C_{20}$-alkoxy-$C_1$-$C_{20}$-alkyl, each of which is optionally substituted by halogen, or represent phenyl which is optionally substituted by halogen, $C_1$-$C_{20}$-halogenoalkyl, $C_1$-$C_{20}$-alkyl or $C_1$-$C_{20}$-alkoxy, or represent benzyl which is optionally substituted by halogen, $C_1$-$C_{20}$-alkyl, $C_1$- -$C_{20}$-halogenoalkyl or $C_1$-$C_{20}$-alkoxy, or together represent a $C_2$-$C_6$-alkylene ring which is optionally interrupted by oxygen,

$R^9$ represents hydrogen or halogen or represents $C_1$-$C_2$-alkyl or $C_1$-$C_2$-alkoxy, each of which is optionally substituted by halogen, and

$R^{10}$ represents $C_1$-$C_7$-alkoxy, amino, $C_1$-$C_4$-alkylamino or $C_1$-$C_4$-dialkylamino.

**2.** Polycyclic 3-arylpyrrolidine-2,4-dione derivatives of the formula (I) according to Claim 1, characterized in that they are one of the following structures (Ia) to (Ig):

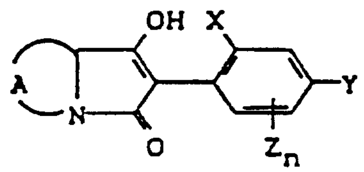

(Ia)

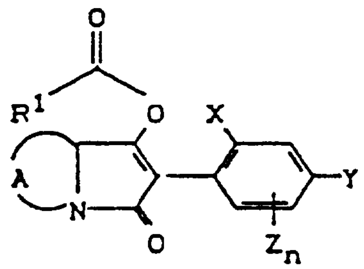

(Ib)

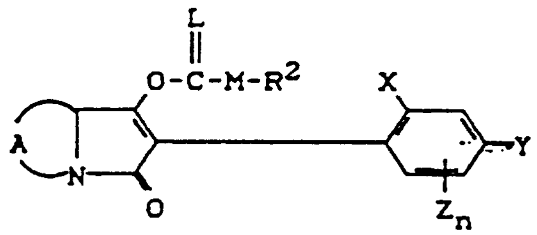

(Ic)

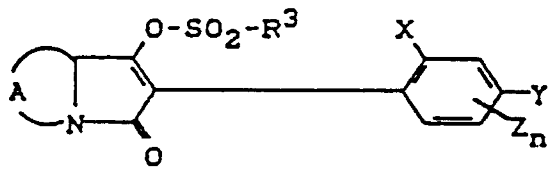

(Id)

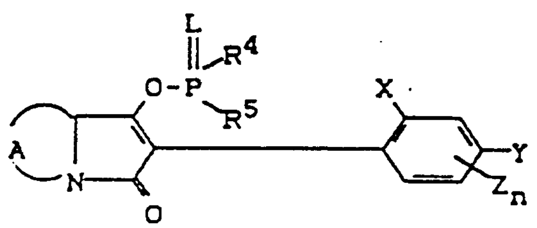

(Ie)

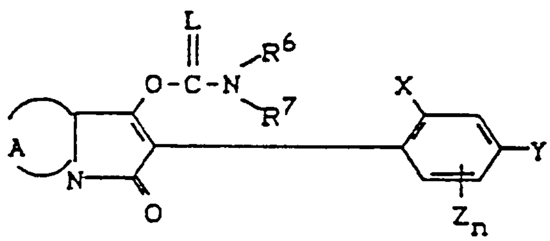

(If)

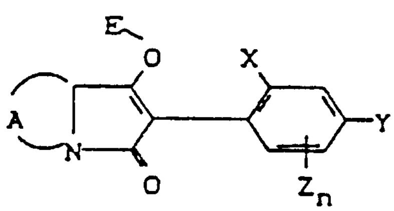

(Ig)

where
A, E, L, M, X, Y, $Z_n$, $R^1$, $R^2$, $R^3$, $R^4$, $R^5$, $R^6$ and $R^7$ have the meanings given in Claim 1.

**3.** Polycyclic 3-arylpyrrolidine-2,4-dione derivatives of the formula (I) according to Claim 1, characterized in that the group

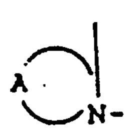

represents one of the following meanings 1 to 29:

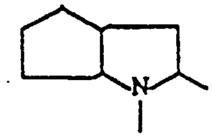

1

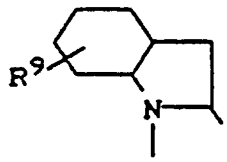

2

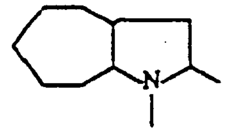

3

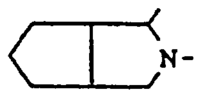

4

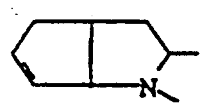

5

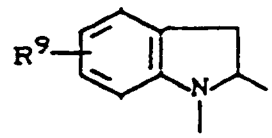

6

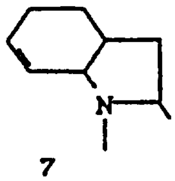

7

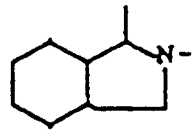

8

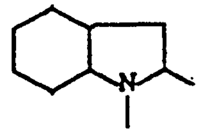

9

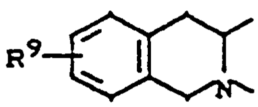

10

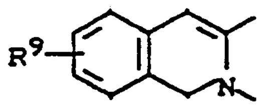

11

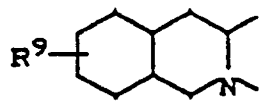

12

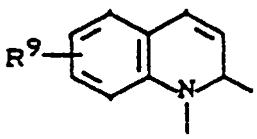

13

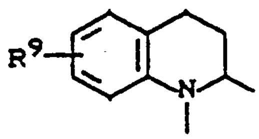

14

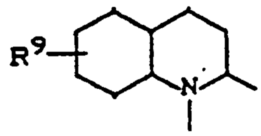

15

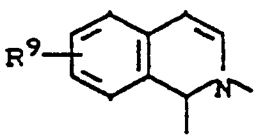

16

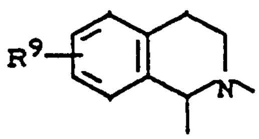

17

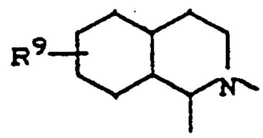

18

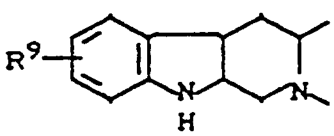

19

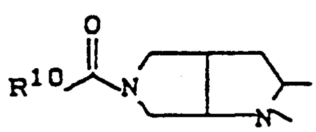

20

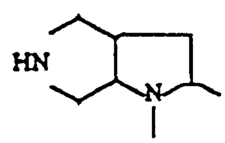

21

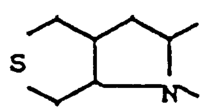

22

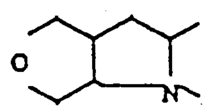

23

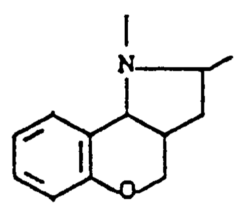

24

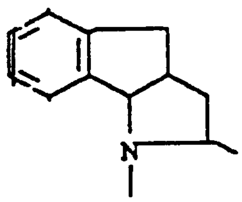

25

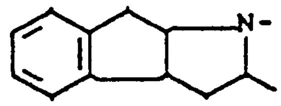

26

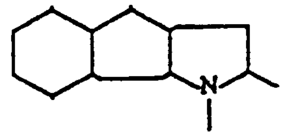

27

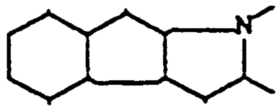

28

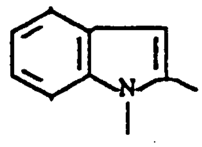

29

X represents $C_1$-$C_4$-alkyl, halogen or $C_1$-$C_4$-alkoxy,
Y represents hydrogen, $C_1$-$C_4$-alkyl, halogen, $C_1$-$C_4$-alkoxy or $C_1$-$C_2$-halogenoalkyl,
Z represents $C_1$-$C_4$-alkyl, halogen or $C_1$-$C_4$-alkoxy,
n represents a number from 0-3,
G represents hydrogen (a), or represents the groups

$-CO-R^1$, (b)

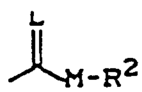 (c)

$-SO_2-R^3$ (d)

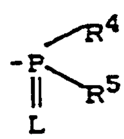 (e)

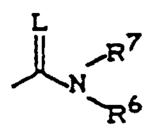 (f)

or E (g)

in which

E represents a metal ion equivalent or an ammonium ion,

L and M in each case represent oxygen and/or sulphur,

$R^1$ represents optionally halogen-substituted $C_1$-$C_{16}$-alkyl, $C_2$-$C_{16}$-alkenyl, $C_1$-$C_6$-alkoxy-$C_2$-$C_6$-alkyl, $C_1$-$C_{16}$-alkylthio-$C_2$-$C_6$-alkyl, $C_1$-$C_6$-polyalkoxy-$C_2$-$C_6$-alkyl and cycloalkyl which has 3-7 ring atoms and which can be interrupted by 1-2 oxygen and/or sulphur atoms,
or represents phenyl optionally substituted by halogen, nitro, $C_1$-$C_4$-alkyl, $C_1$-$C_4$-alkoxy, $C_1$-$C_3$-halogenoalkyl or $C_1$-$C_3$-halogenoalkoxy,
or represents phenyl-$C_1$-$C_4$-alkyl optionally substituted by halogen, $C_1$-$C_4$-alkyl, $C_1$-$C_4$-alkoxy, $C_1$-$C_3$-halogenoalkyl or $C_1$-$C_3$-halogenoalkoxy,
or represents pyridyl, pyrimidyl, thiazolyl or pyrazolyl optionally subsituted by halogen and $C_1$-$C_6$-alkyl,
or represents phenoxy-$C_1$-$C_5$-alkyl optionally substituted by halogen and $C_1$-$C_4$-alkyl,
or represents pyridyloxy-$C_1$-$C_5$-alkyl, pyrimidyloxy-$C_1$-$C_5$-alkyl or thiazolyloxy-$C_1$-$C_5$-alkyl optionally substituted by halogen, amino or $C_1$-$C_4$-alkyl,

$R^2$ represents optionally halogen-substituted $C_1$-$C_{16}$-alkyl, $C_2$-$C_{16}$-alkenyl, $C_1$-$C_{16}$-alkoxy-$C_2$-$C_6$-alkyl or $C_1$-$C_6$-polyalkoxy-$C_2$-$C_6$-alkyl,
or represents phenyl or benzyl optionally substituted by halogen, nitro, $C_1$-$C_4$-alkyl, $C_1$-$C_3$-alkoxy or $C_1$-$C_3$-halogenoalkyl,

$R^3$, $R^4$ and $R^5$ independently of one another represent $C_1$-$C_6$-alkyl, $C_1$-$C_6$-alkoxy, $C_1$-$C_6$-alkylamino, di-($C_1$-$C_6$)-alkylamino, $C_1$-$C_6$-alkylthio, $C_3$-$C_4$-alkenylthio, $C_2$-$C_4$-alkinylthio or $C_3$-$C_6$-cycloalkylthio, each of which is optionally substituted by halogen, or represent phenyl, phenoxy or phenylthio, each of which is optionally substituted by fluorine, chlorine, bromine, nitro, cyano, $C_1$-$C_3$-alkoxy, $C_1$-$C_3$-halogenoalkoxy, $C_1$-$C_3$-alkylthio, $C_1$-$C_3$-halogenoalkylthio, $C_1$-$C_3$-alkyl or $C_1$-$C_3$-halogenoalkyl,

$R^6$ and $R^7$ independently of one another represent $C_1$-$C_{20}$-alkyl, $C_1$-$C_{20}$-alkoxy, $C_2$-$C_8$-alkenyl or $C_1$-$C_{20}$-alkoxy-$C_1$-$C_{20}$-alkyl, each of which is optionally substituted by halogen, represent phenyl which is optionally substituted by halogen, $C_1$-$C_5$-halogenoalkyl, $C_1$-$C_5$-alkyl or $C_1$-$C_5$-alkoxy, or represent benzyl which is optionally substituted by halogen, $C_1$-$C_5$-alkyl, $C_1$-$C_5$-halogenoalkyl or $C_1$-$C_5$-alkoxy,

$R^9$ represents hydrogen or halogen or represents $C_1$-$C_2$-alkyl or $C_1$-$C_2$-alkoxy, each of which is optionally substituted by halogen, and

$R^{10}$ represents $C_1$-$C_7$-alkoxy, amino, $C_1$-$C_4$-alkylamino or $C_1$-$C_4$-dialkylamino.

4. Polycyclic 3-arylpyrrolidine-2,4-dione derivatives of the formula (I) according to Claim 1, characterized in that the group

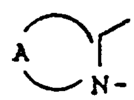

represents one of the following meanings 1 to 29:

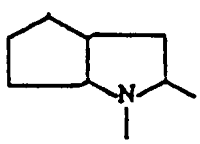

1

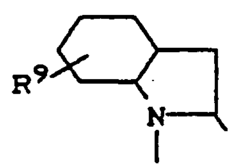

2

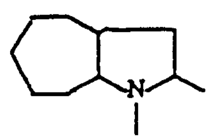

3

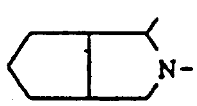

4

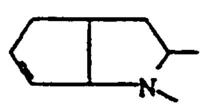

5

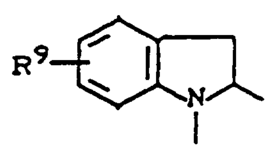

6

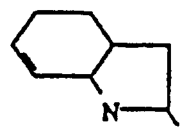
7

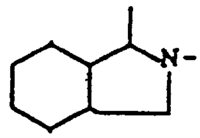
8

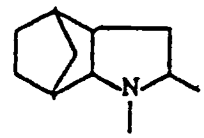
9

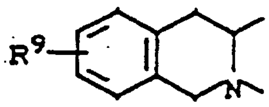
10

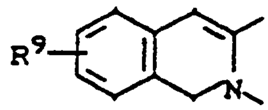
11

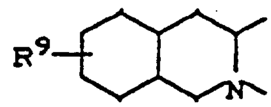
12

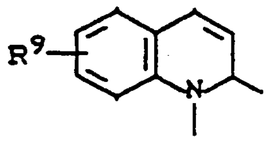
13

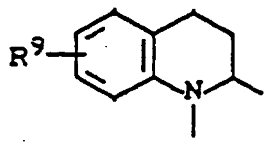
14

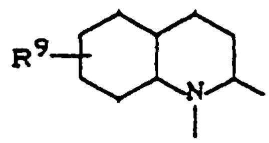
15

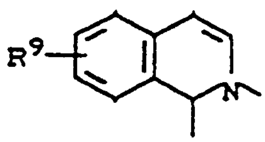
16

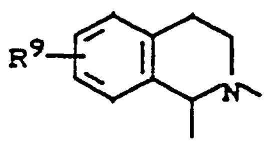
17

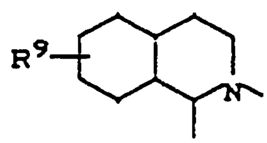
18

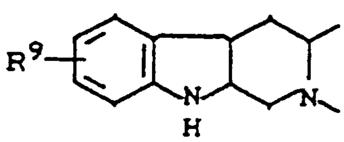
19

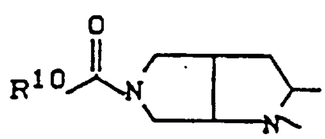
20

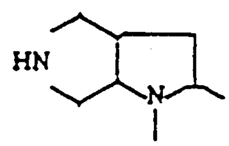
21

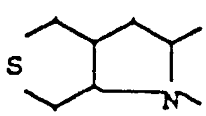
22

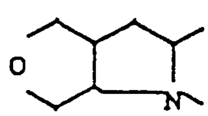
23

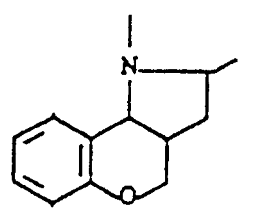
24

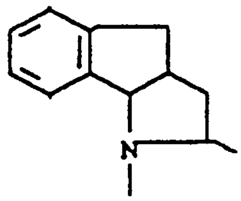

25

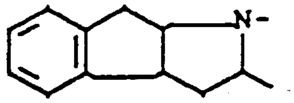

26

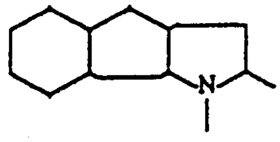

27

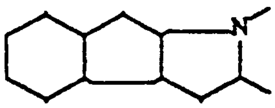

28

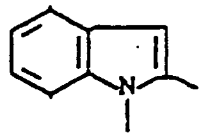

29

X represents methyl, ethyl, propyl, i-propyl, fluorine, chlorine, bromine, methoxy and ethoxy,

Y represents hydrogen, methyl, ethyl, propyl, i-propyl, butyl, i-butyl, tert.-butyl, fluorine, chlorine, bromine, methoxy, ethoxy and trifluoromethyl,

Z represents methyl, ethyl, i-propyl, butyl, i-butyl, tert.-butyl, fluorine, chlorine, bromine, methoxy and ethoxy,

n represents a number from 0-3,

G represents hydrogen (a), or represents the groups

-CO-$R^1$, (b)

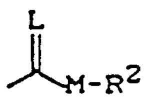 (c)

-$SO_2$-$R^3$ (d)

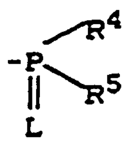 (e)

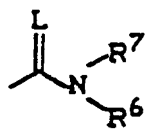 (f)

or E (g)

in which

E represents a metal ion equivalent or an ammonium ion,

L and M represent oxygen and/or sulphur,

$R^1$ represents optionally fluorine- or chlorine-substituted $C_1$-$C_{14}$-alkyl, $C_2$-$C_{14}$-alkenyl, $C_1$-$C_4$-alkoxy-$C_2$-$C_6$-alkyl, $C_1$-$C_4$-alkylthio-$C_2$-$C_6$-alkyl, $C_1$-$C_4$-polyalkoxy-$C_2$-$C_4$-alkyl and cycloalkyl which has 3-6 ring atoms and which can be interrupted by 1-2 oxygen and/or sulphur atoms,

or represents phenyl optionally substituted by fluorine, chlorine, bromine, methyl, ethyl, propyl, i-propyl, methoxy, ethoxy, trifluoromethyl, trifluoromethoxy or nitro,

or represents phenyl-$C_1$-$C_3$-alkyl optionally substituted by fluorine, chlorine, bromine, methyl, ethyl, propyl, i-propyl, methoxy, ethoxy, trifluoromethyl or trifluoromethoxy,

or represents pyridyl, pyrimidyl, thiazolyl or pyrazolyl optionally substituted by fluorine, chlorine, bromine, methyl or ethyl,

or represents phenoxy-$C_1$-$C_4$-alkyl optionally substituted by fluorine, chlorine,

methyl or ethyl,
or represents pyridyloxy-$C_1$-$C_4$-alkyl, pyrimidyloxy-$C_1$-$C_4$-alkyl or thiazolyloxy-$C_1$-$C_4$-alkyl optionally substituted by fluorine, chlorine, amino, methyl or ethyl,

$R^2$ represents $C_1$-$C_{14}$-alkyl, $C_2$-$C_{14}$-alkenyl, $C_1$-$C_4$-alkoxy-$C_2$-$C_6$-alkyl or $C_1$-$C_4$-polyalkoxy-$C_2$-$C_6$-alkyl, each of which is optionally substituted by fluorine or chlorine,
or represents phenyl or benzyl, each of which is optionally substituted by fluorine, chlorine, nitro, methyl, ethyl, propyl, i-propyl, methoxy, ethoxy or trifluoromethyl,

$R^3$, $R^4$ and $R^5$ independently of one another represent $C_1$-$C_4$-alkyl, $C_1$-$C_4$-alkoxy, $C_1$-$C_4$-alkylamino, di-($C_1$-$C_4$-alkyl)-amino or $C_1$-$C_4$-alkylthio, each of which is optionally substituted by fluorine or chlorine, or represent phenyl, phenoxy or phenylthio, each of which is optionally substituted by fluorine, chlorine, bromine, nitro, cyano, $C_1$-$C_2$-alkoxy, $C_1$-$C_4$-fluoroalkoxy, $C_1$-$C_2$-chloroalkoxy, $C_1$-$C_2$-alkylthio, $C_1$-$C_2$-fluoroalkylthio, $C_1$-$C_2$-chloroalkylthio or $C_1$-$C_3$-alkyl,

$R^6$ and $R^7$ independently of one another represent $C_1$-$C_{10}$-alkyl, $C_1$-$C_{10}$-alkoxy or $C_1$-$C_{10}$-alkoxy-($C_1$-$C_{10}$) alkyl, each of which is optionally substituted by fluorine, chlorine or bromine, or represent phenyl which is optionally substituted by fluorine, chlorine, bromine, $C_1$-$C_{20}$-halogenoalkyl, $C_1$-$C_{20}$-alkyl or $C_1$-$C_4$-alkoxy, or represent benzyl which is optionally substituted by fluorine, chlorine, bromine, $C_1$-$C_4$-alkyl, $C_1$-$C_4$-halogenoalkyl or $C_1$-$C_4$-alkoxy,

$R^9$ represents hydrogen or halogen, or represents $C_1$-$C_2$-alkyl or $C_1$-$C_2$-alkoxy, each of which is optionally substituted by halogen, and

$R^{10}$ represents methoxy, ethoxy, propoxy, iso-propoxy, butoxy, iso-butoxy, sec.-butoxy, tert.-butoxy, phenoxy, benzyloxy, amino, $C_1$-$C_2$-alkylamino or $C_1$-$C_2$-dialkylamino.

**5.** Process for the preparation of polycyclic 3-arylpyrrolidine-2,4-dione derivatives of the formula (I) according to Claim 1

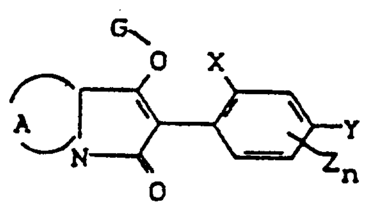

(I)

in which
A, G, X, Y, Z and n have the meanings given in Claim 1,
characterized in that, to obtain 3-aryl-pyrrolidine-2,4-diones or their enols of the formula (Ia)

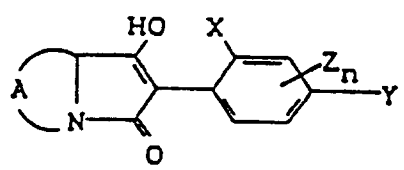

(Ia)

in which
A, X, Y, Z and n have the abovementioned meaning,
(A)
N-acylamino acid esters of the formula (II)

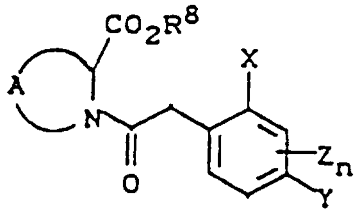

(II)

in which

A, Y, Z and n  have the abovementioned meaning

and

$R^8$  represents alkyl

are subjected to intramolecular condensation in the presence of a diluent and in the presence of a base,

(B)

or in that, to obtain compounds of the formula (Ib)

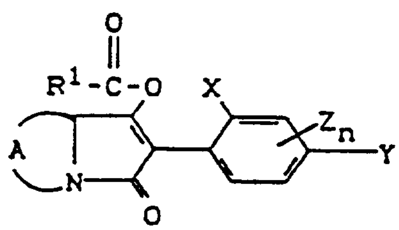

(Ib)

in which

A, X, Y, Z, $R^1$ and n  have the abovementioned meaning,

compounds of the formula (Ia)

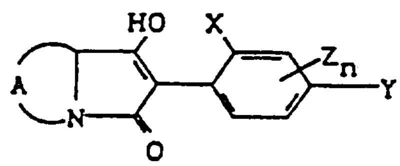

(Ia)

in which

A, X, Y, Z and n  have the abovementioned meaning

are reacted

α) with acid halides of the general formula (III)

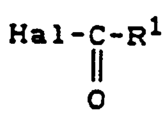

(III)

in which

$R^1$  has the abovementioned meaning

and

Hal  represents halogen, in particular chlorine and bromine,

if appropriate in the presence of a diluent and if appropriate in the presence of an acid-binding agent, or

β) with carboxylic anhydrides of the general formula (IV)

$R^1$-CO-O-CO-$R^1$  (IV)

in which

$R^1$ has the abovementioned meaning,

if appropriate in the presence of a diluent and if appropriate in the presence of an acid-binding agent,

(C)

or in that, to obtain compounds of the formula (Ic)

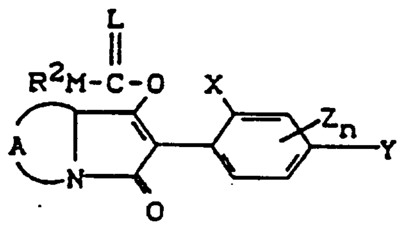

(Ic)

in which

A, X, Y, Z, $R^2$ and n have the abovementioned meaning,

L represents oxygen

and

M represents oxygen or sulphur

compounds of the formula (Ia)

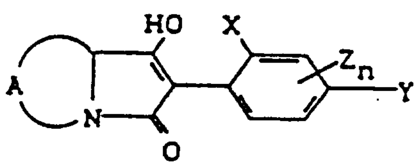

(Ia)

in which

A, X, Y, Z and n have the abovementioned meaning,

are reacted with chloroformic ester or chloroformic thioester of the general formula (V)

$R^2$-M-CO-Cl (V)

in which

$R^2$ and M have the abovementioned meaning,

if appropriate in the presence of a diluent and if appropriate in the presence of an acid-binding agent,

(D)

or in that, to obtain compounds of the formula (Ic)

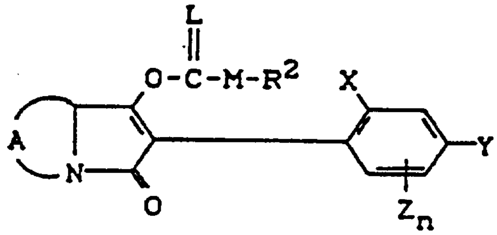

(Ic)

in which

A, $R^2$, X, Y, Z and n have the abovementioned meaning,

L represents sulphur

and

M represents oxygen or sulphur

compounds of the formula (Ia)

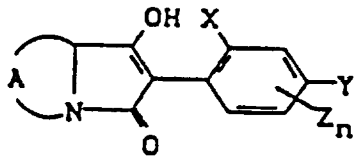

(Ia)

in which

A, X, Y, Z and n have the abovementioned meaning

are reacted

α) with chloromonothioformic esters or chlorodithioformic esters of the general formula (VI)

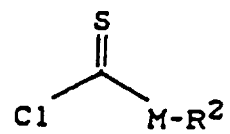

(VI)

in which

M and $R^2$ have the abovementioned meaning,

if appropriate in the presence of a diluent and if appropriate in the presence of an acid-binding agent,

or

β) with carbon disulphide and then with alkyl halides of the general formula (VII)

$R^2$-Hal (VII)

in which

$R^2$ has the abovementioned meaning

and

Hal represents chlorine, bromine or iodine,

(E)

or in that, to obtain compounds of the formula (Id),

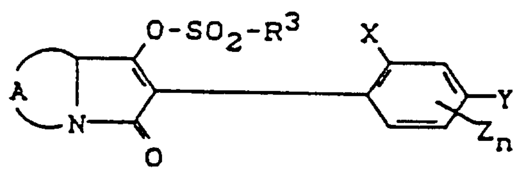

(Id)

in which

A, X, Y, Z, $R^3$ and n have the abovementioned meaning,

compounds of the formula (Ia)

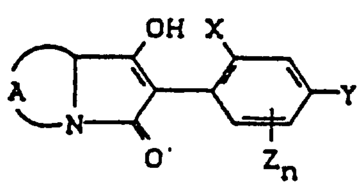

(Ia)

in which

A, X, Y, Z and n have the abovementioned meaning,

are reacted with sulphonyl chlorides of the general formula (VIII)

$R^3$-$SO_2$-Cl (VIII)

in which

$R^3$ has the abovementioned meaning,

if appropriate in the presence of a diluent and if appropriate in the presence of an acid-binding agent,

(F)

or in that, to obtain 3-aryl-pyrrolidine-2,4-diones of the formula (Ie)

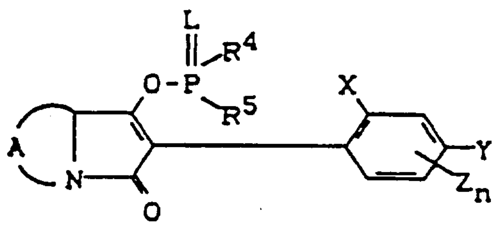

(Ie)

in which

A, L, X, Y, Z, $R^4$, $R^5$ and n have the abovementioned meaning,

3-aryl-pyrrolidine-2,4-diones of the formula (Ia) or their enols of the formula

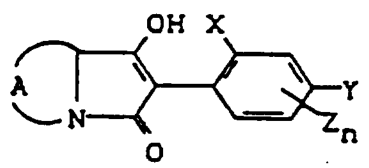

(Ia)

in which

A, X, Y, Z and n have the abovementioned meaning,

are reacted with phosphorus compounds of the general formula (IX)

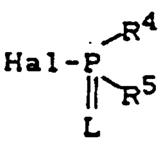

(IX)

in which

L, $R^4$ and $R^5$ have the abovementioned meaning

and

Hal represents halogen, in particular chlorine and bromine,

if appropriate in the presence of a diluent and if appropriate in the presence of an acid-binding agent,

(G)

or in that, to obtain compounds of the formula (If)

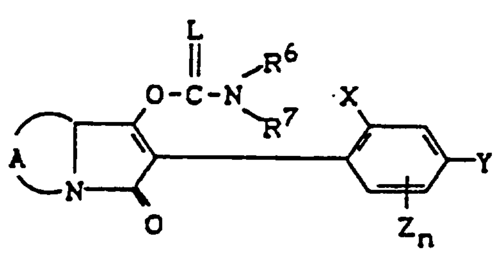

(If)

in which
A, L, X, Y, Z, $R^6$, $R^7$ and n have the abovementioned meaning,
compounds of the formula (Ia)

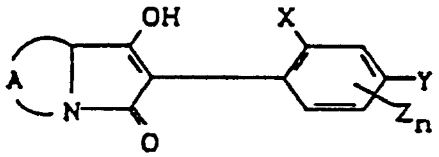

(Ia)

in which
A, X, Y, Z and n have the abovementioned meaning,
α) with isocyanates of the general formula (X)

$R^6$-N=C=O (X)

in which
$R^6$ has the abovementioned meaning,
if appropriate in the presence of a diluent and if appropriate in the presence of a catalyst,
or
β) with carbamoyl chlorides or thiocarbamoyl chlorides of the general formula (XI)

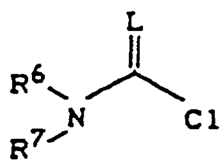

(XI)

in which
L, $R^6$ and $R^7$ have the abovementioned meaning,
if appropriate in the presence of a diluent and if appropriate in the presence of an acid-binding agent,
(H)
or in that, to obtain compounds of the formula (Ig)

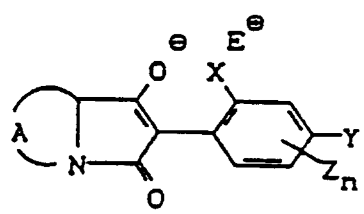

(Ig)

in which
X, Y, Z, A and n have the abovementioned meaning,
compounds of the formula (Ia)

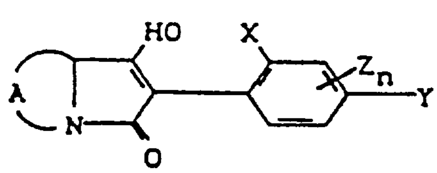

(Ia)

in which
X, Y, Z, A and n have the abovementioned meaning,
are reacted with metal hydroxides or amines of the general formulae (XII) and (XIII)

$MeE_sOH_t$ (XII)

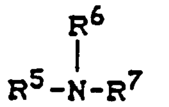

(XIII)

in which
Me represents mono- or divalent metal ions,
s and t represent the number 1 and 2 and
$R^5$, $R^6$ and $R^7$ independently of one another represent hydrogen and alkyl,
if appropriate in the presence of a diluent.

**6.** Insecticidal, acaricidal and herbicidal agents, characterized in that they contain at least one polycyclic 3-arylpyrrolidine-2,4-dione derivative of the formula (I) according to Claim 1.

**7.** Method of combating insects, arachnids and/or weeds, characterized in that polycyclic 3-arylpyrrolidine-2,4-dione derivatives of the formula (I) according to Claim 1 are allowed to act on insects, arachnids and/or weeds and/or their environment.

**8.** Use of polycyclic 3-arylpyrrolidine-2,4-dione derivatives of the formula (I) according to Claim 1 for combating insects, arachnids and/or weeds.

**9.** Process for the preparation of insecticidal, acaricidal and herbicidal agents, characterized in that polycyclic 3-arylpyrrolidine-2,4-dione derivatives of the formula (I) according to Claim 1 are mixed with extenders and/or surface-active agents.

**Revendications**

**1.** Dérivés polycycliques de la 3-aryl-pyrrolidine-2,4-dione, répondant à la formule (I)

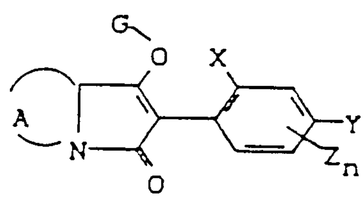

(I)

dans laquelle le groupement

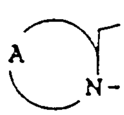

prend une des significations 1 à 29 ci-après :

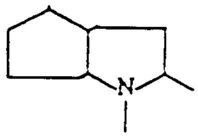

1

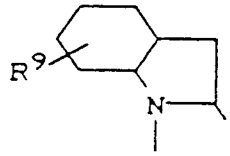

2

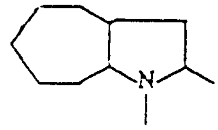

3

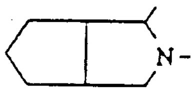

4

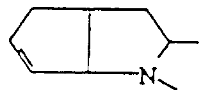

5

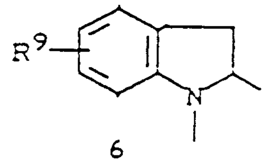

6

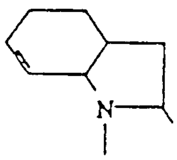

7

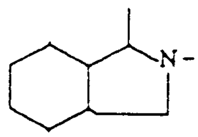

8

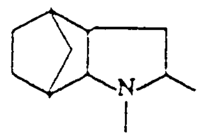

9

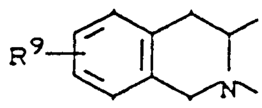

10

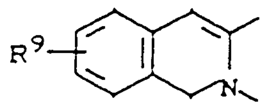

11

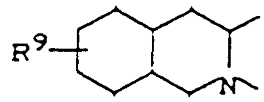

12

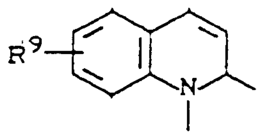

13

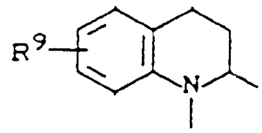

14

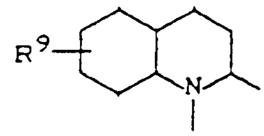

15

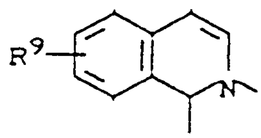

16

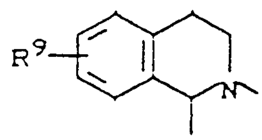

17

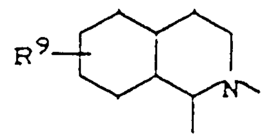

18

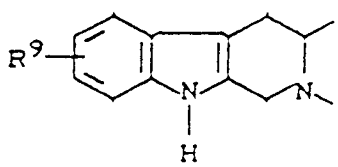

19

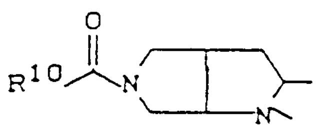

20

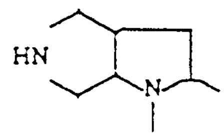

21

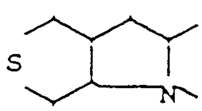

22

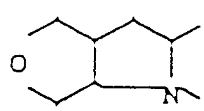

23

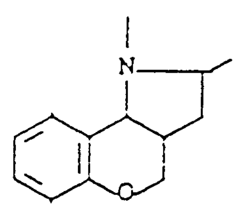

24

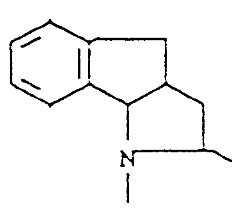

25

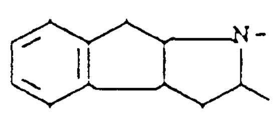

26

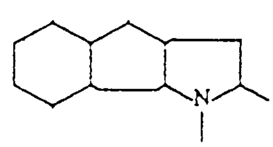

27

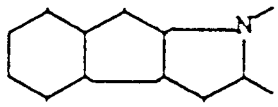

28

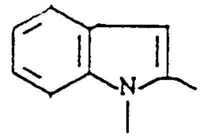

29

X représente un groupe alkyle en $C_1$-$C_6$, un atome d'halogène ou un groupe alcoxy en $C_1$-$C_6$,
Y représente un atome d'hydrogène, un groupe alkyle en $C_1$-$C_6$, un atome d'halogène, un groupe alcoxy en $C_1$-$C_6$, un groupe halogénalkyle en $C_1$-$C_3$,
Z représente un groupe alkyle en $C_1$-$C_6$, un atome d'halogène, un groupe alcoxy en $C_1$-$C_6$,
n représente un nombre de 0-3,
G représente un atome d'hydrogène (a) ou encore les groupes
-CO-$R^1$, (b)

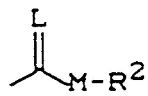

(c)

-$SO_2$-$R^3$ (d)

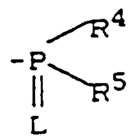

(e)

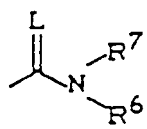

(f)

E (g)
dans lesquels
E représente un équivalent d'ion métallique ou un ion ammonium,
L et M représentent un atome d'oxygène et/ou de soufre,
$R^1$ représente un groupe alkyle en $C_1$-$C_{20}$, un groupe alcényle en $C_2$-$C_{20}$, un groupe alcoxy(en $C_1$-$C_8$)alkyle en $C_2$-$C_8$, un groupe alkyl(en $C_1$-$C_8$)thioalkyle en $C_2$-$C_8$, un groupe polyalcoxy(en $C_1$-$C_8$)alkyle en $C_2$-$C_8$ ou encore un groupe cycloalkyle contenant 3 à 8 atomes cycliques, qui peut être interrompu par des atomes d'oxygène et/ou de soufre, les groupes précités portant éventuellement un ou plusieurs substituants halogéno,
représente un groupe phényle portant éventuellement un ou plusieurs substituants identiques ou différents halogéno, nitro, alkyle en $C_1$-$C_6$, alcoxy en $C_1$-$C_6$, halogénalkyle en $C_1$-$C_6$, halogénalcoxy en $C_1$-$C_6$;
représente un groupe phénylalkyle en $C_1$-$C_6$ portant éventuellement un ou plusieurs substituants identiques ou différents halogéno, alkyle en $C_1$-$C_6$, alcoxy en $C_1$-$C_6$, halogénalkyle en $C_1$-$C_6$, halogénalcoxy en $C_1$-$C_6$,
représente un groupe pyridyle, un groupe pyrimidyle, un groupe thiazolyle ou un groupe pyrazolyle, les groupes précités portant éventuellement un ou plusieurs substituants identiques ou différents halogéno et/ou alkyle en $C_1$-$C_6$,
représente un groupe phénoxyalkyle en $C_1$-$C_6$ portant éventuellement un ou plusieurs substituants identiques ou différents halogéno et/ou alkyle en $C_1$-$C_6$,
représente un groupe pyridyloxyalkyle en $C_1$-$C_6$, un groupe pyrimidyloxyalkyle en $C_1$-$C_6$ ou un groupe thiazolyloxyalkyle en $C_1$-$C_6$, les groupes précités portant éventuellement un ou plusieurs substituants identiques ou différents halogéno, amino et alkyle en $C_1$-$C_6$,
$R^2$ représente un groupe alkyle en $C_1$-$C_{20}$, un groupe alcényle en $C_2$-$C_{20}$, un groupe alcoxy(en $C_1$-$C_8$)alkyle en $C_2$-$C_8$, un groupe polyalcoxy(en $C_1$-$C_8$)alkyle en $C_2$-$C_8$,

les groupes précités portant éventuellement un ou plusieurs substituants halogéno, représente un groupe phényle ou un groupe benzyle, les groupes précités portant éventuellement un ou plusieurs substituants identiques ou différents halogéno, nitro, alkyle en $C_1$-$C_6$, alcoxy en $C_1$-$C_6$, halogénalkyle en $C_1$-$C_6$,

$R^3$, $R^4$ et $R^5$ représentent, indépendamment l'un de l'autre, un groupe alkyle en $C_1$-$C_8$, un groupe alcoxy en $C_1$-$C_8$, un groupe alkylamino en $C_1$-$C_8$, un groupe dialkyl(en $C_1$-$C_8$)amino, un groupe alkyl(en $C_1$-$C_8$)thio, un groupe alcényl(en $C_2$-$C_5$)thio, un groupe alcynyl(en $C_2$-$C_5$)thio, un groupe cycloalkyl(en $C_3$-$C_7$)thio, les groupes précités portant éventuellement un ou plusieurs substituants halogéno; représentent un groupe phényle, un groupe phénoxy ou un groupe phénylthio, les groupes précités portant éventuellement un ou plusieurs substituants identiques ou différents halogéno, nitro, cyano, alcoxy en $C_1$-$C_4$, halogénalcoxy en $C_1$-$C_4$ ou alkyl(en $C_1$-$C_4$)thio, halogénalkyl(en $C_1$-$C_4$)thio, alkyle en $C_1$-$C_4$, halogénalkyle en $C_1$-$C_4$,

$R^6$ et $R^7$ représentent, indépendamment l'un de l'autre, un groupe alkyle en $C_1$-$C_{20}$, un groupe alcoxy en $C_1$-$C_{20}$, un groupe alcényle en $C_2$-$C_8$, un groupe alcoxy(en $C_1$-$C_{20}$)alkyle en $C_1$-$C_{20}$, les groupes précités portant éventuellement un ou plusieurs substituants halogéno; représentent un groupe phényle portant éventuellement un ou plusieurs substituants identiques ou différents halogéno, halogénalkyle en $C_1$-$C_{20}$, alkyle en $C_1$-$C_{20}$ ou alcoxy en $C_1$-$C_{20}$; représentent un groupe benzyle portant éventuellement un ou plusieurs substituants identiques ou différents halogéno, alkyle en $C_1$-$C_{20}$, halogénalkyle en $C_1$-$C_{20}$ ou alcoxy en $C_1$-$C_{20}$; ou représentent ensemble un cycle alkylène en $C_2$-$C_6$ éventuellement interrompu par un atome d'oxygène,

$R^9$ représente un atome d'hydrogène, un atome d'halogène; ou encore un groupe alkyle en $C_1$-$C_2$ ou un groupe alcoxy en $C_1$-$C_2$, les groupes précités portant éventuellement un ou plusieurs substituants halogéno,

$R^{10}$ représente un groupe alcoxy en $C_1$-$C_7$, un groupe amino, un groupe alkyl(en $C_1$-$C_4$)amino, un groupe dialkyl(en $C_1$-$C_4$)amino.

**2.** Dérivés polycycliques de la 3-aryl-pyrrolidine-2,4-dione, répondant à la formule (I) selon la revendication 1, caractérisés en ce qu'il s'agit d'une des structures (Ia) à (Ig) ci-après :

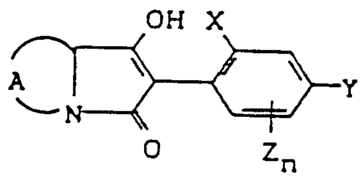

(Ia)

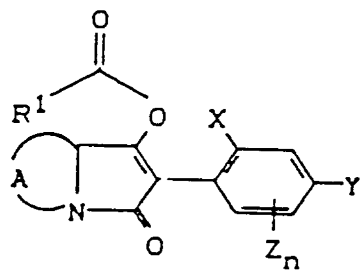

(Ib)

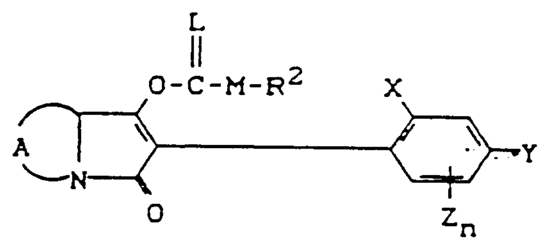

(Ic)

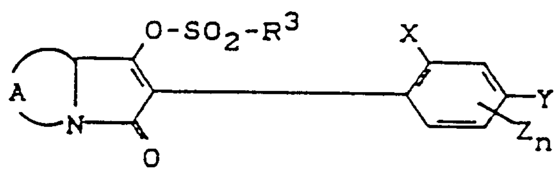

(Id)

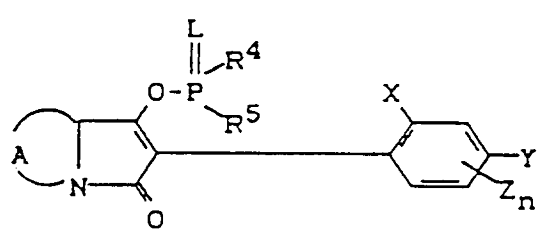

(Ie)

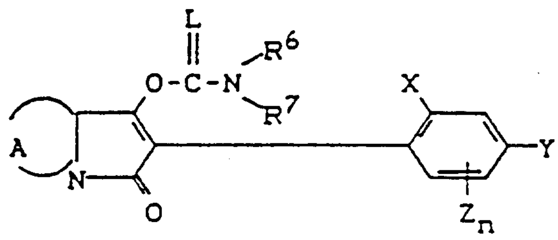

(If)

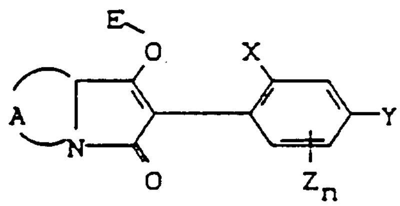

(Ig)

dans lesquelles

A, E, L, M, X, Y, $Z_n$, $R^1$, $R^2$, $R^3$, $R^4$, $R^5$, $R^6$ et $R^7$ possèdentles significations indiquées dans la revendication 1.

**3.** Dérivés polycycliques de la 3-aryl-pyrrolidine-2,4-dione, répondant à la formule (I) selon la revendication 1, caractérisés en ce que le groupement

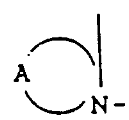

représente une des significations 1 à 29 ci-après :

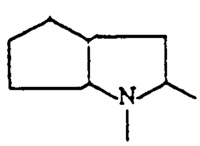

1

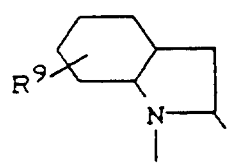

2

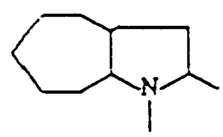

3

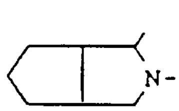
4

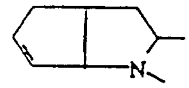
5

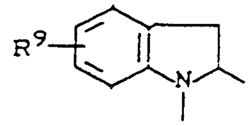
6

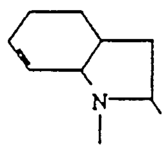
7

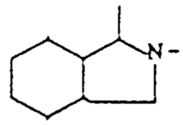
8

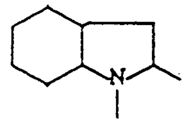
9

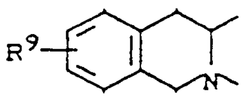
10

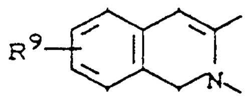
11

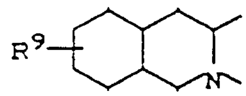
12

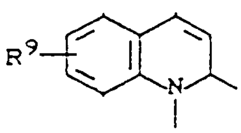
13

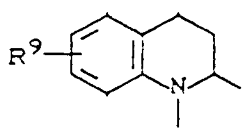
14

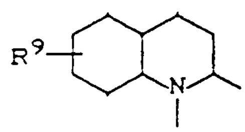
15

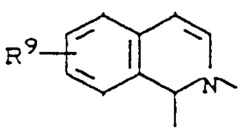
16

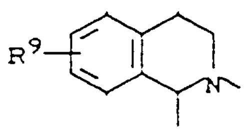
17

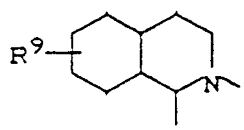
18

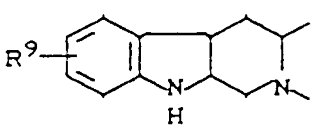
19

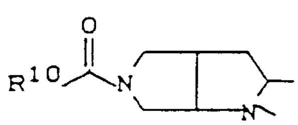
20

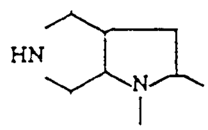
21

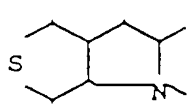
22

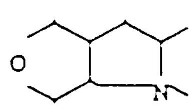
23

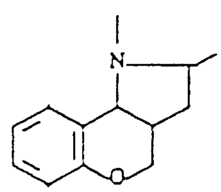
24

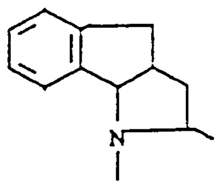 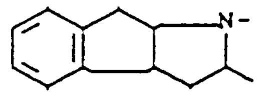 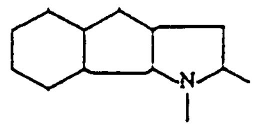

25 26 27

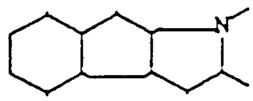 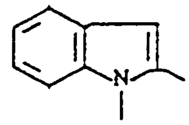

28 29

X représente un groupe alkyle en $C_1$-$C_4$, un atome d'halogène ou un groupe alcoxy en $C_1$-$C_4$,

Y représente un atome d'hydrogène, un groupe alkyle en $C_1$-$C_4$, un atome d'halogène, un groupe alcoxy en $C_1$-$C_4$, un groupe halogénalkyle en $C_1$-$C_2$,

Z représente un groupe alkyle en $C_1$-$C_4$, un atome d'halogène, un groupe alcoxy en $C_1$-$C_4$,

n représente un nombre de 0-3,

G représente un atome d'hydrogène (a) ou encore les groupes

-CO-$R^1$, (b)

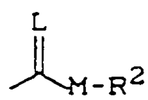 (c)

-$SO_2$-$R^3$ (d)

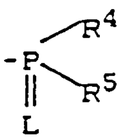 (e) 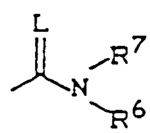 (f)

ou E (g)

dans lesquels

E représente un équivalent d'ion métallique ou un ion ammonium,

L et M représentent un atome d'oxygène et/ou de soufre,

$R^1$ représente un groupe alkyle en $C_1$-$C_{16}$, un groupe alcényle en $C_2$-$C_{16}$, un groupe alcoxy(en $C_1$-$C_6$)alkyle en $C_2$-$C_6$, un groupe alkyl(en $C_1$-$C_{16}$)thioalkyle en $C_2$-$C_6$, un groupe polyalcoxy(en $C_1$-$C_6$)alkyle en $C_2$-$C_6$ ou encore un groupe cycloalkyle contenant 3 à 7 atomes cycliques, qui peut être interrompu par 1-2 atomes d'oxygène et/ou de soufre, les groupes précités portant éventuellement un ou plusieurs substituants halogéno,

représente un groupe phényle portant éventuellement un ou plusieurs substituants identiques ou différents halogéno, nitro, alkyle en $C_1$-$C_4$, alcoxy en $C_1$-$C_4$, halogénalkyle en $C_1$-$C_3$, halogénalcoxy en $C_1$-$C_3$;

représente un groupe phénylalkyle en $C_1$-$C_4$ portant éventuellement un ou plusieurs substituants identiques ou différents halogéno, alkyle en $C_1$-$C_4$, alcoxy en $C_1$-$C_4$, halogénalkyle en $C_1$-$C_3$, halogénalcoxy en $C_1$-$C_3$,

représente un groupe pyridyle, un groupe pyrimidyle, un groupe thiazolyle ou un groupe pyrazolyle, les groupes précités portant éventuellement un ou plusieurs

substituants identiques ou différents halogéno et/ou alkyle en $C_1$-$C_6$,
représente un groupe phénoxyalkyle en $C_1$-$C_5$ portant éventuellement un ou plusieurs substituants identiques ou différents halogéno et/ou alkyle en $C_1$-$C_4$,
représente un groupe pyridyloxyalkyle en $C_1$-$C_5$, un groupe pyrimidyloxyalkyle en $C_1$-$C_5$ ou un groupe thiazolyloxyalkyle en $C_1$-$C_5$, les groupes précités portant éventuellement un ou plusieurs substituants identiques ou différents halogéno, amino, alkyle en $C_1$-$C_4$,

$R^2$ représente un groupe alkyle en $C_1$-$C_{16}$, un groupe alcényle en $C_2$-$C_{16}$, un groupe alcoxy(en $C_1$-$C_{16}$)alkyle en $C_2$-$C_6$, un groupe polyalcoxy(en $C_1$-$C_6$)alkyle en $C_2$-$C_6$, les groupes précités portant éventuellement un ou plusieurs substituants halogéno,
représente un groupe phényle ou un groupe benzyle, les groupes précités portant éventuellement un ou plusieurs substituants identiques ou différents halogéno, nitro, alkyle en $C_1$-$C_4$, alcoxy en $C_1$-$C_3$, halogénalkyle en $C_1$-$C_3$,

$R^3$, $R^4$ et $R^5$ représentent, indépendamment l'un de l'autre, un groupe alkyle en $C_1$-$C_6$, un groupe alcoxy en $C_1$-$C_6$, un groupe alkylamino en $C_1$-$C_6$, un groupe dialkyl(en $C_1$-$C_6$)amino, un groupe alkyl(en $C_1$-$C_6$)thio, un groupe alcényl(en $C_3$-$C_4$)thio, un groupe alcynyl(en $C_2$-$C_4$)thio, un groupe cycloalkyl(en $C_3$-$C_6$)thio, les groupes précités portant éventuellement un ou plusieurs substituants identiques ou différents halogéno; représentent un groupe phényle, un groupe phénoxy ou un groupe phénylthio, les groupes précités portant éventuellement un ou plusieurs substituants identiques ou différents fluor, chlore, brome, nitro, cyano, alcoxy en $C_1$-$C_3$, halogénalcoxy en $C_1$-$C_3$ ou alkyl(en $C_1$-$C_3$)thio, halogénalkyl(en $C_1$-$C_3$)thio, alkyle en $C_1$-$C_3$, halogénalkyle en $C_1$-$C_3$,

$R^6$ et $R^7$ représentent, indépendamment l'un de l'autre, un groupe alkyle en $C_1$-$C_{20}$, un groupe alcoxy en $C_1$-$C_{20}$, un groupe alcényle en $C_2$-$C_8$, un groupe alcoxy(en $C_1$-$C_{20}$)alkyle en $C_1$-$C_{20}$, les groupes précités portant éventuellement un ou plusieurs substituants halogéno; représentent un groupe phényle portant éventuellement un ou plusieurs substituants identiques ou différents halogéno, halogénalkyle en $C_1$-$C_5$, alkyle en $C_1$-$C_5$ ou alcoxy en $C_1$-$C_5$; représentent un groupe benzyle portant éventuellement un ou plusieurs substituants identiques ou différents halogéno, alkyle en $C_1$-$C_5$, halogénalkyle en $C_1$-$C_5$, alcoxy en $C_1$-$C_5$,

$R^9$ représente un atome d'hydrogène, un atome d'halogène; ou encore un groupe alkyle en $C_1$-$C_2$ ou un groupe alcoxy en $C_1$-$C_2$, les groupes précités portant éventuellement un ou plusieurs substituants halogéno,

$R^{10}$ représente un groupe alcoxy en $C_1$-$C_7$, un groupe amino, un groupe alkyl(en $C_1$-$C_4$)amino, un groupe dialkyl(en $C_1$-$C_4$)amino.

**4.** Dérivés polycycliques de la 3-aryl-pyrrolidine-2,4-dione, répondant à la formule (I) selon la revendication 1, caractérisés en ce que le groupement

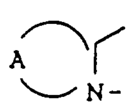

représente une des significations 1 à 29 ci-après :

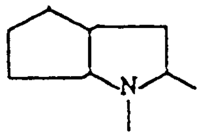

1

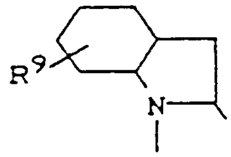

2

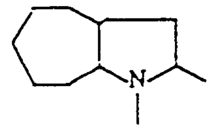

3

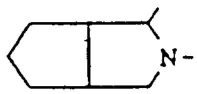

4

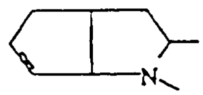

5

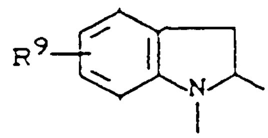

6

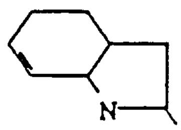
7

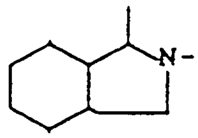
8

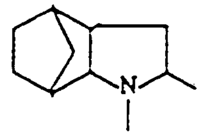
9

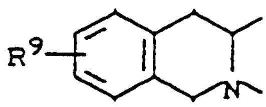
10

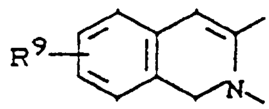
11

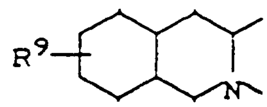
12

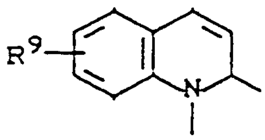
13

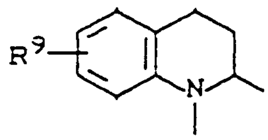
14

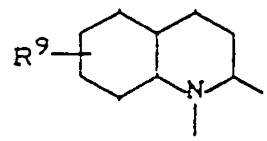
15

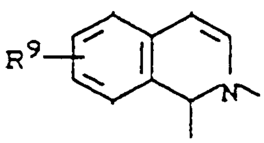
16

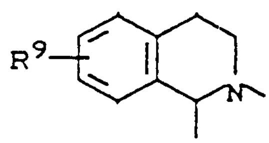
17

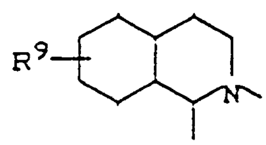
18

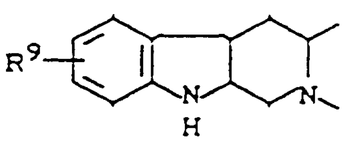
19

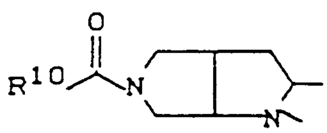
20

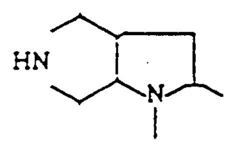
21

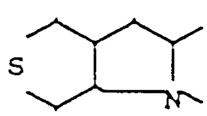
22

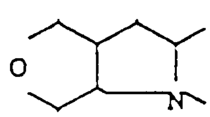
23

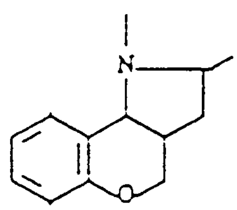
24

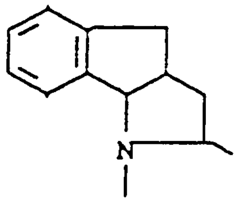

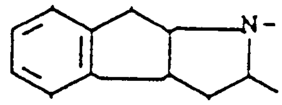

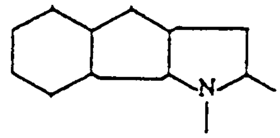

25 26 27

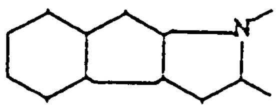

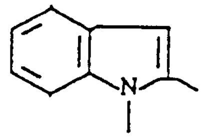

28 29

X représente un groupe méthyle, un groupe éthyle, un groupe propyle, un groupe i-propyle, un atome de fluor, un atome de chlore, un atome de brome, un groupe méthoxy et un groupe éthoxy,

Y représente un atome d'hydrogène, un groupe méthyle, un groupe éthyle, un groupe propyle, un groupe i-propyle, un groupe butyle, un groupe i-butyle, un groupe tert.-butyle, un atome de fluor, un atome de chlore, un atome de brome, un groupe méthoxy, un groupe éthoxy et un groupe trifluorométhyle,

Z représente un groupe méthyle, un groupe éthyle, un groupe i-propyle, un groupe butyle, un groupe i-butyle, un groupe tert.-butyle, un atome de fluor, un atome de chlore, un atome de brome, un groupe méthoxy et un groupe éthoxy,

n représente un nombre de 0-3,

G représente un atome d'hydrogène (a) ou les groupes

$-CO-R^1$, (b)

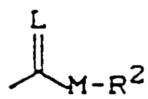 (c)

$-SO_2-R^3$ (d)

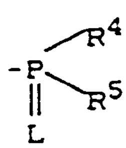 (e)

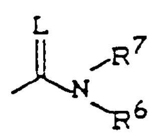 (f)

ou E (g)

dans lesquels

E représente un équivalent d'ion métallique ou un ion ammonium,

L et M représentent un atome d'oxygène et/ou de soufre,

$R^1$ représente un groupe alkyle en $C_1$-$C_{14}$, un groupe alcényle en $C_2$-$C_{14}$, un groupe alcoxy(en $C_1$-$C_4$)alkyle en $C_2$-$C_6$, un groupe alkyl(en $C_1$-$C_4$)thioalkyle en $C_2$-$C_6$, un groupe polyalcoxy(en $C_1$-$C_4$)alkyle en $C_2$-$C_4$ et un groupe cycloalkyle contenant 3 à 6 atomes cycliques, qui peut être interrompu par 1-2 atomes d'oxygène et/ou de soufre, les groupes précités portant éventuellement un ou plusieurs substituants identiques ou différents fluor ou chlore,

représente un groupe phényle portant éventuellement un ou plusieurs substituants identiques ou différents fluor, chlore, brome, méthyle, éthyle, propyle, i-propyle, méthoxy, éthoxy, trifluorométhyle, trifluorométhoxy, nitro,
représente un groupe phénylalkyle en $C_1$-$C_3$ portant éventuellement un ou plusieurs substituants identiques ou différents fluor, chlore, brome, méthyle, éthyle, propyle, i-propyle, méthoxy, éthoxy, trifluorométhyle, trifluorométhoxy,
représente un groupe pyridyle, un groupe pyrimidyle, un groupe thiazolyle ou un groupe pyrazolyle, les groupes précités portant éventuellement un ou plusieurs substituants identiques ou différents fluor, chlore, brome, méthyle, éthyle,
représente un groupe phénoxyalkyle en $C_1$-$C_4$ portant éventuellement un ou plusieurs substituants identiques ou différents fluor, chlore, méthyle, éthyle,
représente un groupe pyridyloxyalkyle en $C_2$-$C_4$, un groupe pyrimidyloxyalkyle en $C_1$-$C_4$ ou un groupe thiazolyloxyalkyle en $C_1$-$C_4$, les groupes précités portant éventuellement un ou plusieurs substituants identiques ou différents fluor, chlore, amino, méthyle, éthyle,

$R^2$ représente un groupe alkyle en $C_1$-$C_{14}$, un groupe alcényle en $C_2$-$C_{14}$, un groupe alcoxy(en $C_1$-$C_4$)alkyle en $C_2$-$C_6$, un groupe polyalcoxy(en $C_1$-$C_4$)alkyle en $C_2$-$C_6$, les groupes précités portant éventuellement un ou plusieurs substituants identiques ou différents fluor ou chlore,
ou représente un groupe phényle ou un groupe benzyle, les groupes précités portant éventuellement un ou plusieurs substituants identiques ou différents fluor, chlore, nitro, méthyle, éthyle, propyle, i-propyle, méthoxy, éthoxy, trifluorométhyle,

$R^3$, $R^4$, et $R^5$ représentent, indépendamment l'un de l'autre, un groupe alkyle en $C_1$-$C_4$, un groupe alcoxy en $C_1$-$C_4$, un groupe alkyl(en $C_1$-$C_4$)amino, un groupe dialkyl(en $C_1$-$C_4$)amino, un groupe alkyl(en $C_1$-$C_4$)thio, les groupes précités portant éventuellement un ou plusieurs substituants identiques ou différents fluor ou chlore; représentent un groupe phényle, un groupe phénoxy ou un groupe phénylthio, les groupes précités portant éventuellement un ou plusieurs substituants identiques ou différents fluor, chlore, brome, nitro, cyano, alcoxy en $C_1$-$C_2$, fluoralcoxy en $C_1$-$C_4$, chloralcoxy en $C_1$-$C_2$, alkyl(en $C_1$-$C_2$)thio, fluoralkyl(en $C_1$-$C_2$)thio, chloralkyl(en $C_1$-$C_2$)thio, alkyle en $C_1$-$C_3$,

$R^6$ et $R^7$ représentent, indépendamment l'un de l'autre, un groupe alkyle en $C_1$-$C_{10}$, un groupe alcoxy en $C_1$-$C_{10}$, un groupe alcoxy(en $C_1$-$C_{10}$)alkyle en $C_1$-$C_{10}$, les groupes précités portant éventuellement un ou plusieurs substituants identiques ou différents fluor, chlore, brome; représentent un groupe phényle portant éventuellement un ou plusieurs substituants identiques ou différents fluor, chlore, brome, halogénalkyle en $C_1$-$C_{20}$, alkyle en $C_1$-$C_{20}$ ou alcoxy en $C_1$-$C_4$; représentent un groupe benzyle portant éventuellement un ou plusieurs substituants identiques ou différents fluor, chlore, brome, alkyle en $C_1$-$C_4$, halogénalkyle en $C_1$-$C_4$ ou alcoxy en $C_1$-$C_4$,

$R^9$ représente un atome d'hydrogène, un atome d'halogène ou encore un groupe alkyle en $C_1$-$C_2$ ou un groupe alcoxy en $C_1$-$C_2$, les groupes précités portant éventuellement un ou plusieurs substituants halogéno,

$R^{10}$ représente un groupe méthoxy, un groupe éthoxy, un groupe propoxy, un groupe isopropoxy, un groupe butoxy, un groupe isobutoxy, un groupe sec.-butoxy, un groupe tert.-butoxy, un groupe phénoxy, un groupe benzyloxy, un groupe amino, un groupe alkyl(en $C_1$-$C_2$)amino, un groupe dialkyl(en $C_1$-$C_2$)amino.

**5.** Procédé pour la préparation de dérivés polycycliques de la 3-aryl-pyrrolidine-2,4-dione, répondant à la formule (I) selon la revendication 1,

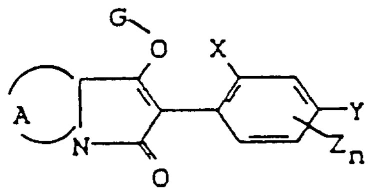

(I)

dans laquelle

A, G, X, Y, Z et n ont la signification indiquée à la revendication 1,

qui se caractérise par le fait que, pour obtenir les 3-aryl-pyrrolidine-2,4-diones, respectivement leurs composés énoliques de formule (Ia)

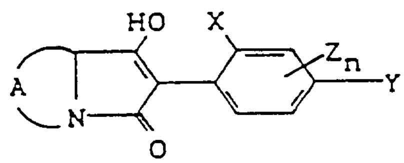

(Ia)

dans laquelle

A, X, Y, Z et n ont la signification indiquée ci-dessus,

(A)

on condense, par voie intramoléculaire, des esters de N-acylaminoacides de formule (II)

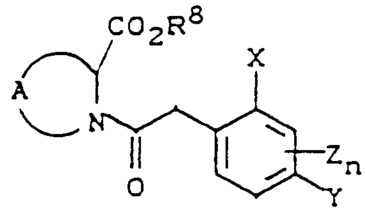

(II)

dans laquelle

A, Y, Z et n ont la signification indiquée ci-dessus,

et

$R^8$ représente un groupe alkyle,

en présence d'un diluant et en présence d'une base,

(B)

ou bien en ce que, pour obtenir les composés de formule (Ib)

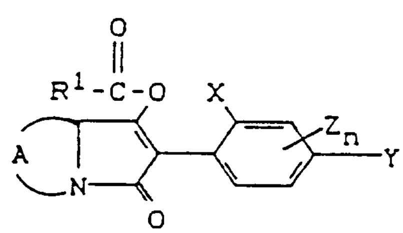

(Ib)

dans laquelle

A, X, Y, Z, $R^1$ et n ont la signification indiquée ci-dessus,

on fait réagir les composés de formule (Ia)

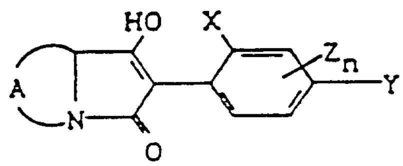

(Ia)

dans laquelle

A, X, Y, Z et n ont la signification indiquée ci-dessus,

α) avec des halogénures d'acides répondant à la formule générale (III)

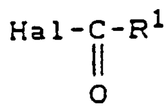

(III)

dans laquelle

$R^1$ a la signification indiquée ci-dessus,

et

Hal représente un atome d'halogène, en particulier un atome de chlore et un atome de brome,

éventuellement en présence d'un diluant et éventuellement en présence d'un agent neutralisateur d'acides,

ou

β) avec des anhydrides carboxyliques répondant à la formule générale (IV)

$R^1$-CO-O-CO-$R^1$ (IV)

dans laquelle

$R^1$ a la signification indiquée ci-dessus,

éventuellement en présence d'un diluant et éventuellement en présence d'un agent neutralisateur d'acides,

(C)

ou bien en ce que, pour obtenir les composés de formule (Ic)

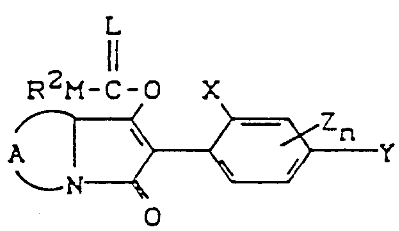

(Ic)

dans laquelle

A, X, Y, Z, $R^2$ et n ont la signification indiquée ci-dessus,

L représente un atome d'oxygène

et

M représente un atome d'oxygène ou un atome de soufre,

on fait réagir des composés de formule (Ia)

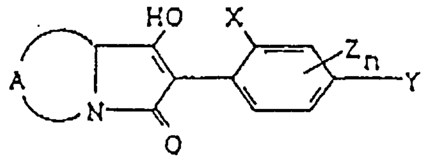

(Ia)

dans laquelle

A, X, Y, Z et n ont la signification indiquée ci-dessus,

avec des esters chloroformiques ou des thioesters chloroformiques répondant à la formule générale (V)

$R^2$-M-CO-Cl (V)

dans laquelle

$R^2$ et M ont la signification indiquée ci-dessus,

éventuellement en présence d'un diluant et éventuellement en présence d'un agent neutralisateur d'acides,

(D)

ou bien en ce que, pour obtenir les composés de formule (Ic)

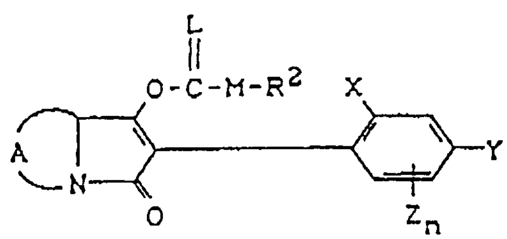

(Ic)

dans laquelle

A, $R^2$, X, Y, Z et n ont la signification indiquée ci-dessus,

L représente un atome de soufre

et

M représente un atome d'oxygène ou un atome de soufre,

on fait réagir des composés de formule (Ia)

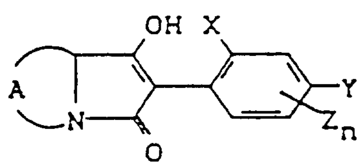

(Ia)

dans laquelle

A, X, Y, Z et n ont la signification indiquée ci-dessus,

α) avec des esters chloromonothioformiques ou des esters chlorodithioformiques répondant à la formule générale (VI)

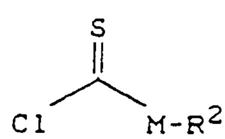

(VI)

dans laquelle

M et $R^2$ ont la signification indiquée ci-dessus,

éventuellement en présence d'un diluant et éventuellement en présence d'un agent neutralisateur d'acides,

ou

$\beta$) avec du sulfure de carbone et ensuite, avec des halogénures d'alkyle répondant à la formule générale (VII)

$R^2$-Hal (VII)

dans laquelle

$R^2$ a la signification indiquée ci-dessus,

et

Hal représente un atome de chlore, un atome de brome, un atome d'iode,

(E)

ou bien en ce que, pour obtenir les composés de formule (Id)

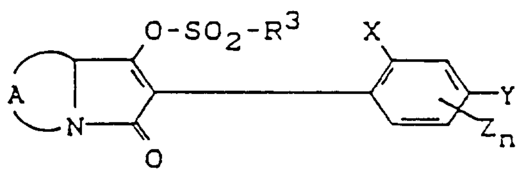

(Id)

dans laquelle

A, X, Y, Z, $R^3$ et n ont la signification indiquée ci-dessus,

on fait réagir des composés de formule (Ia)

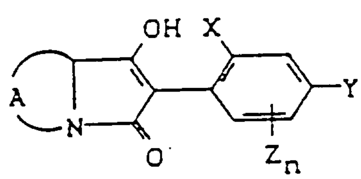

(Ia)

dans laquelle

A, X, Y, Z et n ont la signification indiquée ci-dessus,

avec des chlorures d'acides sulfoniques répondant à la formule générale (VIII)

$R^3-SO_2-Cl$ (VIII)

dans laquelle

$R^3$ a la signification indiquée ci-dessus,

éventuellement en présence d'un diluant et éventuellement en présence d'un agent neutralisateur d'acides,

(F)

ou bien en ce que, pour obtenir des 3-aryl-pyrrolidine-2,4-diones de formule (Ie)

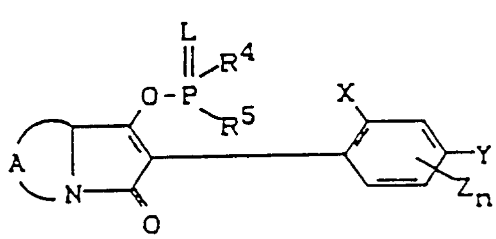

(Ie)

dans laquelle

A, L, X, Y, Z, $R^4$, $R^5$ et n ont la signification indiquée ci-dessus,
on fait réagir des 3-aryl-pyrrolidine-2,4-diones de formule (Ia), respectivement leurs composés énoliques de formule

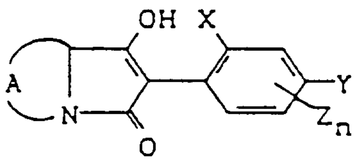

(Ia)

dans laquelle
A, X, Y, Z et n ont la signification indiquée ci-dessus,
avec des composés phosphorés répondant à la formule générale (IX)

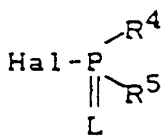

(IX)

dans laquelle
L, $R^4$ et $R^5$ ont la signification indiquée ci-dessus,
et
Hal représente un atome d'halogène, en particulier un atome de chlore et un atome de brome,
éventuellement en présence d'un diluant et éventuellement en présence d'un agent neutralisateur d'acides,
(G)
ou bien en ce que, pour obtenir les composés de formule (If)

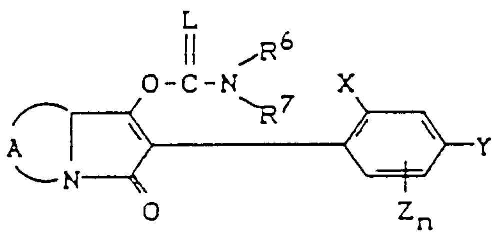

(If)

dans laquelle
A, L, X, Y, Z, $R^6$, $R^7$ et n ont la signification indiquée ci-dessus,
on fait réagir des composés de formule (Ia)

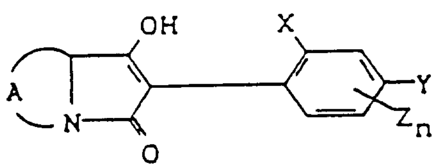

(Ia)

dans laquelle
A, X, Y, Z et n ont la signification indiquée ci-dessus,
α) avec des isocyanates de formule générale (X)

$R^6$-N=C=O (X)

dans laquelle

$R^6$ a la signification indiquée ci-dessus,

éventuellement en présence d'un diluant et éventuellement en présence d'un catalyseur,

ou

$\beta$) avec des chlorures d'acides carbamiques ou des chlorures d'acides thiocarbamiques répondant à la formule générale (XI)

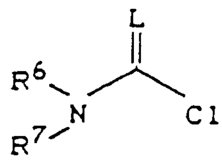

(XI)

dans laquelle

L, $R^6$ et $R^7$ ont la signification indiquée ci-dessus,

éventuellement en présence d'un diluant et éventuellement en présence d'un agent neutralisateur d'acides,

(H)

ou bien en ce que, pour obtenir les composés de formule (Ig)

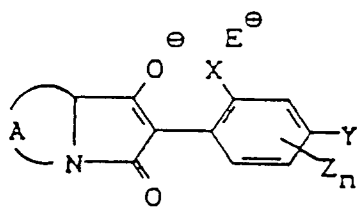

(Ig)

dans laquelle

X, Y, Z, A et n ont la signification indiquée ci-dessus,

on fait réagir des composés de formule (Ia)

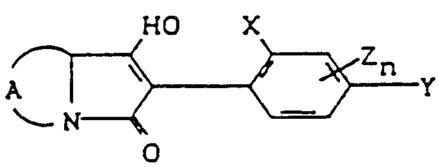

(Ia)

dans laquelle

X, Y, Z, A et n ont la signification indiquée ci-dessus,

avec des hydroxydes métalliques ou des amines répondant aux formules générales (XII) et (XIII)

$Me_sOH_t$ (XII)

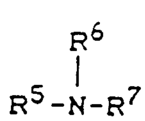

(XIII)

dans lesquelles

Me représente un ion métallique mono- ou bivalent,

s et t représentent le nombre 1 et 2 et
$R^5$, $R^6$ et $R^7$ représentent, indépendamment l'un de l'autre, un atome d'hydrogène et un groupe alkyle,

éventuellement en présence d'un diluant.

6. Agents insecticides, acaricides et herbicides caractérisés par une teneur en au moins un dérivé polycyclique de la 3-aryl-pyrrolidine-2,4-dione de formule (I) selon la revendication 1.

7. Procédé pour lutter contre les insectes, les arachnides et/ou les mauvaises herbes, caractérisé en ce qu'on laisse agir des dérivés polycycliques de la 3-aryl-pyrrolidine-2,4-dione, répondant à la formule (I) selon la revendication 1, sur des insectes, des arachnides et/ou des mauvaises herbes et/ou sur leur biotope.

8. Utilisation de dérivés polycycliques de la 3-aryl-pyrrolidine-2,4-dione, de formule (I) selon la revendication 1, pour lutter contre les insectes, les arachnides et/ou les mauvaises herbes.

9. Procédé pour préparer des agents insecticides, acaricides et herbicides, caractérisé en ce qu'on mélange des dérivés polycycliques de la 3-aryl-pyrrolidine-2,4-dione, de formule (I) selon la revendication 1, avec des diluants et/ou des agents tensioactifs.